# EUROPEAN PATENT APPLICATION

(11) **EP 4 844 030 A1**
(43) Date of publication of application: **25.11.2026**
(21) Application number: 25741923.4
(22) Date of filing: 14.01.2025
(51) Int. Cl.: C09B 1/60, C07C 225/36, C07C 323/38, C07D 295/135, C09B 1/32, C09B 1/514, C09B 1/54, C09B 1/58

(54) **ANTHRAQUINONE DERIVATIVE**

(30) Priority: 15.01.2024 JP 2024004138; 15.01.2024 JP 2024004139; 15.01.2024 JP 2024004140; 06.08.2024 JP 2024129818; 06.08.2024 JP 2024129819; 06.08.2024 JP 2024129820
(71) Applicant: Toppan Holdings Inc., Tokyo 110-0016 (JP)
(72) Inventor: SHIMADA, Erika, Tokyo 110-0016 (JP); MORINAGA, Takahiro, Tokyo 110-0016 (JP); TAGAMI, Hanae, Tokyo 110-0016 (JP); FURUKAWA, Shigeki, Tokyo 110-0016 (JP); IWAKI, Kentaro, Tokyo 110-0016 (JP)
(74) Representative: TBK
(86) International application number: PCT/JP2025/000787
(87) International publication number: WO 2025/154697

(57) **Abstract**

The disclosure provides an anthraquinone derivative expressed by the following Formula (9-1). In Formula (9-1), R¹ and R² each independently represent an amino group or hydroxyl group, Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents.

## Description

### [Technical Field]

The present disclosure relates to anthraquinone derivatives.

### [Background Art]

Organic pigments are widely used in various printing inks and optical filters. Furthermore, development is also progressing on organic pigments which can be used as dichroic pigments for liquid crystal elements or polarizing films.

Among organic pigments, many anthraquinone derivatives, which are compounds having an anthraquinone skeleton, have high stability and excellent robustness against light, heat, temperature, etc. Therefore, among anthraquinone derivatives, compounds that can be used as pigments corresponding to the three primary colors have been studied extensively from the perspectives of controlling absorption wavelength or coloring power, improving solubility in solvents and resins, and enhancing dichroism. For example, PTLs 1 and 2 describe anthraquinone derivatives that can be used as cyanide pigments and have a maximum absorption wavelength in the wavelength range of 580 nm or greater.

### [Citation List]

### [Patent Literature]

PTL1: JPS63-90568A
PTL2: JPS63-278994A

### [Summary of the Invention]

### [Technical Problem]

It is desirable that pigments have good light resistance, i.e., fade little when exposed to light. As mentioned above, many anthraquinone derivatives are compounds having high robustness; however, anthraquinone derivatives used as cyanide pigments tend to have difficulty in achieving light resistance. Therefore, there is a need for anthraquinone derivatives having a maximum absorption wavelength in the wavelength range of 580 nm or greater and having good light resistance.

### [Solution to Problems]

An aspect of an anthraquinone derivative is expressed by the following Formula (9-1). In Formula (9-1), R¹ and R² each independently represent an amino group or hydroxyl group, and Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents.

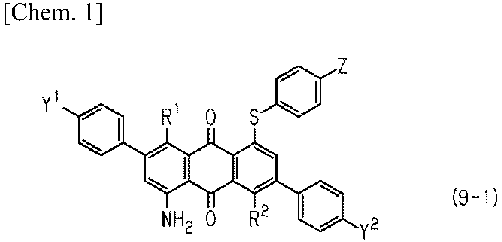

Another aspect of an anthraquinone derivative is expressed by the following Formula (1-1). In Formula (1-1), R¹, R² and R³ each independently represent an amino group or hydroxyl group, at least one of R¹, R² and R³ representing a hydroxyl group. At least one of Y¹ and Y² represents an electron-donating group, the electron-donating group being an alkylamino group, amino group, or piperidyl group, and if only one of Y¹ and Y² is an electron-donating group, the other of Y¹ and Y² is a hydrogen atom, alkyl group having 1 to 10 carbon atoms, alkoxy group having 1 to 10 carbon atoms, aryl group, or cyclohexyl group, and the aryl group and the cyclohexyl group may have substituents. Z represents a hydrogen atom, alkyl group having 1 to 10 carbon atoms, alkoxy group having 1 to 10 carbon atoms, cyano group, nitro group, halogen atom, amino group, or alkylamino group.

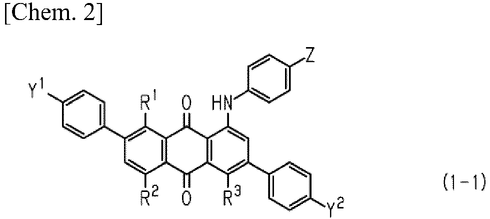

Another aspect of an anthraquinone derivative is expressed by the following Formula (1-1). In Formula (1-1), R¹, R² and R³ each independently represent an amino group or hydroxyl group. At least one of Y¹ and Y² represents an electron-donating group, the electron-donating group being an acetamide group or hydroxyl group, and if only one of Y¹ and Y² is an electron-donating group, the other of Y¹ and Y² is a hydrogen atom, alkyl group having 1 to 10 carbon atoms, alkoxy group having 1 to 10 carbon atoms, aryl group, or cyclohexyl group, and the aryl group and the cyclohexyl group may have substituents. Z represents a hydrogen atom, alkyl group having 1 to 10 carbon atoms, alkoxy group having 1 to 10 carbon atoms, cyano group, nitro group, halogen atom, amino group, or alkylamino group.

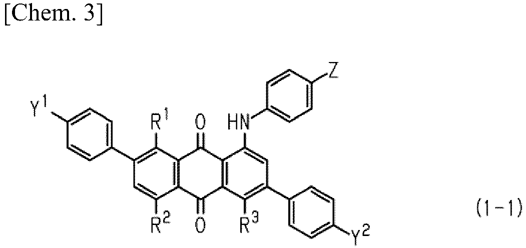

Another aspect of an anthraquinone derivative is expressed by the following Formula (2-1). In Formula (2-1), R¹, R² and R³ each independently represent an amino group or hydroxyl group, at least one of R¹, R² and R³ representing a hydroxyl group. Y¹ and Y² each independently represent a hydrogen atom, alkyl group having 1 to 10 carbon atoms, alkoxy group having 1 to 10 carbon atoms, aryl group, cyano group, nitro group, halogen atom, amino group, alkylamino group, or cyclohexyl group, and the aryl group and the cyclohexyl group may have substituents. Z represents an electron-withdrawing group, the electron-withdrawing group being a cyano group or halogenated alkyl group.

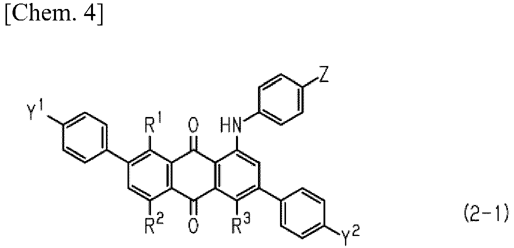

Another aspect of an anthraquinone derivative is expressed by the following Formula (2-1). In Formula (2-1), R¹, R² and R³ each independently represent an amino group or hydroxyl group. Y¹ and Y² each independently represent a hydrogen atom, alkyl group having 1 to 10 carbon atoms, alkoxy group having 1 to 10 carbon atoms, aryl group, cyano group, nitro group, halogen atom, amino group, alkylamino group, or cyclohexyl group, and the aryl group and the cyclohexyl group may have substituents. Z represents an electron-withdrawing group, the electron-withdrawing group being an aldehyde group, acetyl group, or sulfo group.

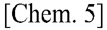

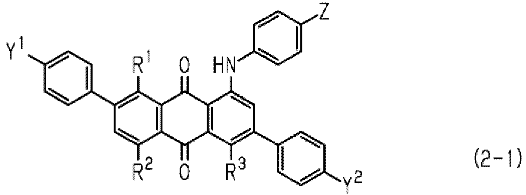

Another aspect of an anthraquinone derivative is expressed by the following Formula (3-1). In Formula (3-1), R¹, R² and R³ each independently represent an amino group or hydroxyl group, at least one of R¹, R² and R³ representing a hydroxyl group. At least one of Y¹ and Y² represents a cyclohexyl group, and the cyclohexyl group may have substituents, and if only one of Y¹ and Y² is a cyclohexyl group, the other of Y¹ and Y² is a hydrogen atom, alkyl group having 1 to 10 carbon atoms, alkoxy group having 1 to 10 carbon atoms, aryl group, cyano group, nitro group, halogen atom, amino group, or alkylamino group, and the aryl group may have substituents. Z represents a hydrogen atom, alkyl group having 1 to 10 carbon atoms, alkoxy group having 1 to 10 carbon atoms, cyano group, nitro group, halogen atom, amino group, or alkylamino group.

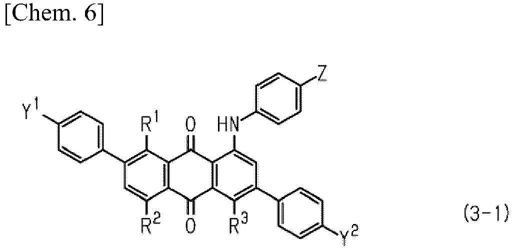

Another aspect of an anthraquinone derivative is expressed by the following Formula (8-1). In Formula (8-1), A represents a direct bond, oxygen atom, or -NH-; and Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, aryl group, piperidyl group, or cyclohexyl group, and the aryl group and the cyclohexyl group may have substituents.

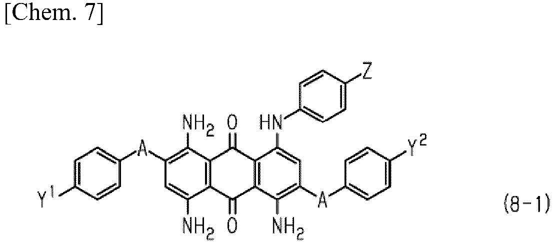

### [Description of the Embodiments]

Hereinafter, anthraquinone derivatives according to first to ninth embodiments will be described. In these embodiments, an anthraquinone derivative refers to a compound having a 9,10-anthraquinone skeleton. Furthermore, at the substitution sites of the anthraquinone skeleton, 1st, 4th, 5th and 8th positions correspond to α-positions, and 2nd, 3rd, 6th and 7th positions correspond to β-positions.

The anthraquinone derivatives of these embodiments are used as pigments. Uses as pigments are not particularly limited. For example, the anthraquinone derivatives can be used as pigments which are used for inks for sublimation transfer printing or inkjet printing, toners for laser printers or copiers, optical filters such as color filters for liquid crystal displays or color separation filters for image capture devices, and inks for tampering and forgery prevention printing. Anthraquinone derivatives can also be used as dichroic pigments used for host-guest liquid crystal elements or polarizing films.

The expression at least one used in the present specification refers to one or more of desired options. As an example, the expression at least one used in the present specification may refer to one option only or both of two options if the number of options is two. As another example, the expression at least one used in the present specification may refer to one option only or a combination of any two or more options if the number of options is three or more.

### (First Embodiment)

An anthraquinone derivative according to a first embodiment will be described. The anthraquinone derivative according to the first embodiment is a compound expressed by the following Formula (1-1).

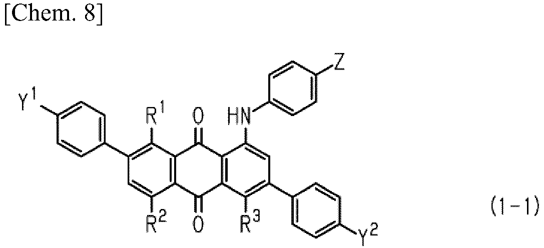

In Formula (1-1), R¹, R² and R³ each independently represent an amino group or hydroxyl group. In Formula (1-1), at least one of Y¹ and Y² represents an electron-donating group; if only one of Y¹ and Y² is an electron-donating group, the other of Y¹ and Y² is a hydrogen atom, alkyl group having 1 to 10 carbon atoms, alkoxy group having 1 to 10 carbon atoms, aryl group, or cyclohexyl group; and the aryl group and the cyclohexyl group may have substituents. The substituents are preferably alkyl groups having 1 to 10 carbon atoms or cyclic hydrocarbon groups. The cyclic hydrocarbon groups are preferably cyclohexyl groups or phenyl groups.

The electron-donating group is preferably an alkylamino group, amino group, piperidyl group, acetamide group, or hydroxyl group. The alkylamino group may have one or two alkyl groups. The alkyl group of the alkylamino group preferably has 1 to 10 carbon atoms. If both of Y¹ and Y² are electron-donating groups, Y¹ and Y² may be the same as or different from each other.

In Formula (1-1), Z represents a hydrogen atom, alkyl group having 1 to 10 carbon atoms, alkoxy group having 1 to 10 carbon atoms, cyano group, nitro group, halogen atom, amino group, or alkylamino group. The halogen atom is preferably F, Cl or Br. The alkylamino group may have one or two alkyl groups. The alkyl group of the alkylamino group preferably has 1 to 10 carbon atoms.

The effects of the anthraquinone derivative according to the present embodiment will be described. The types of the substituents in the anthraquinone skeleton affect the absorption wavelength of the anthraquinone derivative. For the four α-positions, the anthraquinone derivative of the first embodiment has a substituted or unsubstituted anilino group at the 1st position and amino groups or hydroxyl groups at the 4th, 5th and 8th positions. Furthermore, the anthraquinone derivative has substituents at only two of the four β-positions, i.e., 3rd and 7th positions, and these substituents are substituted or unsubstituted phenyl groups. Anthraquinone derivatives having such a structure have a maximum absorption wavelength in the wavelength range of 600 nm or greater, and thus can be used as cyanide pigments.

It is considered that photodegradation in an anthraquinone derivative is induced in part by photoreduction occurring in the anthraquinone skeleton, using resins and other materials surrounding the anthraquinone derivative as hydrogen sources. Conventional anthraquinone derivatives are known to have a structure in which functional groups such as phenyl groups are bonded to the β-positions via ether bonds. In contrast, in the anthraquinone derivative of the present embodiment, phenyl groups are directly bonded to the carbon atoms of the anthraquinone skeleton at the β-positions. Such a structure can suppress the progress of photoreduction reaction, and therefore high light resistance can be achieved.

Specifically, it is considered that if phenyl groups are directly bonded at the β-positions, the molecular structures may be less likely to rotate at the bonding sites of the β-positions and that it may become difficult for the anthraquinone skeleton to achieve a stable structure after photoreduction, compared to the case where ether bonds are present at the β-positions, and therefore the progress of photoreduction reaction may be suppressed.

Pigments are desired to have high coloring power, i.e., high absorbance. The higher the coloring power, the less the amount of pigment required to be mixed, which is beneficial from the perspective of cost. Regarding the absorbance of anthraquinone derivatives, it is known that the greater the overlap of the molecular orbital distributions between the ground state and the excited state, the higher the absorbance. The molecular orbitals in the ground state are easily affected by the molecular structures of the substituents, etc. and tend to expand even more than in the excited state. In this regard, the anthraquinone derivative of the first embodiment has a para-substituted phenyl group containing an electron-donating group at at least one β-position. Due to the para substituent in this phenyl group being an electron-donating group, expansion of the molecular orbitals in the ground state is suppressed, increasing the absorbance coefficient, resultantly enhancing the coloring power of the anthraquinone derivative.

In the anthraquinone derivative of the first embodiment, it is preferable that R¹ and R³ are hydroxyl groups and R² is an amino group. Such a compound is expressed by the following Formula (1-2).

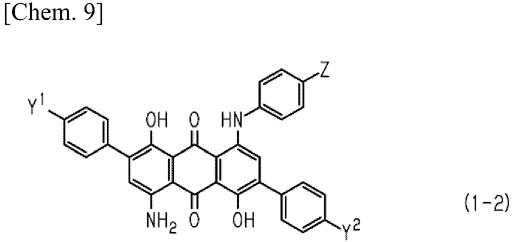

In Formula (1-2), Y¹, Y² and Z are defined as in Formula (1-1). As long as anthraquinone derivatives have a structure expressed by Formula (1-2), they are easy to synthesize. While the presence of a hydroxyl group at an α-position tends to cause the photoreduction mentioned above, the anthraquinone derivative of the present embodiment can suppress the photoreduction reaction as a result of the structure in which phenyl groups are directly bonded at the β-positions as described above. Therefore, the benefits of this structure can be enjoyed, while eliminating the drawbacks of the structure having a hydroxyl group at an α-position.

Furthermore, in the anthraquinone derivative of the first embodiment, both Y¹ and Y² are preferably electron-donating groups. This allows for greater control over the molecular orbitals involved in the photoexcitation mentioned above so as to increase the absorbance coefficient, and therefore higher coloring power can be achieved. In particular, Y¹ and Y² are each preferably an alkylamino group, amino group, or piperidyl group. These functional groups, which have high electron-donating properties, allow for greater control over molecular orbitals involved in the photoexcitation. Therefore, higher coloring power can be achieved.

Compounds in which Y¹ and Y² are each an alkylamino group or amino group are expressed by the following Formula (1-3). Compounds in which Y¹ and Y² are each a piperidyl group are expressed by the following Formula (1-4).

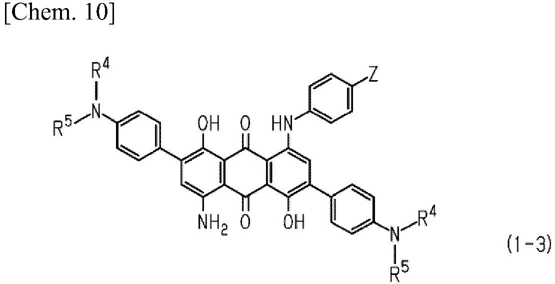

In Formula (1-3), Z is defined as in Formula (1-1). R⁴ and R⁵ each independently represent a hydrogen atom or an alkyl group having 1 to 10 carbon atoms.

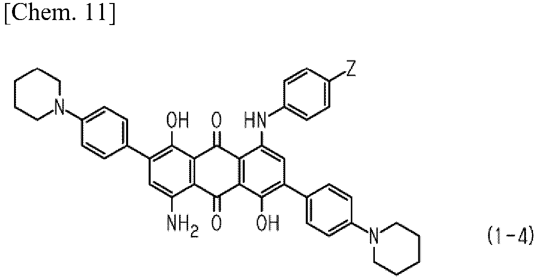

In Formula (1-4), Z is defined as in Formula (1-1).

The anthraquinone derivative of the first embodiment can be produced, for example, by introducing substituents to the anthraquinone skeleton using 1,5-dihydroxy-4,8-dinitroanthraquinone or 1,5-diamino-4,8-dinitroanthraquinone as a starting material. Substituents can be introduced using any known method. For example, desired substituents can be introduced using reduction or substituent conversion of the nitro group, or bromination at the β-positions and conversion of the bromo groups.

### (Second Embodiment)

An anthraquinone derivative according to a second embodiment will be described. The anthraquinone derivative according to the second embodiment is a compound expressed by the following Formula (2-1).

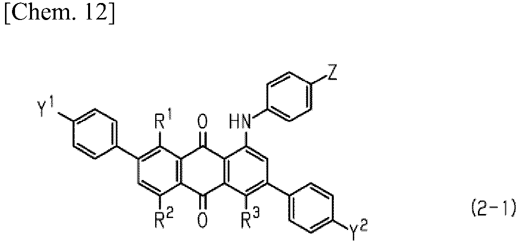

In Formula (2-1), R¹, R² and R³ each independently represent an amino group or hydroxyl group. Y¹ and Y² each independently represent a hydrogen atom, alkyl group having 1 to 10 carbon atoms, alkoxy group having 1 to 10 carbon atoms, aryl group, cyano group, nitro group, halogen atom, amino group, alkylamino group, or cyclohexyl group, and the aryl group and the cyclohexyl group may have substituents.

The halogen atom is preferably F, Cl or Br. The alkylamino group may have one or two alkyl groups. The alkyl group of the alkylamino group preferably has 1 to 10 carbon atoms. The substituents on the aryl group or the cyclohexyl group are preferably alkyl groups having 1 to 10 carbon atoms or cyclic hydrocarbon groups. The cyclic hydrocarbon groups are preferably cyclohexyl groups or phenyl groups.

In Formula (2-1), Z represents an electron-withdrawing group. The electron-withdrawing group is preferably a cyano group, aldehyde group, carboxylic acid-derived ester group, acetyl group, sulfo group, nitro group, or halogenated alkyl group. The halogen contained in the halogenated alkyl group is preferably F, Cl or Br. The halogenated alkyl group preferably has 1 to 10 carbon atoms.

For the four α-positions, the anthraquinone derivative of the second embodiment has a substituted anilino group at the 1st position and amino groups or hydroxyl groups at the 4th, 5th and 8th positions. Furthermore, the anthraquinone derivative of the second embodiment has two substituted or unsubstituted phenyl groups at the β-positions. Accordingly, similarly to the first embodiment, the anthraquinone derivative of the second embodiment also has a maximum absorption wavelength in the wavelength range of 600 nm or greater, and can be used as a cyanide pigment.

In the anthraquinone derivative of the second embodiment also, phenyl groups are directly bonded to the carbon atoms constituting the anthraquinone skeleton at the β-positions. Similarly to the first embodiment, such a structure can suppress the progress of photoreduction reaction, resulting in achieving high light resistance.

Furthermore, the anthraquinone derivative of the second embodiment has a para-substituted anilino group containing an electron-withdrawing group at the α-position. Due to the para substituent in the anilino group being an electron-withdrawing group, the electron density of the aromatic ring of the anilino group decreases, and expansion of the molecular orbitals toward the anilino group is suppressed. This also controls the molecular orbitals involved in photoexcitation to increase the absorbance coefficient, resultantly enhancing coloring power of the anthraquinone derivative.

If the anthraquinone derivative has phenyl groups with electron-donating groups as para substituents at the β-positions, as in the first embodiment, the effect of improving coloring power can be further enhanced.

In the anthraquinone derivative of the second embodiment, it is preferable that R¹ and R³ are hydroxyl groups and R² is an amino group. Such a compound is expressed by the following Formula (2-2).

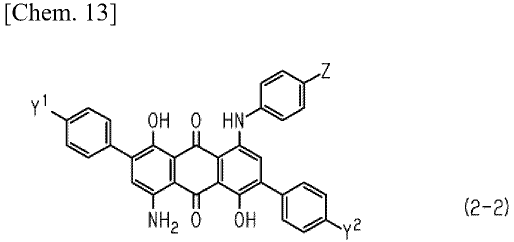

In Formula (2-2), Y¹, Y² and Z are defined as in Formula (2-1). As long as anthraquinone derivatives have a structure expressed by Formula (2-2), they are easy to synthesize. While the presence of a hydroxyl group at an α-position tends to cause the photoreduction mentioned above, the anthraquinone derivative of the present embodiment can suppress the photoreduction reaction as a result of the structure in which phenyl groups are directly bonded at the β-positions as described above. Therefore, the benefits of this structure can be enjoyed, while eliminating the drawbacks of the structure having a hydroxyl group at an α-position.

The anthraquinone derivative of the second embodiment is preferably a compound expressed by the following Formula (2-3).

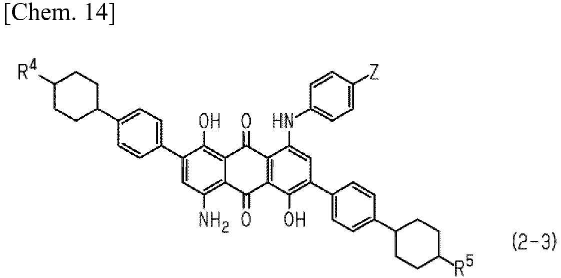

In Formula (2-3), Z is defined as in Formula (2-1). R⁴ and R⁵ each independently represent a hydrogen atom, alkyl group having 1 to 10 carbon atoms, or cyclic hydrocarbon group. Many liquid crystal compounds contain a cyclohexane ring, and phenylcyclohexane-based liquid crystals may also be suitably used. As long as anthraquinone derivatives have a structure expressed by Formula (2-3), they are readily mixed with such liquid crystal materials and can therefore be suitably used as dichroic pigments together with the liquid crystal materials.

The anthraquinone derivative of the second embodiment can be produced, for example, by introducing substituents to the anthraquinone skeleton using 1,5-dihydroxy-4,8-dinitroanthraquinone or 1,5-diamino-4,8-dinitroanthraquinone as a starting material. Substituents can be introduced using any known method. For example, desired substituents can be introduced using reduction or substituent conversion of the nitro group, or bromination at the β-positions and conversion of the bromo groups.

### (Third Embodiment)

An anthraquinone derivative according to a third embodiment will be described. The anthraquinone derivative according to the third embodiment is a compound expressed by the following Formula (3-1).

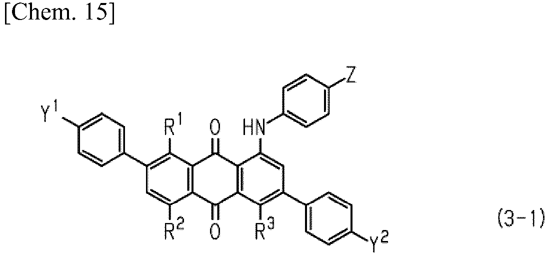

In Formula (3-1), R¹, R² and R³ each independently represent an amino group or a hydroxyl group. In Formula (3-1), at least either of Y¹ and Y² is a cyclohexyl group which may have substituents. The substituent on the cyclohexyl group is preferably an alkyl group having 1 to 10 carbon atoms or cyclic hydrocarbon group. The cyclic hydrocarbon group is preferably a cyclohexyl group or phenyl group.

If only either of Y¹ and Y² is a cyclohexyl group, the other of Y¹ and Y² may be a hydrogen atom, alkyl group having 1 to 10 carbon atoms, alkoxy group having 1 to 10 carbon atoms, aryl group, cyano group, nitro group, halogen atom, amino group, or alkylamino group, and the aryl group may have substituents. The halogen atom is preferably F, Cl or Br. The alkylamino group may have one or two alkyl groups. The alkyl group of the alkylamino group preferably has 1 to 10 carbon atoms. The substituent on the aryl group is preferably an alkyl group having 1 to 10 carbon atoms or cyclic hydrocarbon group.

In formula (3-1), Z represents a hydrogen atom, alkyl group having 1 to 10 carbon atoms, alkoxy group having 1 to 10 carbon atoms, cyano group, nitro group, halogen atom, amino group, or alkylamino group. The halogen atom is preferably F, Cl or Br. The alkylamino group may have one or two alkyl groups. The alkyl group of the alkylamino group preferably has 1 to 10 carbon atoms.

In the anthraquinone derivative of the third embodiment, the substituents at the α-positions are the same as those of the first embodiment. Furthermore, similarly to the first embodiment, the anthraquinone derivative of the third embodiment also has two substituted or unsubstituted phenyl groups at the β-positions. Accordingly, the anthraquinone derivative of the third embodiment also has a maximum absorption wavelength in the wavelength range of 600 nm or greater, and therefore can be used as a cyanide pigment.

In the anthraquinone derivative of the third embodiment also, phenyl groups are directly bonded to the carbon atoms constituting the anthraquinone skeleton at the β-positions. Similarly to the first and second embodiments, such a structure can suppress the progress of photoreduction reaction, resulting in achieving high light resistance.

Furthermore, the anthraquinone derivative of the third embodiment has a para-substituted phenyl group at at least one β-position, and the para substituent in this phenyl group is a substituted or unsubstituted cyclohexyl group. The para substituents at the β-positions allow for control of the molecular orbitals involved in photoexcitation, and if these substituents are cyclohexyl groups, absorption in the short-wavelength range shifts to the UV region. Consequently, blue components increase in reflected light, and therefore the pigment will have a stronger blue hue. Having a stronger blue hue is beneficial for cyanide pigments which are used corresponding to the three primary colors. Therefore, the anthraquinone derivative of the third embodiment can be suitably used as a cyanide pigment.

In the anthraquinone derivative of the third embodiment, it is preferable that R¹ and R³ are hydroxyl groups and R² is an amino group. Such a compound is expressed by the following Formula (3-2).

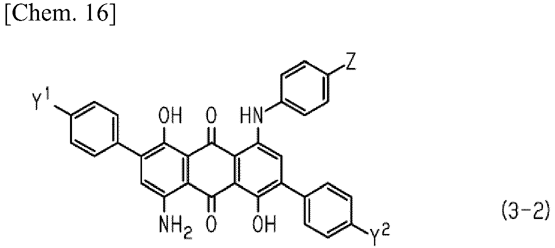

In Formula (3-2), Y¹, Y² and Z are defined as in Formula (3-1). As long as anthraquinone derivatives have a structure expressed by Formula (3-2), they are easy to synthesize. While the presence of a hydroxyl group at an α-position tends to cause the photoreduction mentioned above, the anthraquinone derivative of the present embodiment can suppress the photoreduction reaction as a result of the structure in which phenyl groups are directly bonded at the β-positions as described above. Therefore, the benefits of this structure can be enjoyed, while eliminating the drawbacks of the structure having a hydroxyl group at an α-position.

The anthraquinone derivative of the third embodiment is preferably a compound expressed by the following Formula (3-3).

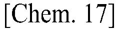

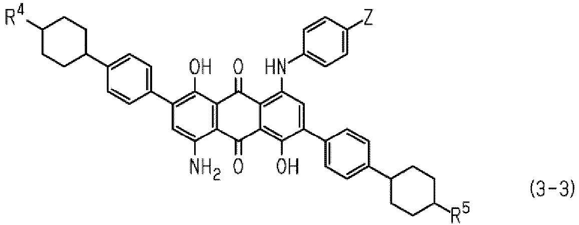

In Formula (3-3), Z is defined as in Formula (3-1). R⁴ and R⁵ each independently represent a hydrogen atom, alkyl group having 1 to 10 carbon atoms, or cyclic hydrocarbon group. In anthraquinone derivatives expressed by Formula (3-3), the para substituents in the phenyl groups at the two β-positions are both substituted or unsubstituted cyclohexyl groups. This results in a more pronounced suppression of absorption in the short-wavelength range, achieving a stronger blue hue.

If anthraquinone derivatives have an anilino group with an electron-withdrawing group as a para substituent at an α-position, as in the second embodiment, the effect of improving coloring power can be achieved.

The anthraquinone derivative of the third embodiment can be produced, for example, by introducing substituents to the anthraquinone skeleton using 1,5-dihydroxy-4,8-dinitroanthraquinone or 1,5-diamino-4,8-dinitroanthraquinone as a starting material. Substituents can be introduced using any known method. For example, desired substituents can be introduced using reduction or substituent conversion of the nitro group, or bromination at the β-positions and conversion of the bromo groups.

### (Fourth Embodiment)

An anthraquinone derivative according to a fourth embodiment will be described. The anthraquinone derivative of the fourth embodiment is a compound expressed by the following Formula (4-1) that is a compound in which a total energy difference ΔE before and after the reaction expressed by the following Reaction Formula (4-2) is -16 kcal/mol or more.

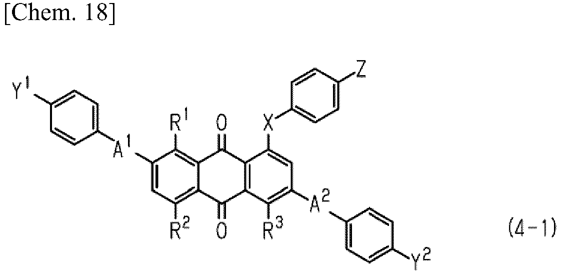

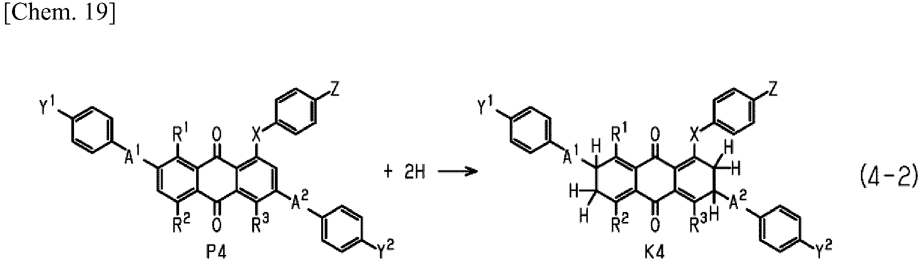

In Formula (4-1), X represents -NH- or a sulfur atom. In Formula (4-1), R¹, R² and R³ each independently represent an amino group or hydroxyl group. In Formula (4-1), A¹ and A² each independently represent a direct bond, oxygen atom, sulfur atom, or -NH-. It should be noted that A¹ and A² each representing a direct bond refers to the substituted or unsubstituted phenyl group being directly bonded to the carbon atom constituting the anthraquinone skeleton at the β-position of the anthraquinone derivative.

In Formula (4-1), Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents. These substituents are preferably alkyl groups having 1 to 10 carbon atoms or cyclic hydrocarbon groups. The cyclic hydrocarbon groups are preferably cyclohexyl groups or phenyl groups. The halogen atom and the halogen atom contained in the halogenated alkyl group are preferably F, Cl or Br. The alkylamino group may have one or two alkyl groups. The alkyl group of the alkylamino group preferably has 1 to 10 carbon atoms.

Reaction Formula (4-2) shows the photoreduction reaction of the anthraquinone derivative expressed by Formula (4-1). In this reaction, an anthraquinone derivative P4 is photoreduced using the surrounding resins and other materials as hydrogen sources to produce a hydrogen adduct K4, a compound in which hydrogen is added to the anthraquinone skeleton.

The total energy difference ΔE is a value obtained by subtracting a total energy Ep of the anthraquinone derivative P4 from a total energy Ek of the hydrogen adduct K4 (ΔE=Ek-Ep).

The energies Ep and Ek of the anthraquinone derivative P4 and the hydrogen adduct K4, respectively, are calculated from a quantum chemical calculation using a density functional theory (DFT). B3LYP is used as the functional and 6-31G(d) is used for the basis functions. Such a quantum chemical calculation can be performed using a general-purpose quantum chemical calculation program such as Gaussian or GAMESS. The energies Ep and Ek each represent the total energy of molecules in an optimized structure obtained from the quantum chemical calculation.

The effects of the anthraquinone derivative according to the fourth embodiment will be described. Anthraquinone derivatives having substituents as shown in Formula (4-1) have a maximum absorption wavelength in the wavelength range of 580 nm or greater and 830 nm or less, and therefore can be used as cyanide pigments.

In the anthraquinone derivative of the fourth embodiment, the total energy difference ΔE is -16 kcal/mol or more. Photodegradation of anthraquinone derivatives is induced in part by the reaction expressed by the above Reaction Formula (4-2). The larger the total energy difference ΔE, the less likely the above reaction is to occur. If the total energy difference ΔE is -16 kcal/mol or more, good light resistance can be achieved. In order to achieve higher light resistance, the total energy difference ΔE is preferably -14 kcal/mol or more.

A 1st example of a suitable anthraquinone derivative in the fourth embodiment is a compound, among the compounds expressed by Formula (4-1) above, in which R¹, R²and R³ are amino groups. With such a compound, the total energy difference ΔE becomes larger, and therefore higher light resistance can be achieved. Anthraquinone derivatives with a hydroxyl group at an α-position are considered to be more prone to photoreduction reaction, and therefore it is considered that degradation due to photoreduction reaction can be effectively suppressed if R¹, R² and R³ are all amino groups.

A 2nd example of a suitable anthraquinone derivative in the fourth embodiment is a compound, among the compounds expressed by Formula (4-1) above, in which R¹ and R³ are hydroxyl groups, R² is an amino group, and A¹ and A² are direct bonds. With such a compound, the total energy difference ΔE becomes larger, and therefore higher light resistance can be achieved. In such a structure, as described in the first embodiment, the direct bonds of the phenyl groups at the β-positions are considered to effectively suppress degradation caused by photoreduction reaction, as a result of the three-dimensional structure of the molecules.

The anthraquinone derivative of the fourth embodiment may be any of the compounds described in the first to third embodiments, as long as the total energy difference ΔE is -16 kcal/mol or more. Even if the compounds of the first to third embodiments are different from the compound expressed by Formula (4-1), good light resistance can be achieved as long as the total energy difference ΔE is -16 kcal/mol or more.

### (Fifth Embodiment)

An anthraquinone derivative according to a fifth embodiment will be described. The anthraquinone derivative of the fifth embodiment is a compound expressed by the following Formula (5-1) and has a 10% weight loss temperature of 350°C or higher in thermogravimetric measurements.

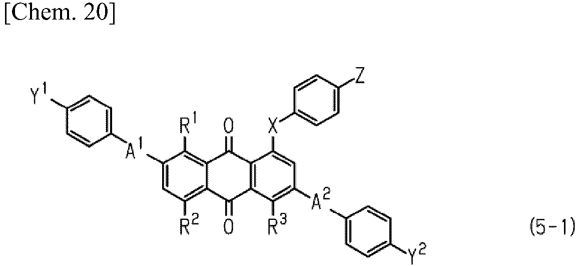

In Formula (5-1), X represents -NH- or a sulfur atom. In Formula (5-1), R¹, R² and R³ each independently represent an amino group or hydroxyl group. In Formula (5-1), A¹ and A² each independently represent a direct bond, oxygen atom, sulfur atom, or -NH-. It should be noted that A¹ and A² each representing a direct bond refers to the substituted or unsubstituted phenyl group being directly bonded to the carbon atom constituting the anthraquinone skeleton at the β-position of the anthraquinone derivative.

In Formula (5-1), Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents. These substituents are preferably alkyl groups having 1 to 10 carbon atoms or cyclic hydrocarbon groups. The cyclic hydrocarbon groups are preferably cyclohexyl groups or phenyl groups. The halogen atom and the halogen atom contained in the halogenated alkyl group are preferably F, Cl or Br. The alkylamino group may have one or two alkyl groups. The alkyl group of the alkylamino group preferably has 1 to 10 carbon atoms.

The above thermogravimetric measurements are performed under the conditions of: gas flow rate: 200 mL/min, and heating rate: 10°C/min. Air is used as the gas.

The effects of the anthraquinone derivative according to the fifth embodiment will be described. Anthraquinone derivatives having substituents as shown in Formula (5-1) have a maximum absorption wavelength in the wavelength range of 580 nm or greater and 830 nm or less, and therefore can be used as cyanide pigments.

The anthraquinone derivative of the fifth embodiment has a 10% weight loss temperature of 350°C or higher in the above thermogravimetric measurements. When exposed to high heat, anthraquinone derivatives decompose, generating radicals, and these radicals further accelerate decomposition and other reactions. Since photodegradation of anthraquinone derivatives is also induced in part by radicals generated from the surroundings or from the anthraquinone derivatives themselves, anthraquinone derivatives that are less likely to cause thermal decomposition, i.e., anthraquinone derivatives with a high 10% weight loss temperature, tend to have high light resistance also. Good light resistance can be achieved if the 10% weight loss temperature is 350°C or higher. In order to achieve even higher light resistance, the 10% weight loss temperature is preferably 365°C or higher.

A 1st example of a suitable anthraquinone derivative in the fifth embodiment is a compound, among the compounds expressed by Formula (5-1) above, in which R² is an amino group. Such compounds are easy to synthesize.

A 2nd example of a suitable anthraquinone derivative in the fifth embodiment is a compound, among the compounds expressed by Formula (5-1) above, in which R¹ and R³ are hydroxyl groups and R² is an amino group. Such compounds are easy to synthesize.

A 3rd example of a suitable anthraquinone derivative in the fifth embodiment is a compound, among the compounds expressed by Formula (5-1) above, in which R¹, R² and R³ are amino groups. With such compounds, the 10% weight loss temperature becomes higher, and therefore higher light resistance can be achieved accordingly.

A 4th example of a suitable anthraquinone derivative in the fifth embodiment is a compound, among the compounds expressed by Formula (5-1) above, in which R² is an amino group and A¹ and A² are direct bonds. With such compounds, the 10% weight loss temperature becomes higher, and therefore higher light resistance can be achieved accordingly.

The anthraquinone derivative of the fifth embodiment may be any of the compounds described in the first to third embodiments, as long as the 10% weight loss temperature is 350°C or higher. Even if the compounds of the first to third embodiments are different from the compound expressed by Formula (5-1), good light resistance can be achieved as long as the 10% weight loss temperature is 350°C or higher.

### (Sixth embodiment)

Pigments are desired to have high coloring power, i.e., high absorbance. The coloring power of anthraquinone derivatives depends on the types and positions of the substituents they possess. Since there are many options for substituents that can be introduced into anthraquinone derivatives, coloring power greatly differs between anthraquinone derivatives, and therefore many compounds of the conventional anthraquinone derivatives can have low coloring power.

The higher the coloring power, the less amount of pigment can be mixed, and this is beneficial from the perspective of cost. Furthermore, anthraquinone derivatives have high molecular planarity and are less likely to increase solubility, and therefore limit increase of color intensity even if the mixing amount is increased. Therefore, improving coloring power is an important issue. The anthraquinone derivative of a sixth embodiment aims to improve coloring power.

The anthraquinone derivative according to the sixth embodiment will be described. In the anthraquinone derivative of the sixth embodiment, the magnitude of the transition dipole moment calculated using the time-dependent density functional theory (TDDFT) is 3.30 D or more and 5.00 D or less.

The transition dipole moment of an anthraquinone derivative is an electric dipole moment generated during electron transitions in a vacuum related to light absorption. When calculating a transition dipole moment, B3LYP is used as the functional and 6-31G(d) is used for the basis functions. Such a quantum chemical calculation can be performed using a general-purpose quantum chemical calculation program such as Gaussian or GAMESS.

Since the molar absorbance coefficient is proportional to the square of the transition dipole moment, a larger transition dipole moment tends to achieve a higher absorbance. If the magnitude of the transition dipole moment is 3.30 D or more, sufficient absorbance can be achieved, resulting in good coloring power.

The types and positions of substituents on an anthraquinone derivative are related not only to the magnitude of the transition dipole moment but also to the magnitude of the maximum absorption wavelength. If the magnitude of the transition dipole moment is 3.30 D or more, both of absorbance and maximum absorption wavelength can be achieved at a favorable level.

If the magnitude of the transition dipole moment is 5.00 D or less, complexity of the substituent arrangement or structure can be suppressed, making synthesis easier.

The anthraquinone derivative of the sixth embodiment is preferably a compound expressed by the following Formula (6-1).

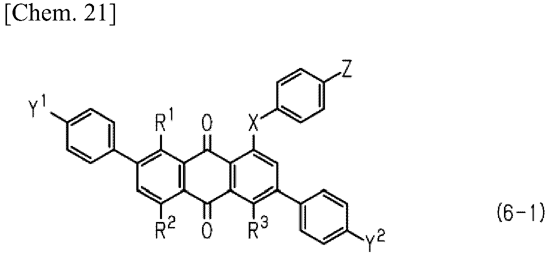

In Formula (6-1), X represents -NH- or a sulfur atom. In Formula (6-1), R¹, R² and R³ each independently represent an amino group or hydroxyl group.

In Formula (6-1), Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents. These substituents are preferably alkyl groups having 1 to 10 carbon atoms or cyclic hydrocarbon groups. The cyclic hydrocarbon groups are preferably cyclohexyl groups or phenyl groups. The halogen atom and the halogen atom contained in the halogenated alkyl group are preferably F, Cl or Br. The alkylamino group may have one or two alkyl groups. The alkyl group of the alkylamino group preferably has 1 to 10 carbon atoms.

As long as anthraquinone derivatives have substituents as shown in Formula (6-1) above, the transition dipole moment can be increased, and the maximum absorption wavelength can also be increased. Specifically, the maximum absorption wavelength is achieved in the wavelength range of 580 nm or greater and 830 nm or less. Therefore, they can be used as cyanide pigments with high coloring power. Furthermore, since phenyl groups are directly bonded at the β-positions, light resistance can also be improved.

A 1st example of a suitable anthraquinone derivative is a compound, among the compounds expressed by Formula (6-1) above, in which R¹ and R³ are hydroxyl groups and R² is an amino group. Such compounds are easy to synthesize.

A 2nd example of a suitable anthraquinone derivative is a compound, among the compounds expressed by Formula (6-1) above, in which R² is an amino group and X is a sulfur atom. Such compounds have both a structure having an amino group or hydroxyl group at an α-position and phenyl groups directly bonded at the β-positions, which contributes significantly to the shifting of the maximum absorption wavelength toward longer wavelengths, and a structure having a phenylthio group at an α-position, which contributes less to the shifting of the maximum absorption wavelength toward longer wavelengths. Therefore, while achieving a maximum absorption wavelength of 580 nm or greater that can be used as a cyanide pigment, excessive increase of the maximum absorption wavelength can be suppressed. Specifically, the maximum absorption wavelength can be in the range of 580 nm or greater and 670 nm or less. Thus, an excellent blue hue can be produced.

The anthraquinone derivative of the sixth embodiment may be the anthraquinone derivative of the first embodiment. Such compounds can achieve high light resistance, and at the same time can enhance coloring power as a result of the effects of the para-substituted phenyl groups having electron-donating groups at the β-positions.

The anthraquinone derivative of the sixth embodiment may be the anthraquinone derivative of the second embodiment. Such compounds can achieve high light resistance, and at the same time enhance coloring power as a result of the effects of the para-substituted anilino group having an electron-withdrawing group at the α-position.

The anthraquinone derivative of the sixth embodiment may be the anthraquinone derivative of the third embodiment. Such compounds can achieve high light resistance, and at the same time can achieve a strong blue hue as a result of the effects of the para-substituted phenyl groups having cyclohexyl groups at the β-positions.

### (Seventh embodiment)

The anthraquinone derivative of a seventh embodiment aims to improve coloring power, similarly to the sixth embodiment.

The anthraquinone derivative according to the seventh embodiment will be described. The anthraquinone derivative of the seventh embodiment is a compound expressed by the following Formula (7-1) and satisfying the conditions for the molecular orbital coefficient described later.

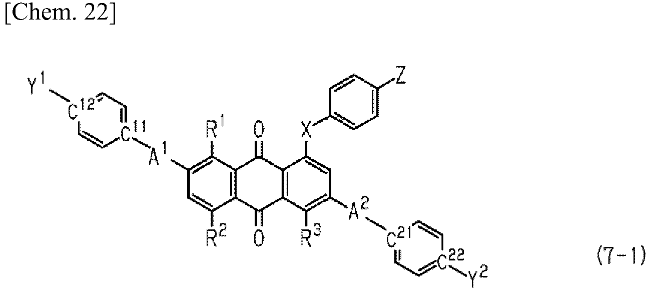

In Formula (7-1), C¹¹, C¹², C²¹ and C²² are carbon atoms. In Formula (7-1), X represents -NH- or a sulfur atom. In Formula (7-1), R¹, R² and R³ each independently represent an amino group or hydroxyl group. In Formula (7-1), A¹ and A² each independently represent a direct bond, oxygen atom, sulfur atom, or -NH-. It should be noted that A¹ and A² each representing a direct bond refers to the substituted or unsubstituted phenyl group being directly bonded to the carbon atom constituting the anthraquinone skeleton at the β-position of the anthraquinone derivative.

In Formula (7-1), Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents. These substituents are preferably alkyl groups having 1 to 10 carbon atoms or cyclic hydrocarbon groups. The cyclic hydrocarbon groups are preferably cyclohexyl groups or phenyl groups. The halogen atom and the halogen atom contained in the halogenated alkyl group are preferably F, Cl or Br. The alkylamino group may have one or two alkyl groups. The alkyl group of the alkylamino group preferably has 1 to 10 carbon atoms.

In the anthraquinone derivative of the seventh embodiment, regarding the coefficient of a highest occupied molecular orbital (HOMO) determined by the quantum chemical calculation using density functional theory (DFT), an average Mv of square roots of the square sums of the coefficients corresponding to the orbitals of the carbon atoms of C¹¹, C¹², C²¹ and C²² is 0.03 or more and 0.2 or less. B3LYP is used as the functional and 6-31G(d) is used for the basis functions.

Specifically, let us assume that the square root of the square sum of the molecular orbital coefficients for C¹¹ is M₁₁, the square root of the square sum of the molecular orbital coefficients for C¹² is M₁₂, the square root of the square sum of the molecular orbital coefficients for C²¹ is M₂₁, and the square root of the square sum of the molecular orbital coefficients for C²² is M₂₂. In this case, an average of M₁₁, M₁₂, M₂₁ and M₂₂ is the average Mv.

The quantum chemical calculation can be performed using a general-purpose quantum chemical calculation program such as Gaussian or GAMESS.

The effects of the anthraquinone derivative according to the seventh embodiment will be described. Anthraquinone derivatives having substituents as shown in Formula (7-1) have a maximum absorption wavelength in the wavelength range of 580 nm or greater and 830 nm or less, and therefore can be used as cyanide pigments.

Generally, the electron transition exhibiting a maximum absorption is the electron transition from HOMO to a lowest unoccupied molecular orbital (LUMO). It is considered that a greater overlap between the molecular orbitals HOMO and LUMO may lead to a higher probability of electron transition, and that therefore a higher absorbance is achieved. As a result of diligent studies, the inventors have found that molecular orbitals HOMO and LUMO tend to expand in an anthraquinone derivative.

Specifically, the type and arrangement of substituents have little effect on the molecular orbitals LUMO of an anthraquinone derivative, and the molecular orbitals LUMO tend to concentrate around the anthraquinone skeleton. On the other hand, the molecular orbitals HOMO vary greatly depending on the type and arrangement of substituents. In the structure expressed by Formula (7-1) above, the molecular orbitals HOMO tend to expand toward the α-position to which an anilino group or phenylthio group is bonded, i.e., toward the substituent containing X.

Therefore, it is considered that if the molecular orbitals HOMO are configured to expand toward the substituents at the β-positions, i.e., to expand over the benzene rings contained in the substituents at the β-positions, the molecular orbitals HOMO will not excessively expand toward the α-positions, but will expand in a balanced manner around the anthraquinone skeleton, thereby increasing the overlap between the molecular orbitals HOMO and LUMO.

The fact that the molecular orbitals HOMO expand over the benzene rings at the β-positions can be expressed by the coefficients of the orbitals on the carbon atoms contained in the benzene rings. In other words, the larger the average Mv, the more the molecular orbitals expand over the benzene rings at the β-positions. If the average Mv is 0.03 or more, the overlap between the molecular orbitals HOMO and LUMO increases to an extent of achieving good absorbance at the maximum absorption wavelength, and high coloring power is achieved. If the average Mv is 0.2 or less, complexity of the substituent arrangement and structure can be suppressed, making synthesis of the anthraquinone derivative easier. Furthermore, this also suppresses the molecular orbitals HOMO from excessively expanding toward the β-position substituents and from reducing the overlap between the molecular orbitals HOMO and LUMO.

A 1st example of a suitable anthraquinone derivative in the seventh embodiment is a compound, among the compounds expressed by Formula (7-1) above, in which R¹ and R³ are hydroxyl groups and R² is an amino group. Such compounds are easy to synthesize.

A 2nd example of a suitable anthraquinone derivative in the seventh embodiment is an anthraquinone derivative, among the compounds expressed by Formula (7-1) above, in which R¹ and R³ are hydroxyl groups, R² is an amino group, and A¹ and A² are direct bonds. Such compounds not only facilitate synthesis, but also enhance light resistance. The average Mv can also be increased.

The anthraquinone derivative of the seventh embodiment may be any of the compounds described in the first to third embodiments, as long as the average Mv is 0.03 or more and 0.2 or less. Even if the compounds of the first to third embodiments are different from the compound expressed by Formula (7-1), good coloring power can be achieved as long as the average Mv is 0.03 or more and 0.2 or less.

In order to increase the average Mv within the range of 0.03 or more and 0.2 or less in an anthraquinone derivative, it is preferable that substituted or unsubstituted phenyl groups are directly bonded at the β-positions. Similarly, in order to increase the average Mv within the above range, it is preferable that the anthraquinone derivative has para-substituted phenyl groups at the β-positions and that the para substituents of the phenyl groups are electron-donating groups. In other words, if the anthraquinone derivative of the first embodiment is used, good coloring power can be achieved. Similarly, in order to increase the average Mv in the above range, it is preferable that the anthraquinone derivative has a para-substituted anilino group at the α-position and that the para substituent of the anilino group is an electron-withdrawing group. In other words, if the anthraquinone derivative of the second embodiment is used, good coloring power can be achieved.

### (Eighth embodiment)

The anthraquinone derivative of an eighth embodiment aims to improve light resistance, similarly to the first embodiment.

The anthraquinone derivative according to the eighth embodiment is a compound expressed by the following Formula (8-1).

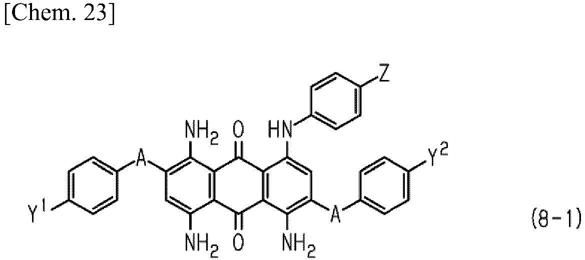

In Formula (8-1), A represents a direct bond, oxygen atom, or -NH-. It should be noted that A representing a direct bond refers to the substituted or unsubstituted phenyl group being directly bonded to the carbon atom constituting the anthraquinone skeleton at the β-position of the anthraquinone derivative.

In Formula (8-1), Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, aryl group, piperidyl group, or cyclohexyl group, and the aryl group and the cyclohexyl group may have substituents. These substituents are preferably alkyl groups having 1 to 10 carbon atoms or cyclic hydrocarbon groups. The cyclic hydrocarbon groups are preferably cyclohexyl groups or phenyl groups. The halogen atom and the halogen atom contained in the halogenated alkyl group are preferably F, Cl or Br. The alkylamino group may have one or two alkyl groups. The alkyl group of the alkylamino group preferably has 1 to 10 carbon atoms.

The types of the substituents in the anthraquinone skeleton affect the absorption wavelength of the anthraquinone derivative. The anthraquinone derivative of the present embodiment has one substituted or unsubstituted anilino group and three amino groups at the α-positions. Anthraquinone derivatives having such a structure have a maximum absorption wavelength in the wavelength range of 600 nm or greater, and thus can be used as cyanide pigments.

It is considered that photodegradation in an anthraquinone derivative is induced in part by photoreduction occurring at the anthraquinone skeleton, using resins and other materials surrounding the anthraquinone derivative as hydrogen sources. Anthraquinone derivatives having a hydroxyl group at an α-position are more likely to cause such photoreduction. Also, changes in chemical structure caused by radicals generated from hydroxyl groups also lead to photodegradation. In this regard, the anthraquinone derivative of the present embodiment, which has no hydroxyl group at an α-position, suppresses photodegradation that would be caused by the hydroxyl group. Therefore, if the anthraquinone derivative of the present embodiment is used, good light resistance can be achieved.

In the anthraquinone derivative of the present embodiment, A is preferably an oxygen atom or direct bond. The compound in which A is an oxygen atom is expressed by the following Formula (8-2), and the compound in which A is a direct bond is expressed by the following Formula (8-3).

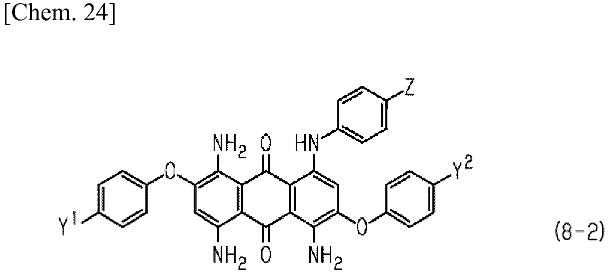

In Formula (8-2), Y¹, Y² and Z are defined as in Formula (8-1). The anthraquinone derivative expressed by Formula (8-2) can enhance solubility in solvents and resins.

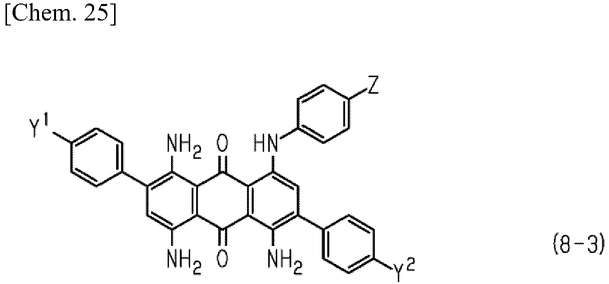

In Formula (8-3), Y¹, Y² and Z are defined as in Formula (8-1). In the anthraquinone derivative expressed by Formula (8-3), substituted or unsubstituted phenyl groups are directly bonded at the β-positions. Such a structure can further enhance light resistance. Specifically, it is considered that if phenyl groups are directly bonded at the β-positions, the molecular structures are less likely to rotate at the bonding sites of the β-positions and that it may be difficult for the anthraquinone skeleton to achieve a stable structure after photoreduction, compared to the case where ether bonds or secondary amines are present at the β-positions, and therefore the progress of photoreduction reaction may be suppressed.

The anthraquinone derivative of the present embodiment can be produced, for example, by introducing substituents to the anthraquinone skeleton using 1,5-diaminoanthraquinone as a starting material. Substituents can be introduced using any known method. For example, desired substituents can be introduced via nitration at the α-positions or bromination at the β-positions.

The compound which is the anthraquinone derivative of the eighth embodiment expressed by Formula (8-1) above may be the anthraquinone derivatives of the first to seventh embodiments.

### (Ninth Embodiment)

The anthraquinone derivative of a ninth embodiment firstly aims to improve light resistance, similarly to the first embodiment.

Among cyanide pigments, compounds with a maximum absorption wavelength close to 700 nm will have a bluish-green color due to increase of green components. In contrast, cyanide pigments, which are used as pigments, corresponding to the three primary colors, are desired to have a strong blue hue. The anthraquinone derivative of the ninth embodiment secondly aims to improve blue hue.

The anthraquinone derivative according to the ninth embodiment is a compound expressed by the following Formula (9-1).

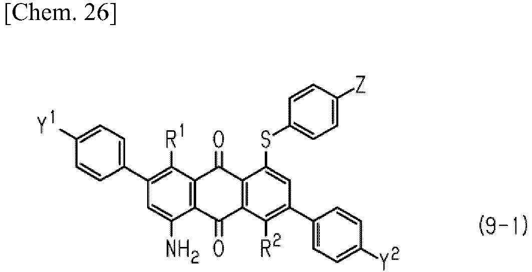

In Formula (9-1), R¹ and R² each independently represent an amino group or hydroxyl group. Y¹, Y² and Z each independently represent a hydrogen atom, alkyl group having 1 to 10 carbon atoms, alkoxy group having 1 to 10 carbon atoms, cyano group, nitro group, halogen atom, halogenated alkyl group having 1 to 10 carbon atoms, amino group, alkylamino group, piperidyl group, aryl group, or cyclohexyl group, and the aryl group and the cyclohexyl group may have substituents. These substituents are preferably alkyl groups having 1 to 10 carbon atoms or cyclic hydrocarbon groups. The cyclic hydrocarbon groups are preferably cyclohexyl groups or phenyl groups.

The halogen atom and the halogen atom contained in the halogenated alkyl group are preferably F, Cl or Br. The alkylamino group may have one or two alkyl groups. The alkyl group of the alkylamino group preferably has 1 to 10 carbon atoms.

The effects of the anthraquinone derivative according to the present embodiment will be described. It is considered that photodegradation in an anthraquinone derivative is induced in part by photoreduction occurring at the anthraquinone skeleton, using resins and other materials surrounding the anthraquinone derivative as hydrogen sources. Conventional anthraquinone derivatives are known to have a structure in which functional groups such as phenyl groups are bonded at the β-positions via ether bonds. In contrast, in the anthraquinone derivative of the present embodiment, substituted or unsubstituted phenyl groups are directly bonded to the carbon atoms of the anthraquinone skeleton at the β-positions. Such a structure can suppress the progress of photoreduction reaction, and therefore high light resistance can be achieved.

Specifically, it is considered that if phenyl groups are directly bonded at the β-positions, the molecular structures may be less likely to rotate at the bonding sites of the β-positions and that it may become difficult for the anthraquinone skeleton to achieve a stable structure after photoreduction, compared to the case where ether bonds are present at the β-positions, and therefore the progress of photoreduction reaction may be suppressed.

The type of the substituents in the anthraquinone skeleton affects the absorption wavelength of the anthraquinone derivative. Regarding substituents at the α-positions, highly electron-donating substituents such as an amino group, hydroxyl group, and anilino group affect such that the maximum absorption wavelength shifts toward longer wavelengths. Structures in which substituted or unsubstituted phenyl groups are directly bonded at the β-positions also have an effect of allowing the maximum absorption wavelength to shift toward longer wavelengths.

It is known that the anthraquinone derivatives as conventional cyanide pigments have a structure in which one of the substituents at the α-positions is an anilino group, and other three are amino groups or hydroxyl groups. In such compounds, if a structure in which phenyl groups are directly bonded at the β-positions is adopted to improve light resistance, the maximum absorption wavelength may shift toward even longer wavelengths, unavoidably increasing green components in the color exhibited by the compounds.

In this regard, the anthraquinone derivative of the present embodiment has a substituted or unsubstituted phenylthio group at an α-position. Since phenylthio groups have lower electron-donating properties than amino groups, hydroxyl groups, or anilino groups, introduction of a phenylthio group to an α-position can suppress the maximum absorption wavelength from shifting toward longer wavelengths, compared to the conventional structures, even when phenyl groups are directly bonded at the β-positions.

In this way, the anthraquinone derivative of the present embodiment has both a structure having an amino group or hydroxyl group at an α-position and phenyl groups directly bonded at the β-positions, which contributes significantly to the shifting of the maximum absorption wavelength toward longer wavelengths, and a structure having a phenylthio group at an α-position, which contributes less to the shifting of the maximum absorption wavelength toward longer wavelengths. Therefore, while achieving a maximum absorption wavelength of 580 nm or greater that can be used as a cyanide pigment, excessive increase of the maximum absorption wavelength can be suppressed. Specifically, the maximum absorption wavelength can be in the range of 580 nm or greater and 670 nm or less, thereby producing an excellent blue hue. Also, while achieving a structure in which phenyl groups are directly bonded at the β-positions, the anthraquinone derivative of the present embodiment can produce an excellent blue hue, thereby achieving both of good light resistance and good blue hue.

In the anthraquinone derivative of the present embodiment, R¹ and R² are preferably hydroxyl groups. Such a compound is expressed by the following Formula (9-2).

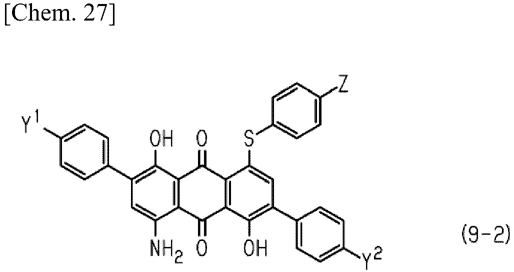

In Formula (9-2), Y¹, Y² and Z are defined as in Formula (9-1). As long as anthraquinone derivatives have a structure expressed by Formula (9-2), they are easy to synthesize. While the presence of a hydroxyl group at an α-position tends to cause the photoreduction mentioned above, the anthraquinone derivative of the present embodiment can suppress the photoreduction reaction as a result of the structure in which phenyl groups are directly bonded at the β-positions as described above. Therefore, the benefits of this structure can be enjoyed, while eliminating the drawbacks of the structure having a hydroxyl group at an α-position.

The anthraquinone derivative of the present embodiment can be produced, for example, by introducing substituents to the anthraquinone skeleton using 1,5-dihydroxy-4,8-dinitroanthraquinone or 1,5-diamino-4,8-dinitroanthraquinone as a starting material. Substituents can be introduced using any known method. For example, desired substituents can be introduced using reduction or substituent conversion of the nitro group, or bromination at the β-positions and conversion of the bromo groups.

The compound which is the anthraquinone derivative of the ninth embodiment expressed by Formula (9-1) above may be any of the anthraquinone derivatives of the fourth to seventh embodiments.

### [Examples]

The anthraquinone derivatives described above will be described using specific examples. Parts by weight of each material listed below indicates a weight ratio relative to the materials mixed together.

### [First Example: Test examples corresponding to first embodiment]

### (Test Example 1-1 to 1-13)

### <Synthesis of Precursor A1>

1,5-dihydroxy-4,8-dinitroanthraquinone (5.0 parts by weight) and N,N-dimethylformamide (100 parts by weight) were placed in a two-necked round-bottom flask. To these materials, N-bromosuccinimide (5.9 parts by weight) was added at room temperature, followed by stirring at room temperature for 1 hour. After confirming completion of reaction by thin-layer chromatography, the reaction solution was added to a sufficient amount of methanol, followed by stirring for 15 minutes. Subsequently, the precipitated solid was recovered by suction filtration, and the recovered material was vacuum-dried overnight at 60°C to obtain Precursor A1 as a yellow solid. Precursor A1 is a compound expressed by the following Formula (1-a).

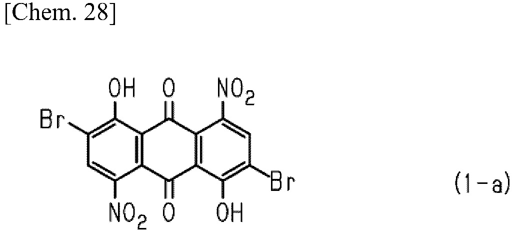

### <Synthesis of Precursor A2>

Precursor A1 (1.0 part by weight) and N-methyl-2-pyrrolidone (20 parts by weight) were placed in a two-necked round-bottom flask. To these materials, 4-heptylaniline (0.8 parts by weight) was added, followed by heating and stirring in a 180°C oil bath. After confirming completion of reaction by thin-layer chromatography, the reaction solution was left to cool to room temperature, then ethyl acetate (30 parts by weight) and water (10 parts by weight) were added to the solution, followed by vigorous stirring at room temperature. Insoluble matter generated during this process was removed by Celite filtration. After separating the two phases, the organic phase was sequentially washed with distilled water, 5% hydrochloric acid solution, and saturated saline solution, and then dried with addition of anhydrous magnesium sulfate. Then, after filtering out the desiccant, the filtrate was concentrated under reduced pressure. The resultant residue was purified using silica gel column chromatography (eluent: hexane/dichloromethane = 2/1 to 2/3), and the resultant solid was vacuum-dried to obtain Precursor A2 as a deep blue solid. Precursor A2 is a compound expressed by the following Formula (1-b).

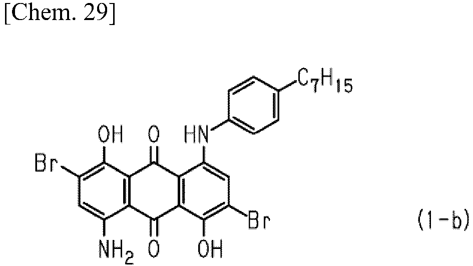

### <Synthesis of Precursor A3>

Precursor A3 was obtained by performing synthesis as in the synthesis process of Precursor A2 described above, except that 4-heptylaniline was replaced with 4-heptyloxyaniline. Precursor A3 is a compound expressed by the following Formula (1-c).

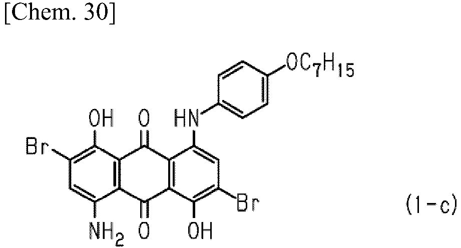

### <Pigment synthesis of Test Example 1-1>

In a round-bottom flask fitted with a Dimroth condenser and purged with nitrogen, toluene (5 parts by weight), water (2.5 parts by weight), Precursor A2 (0.30 parts by weight, 1 equivalent), 4-dimethylaminophenylboronic acid (0.28 parts by weight, 2.4 equivalents), potassium carbonate (0.21 parts by weight) as a base, and tetrakistriphenylphosphine palladium (0.06 parts by weight) as a catalyst were placed, and these materials were heated and stirred at 80°C for 2 hours. After confirming completion of reaction using thin-layer chromatography, the reaction solution was returned to room temperature, to which distilled water was added to perform extraction with ethyl acetate. To the organic phase obtained, sodium sulfate was added, followed by drying, which was further followed by removing the solvent under reduced pressure using an evaporator. The obtained residue was purified using column chromatography (eluent: hexane/ethyl acetate = 10/1 to 5/1) to obtain an anthraquinone derivative of Test Example 1-1. The anthraquinone derivative of Test Example 1-1 is a compound expressed by the following Formula (P1-1).

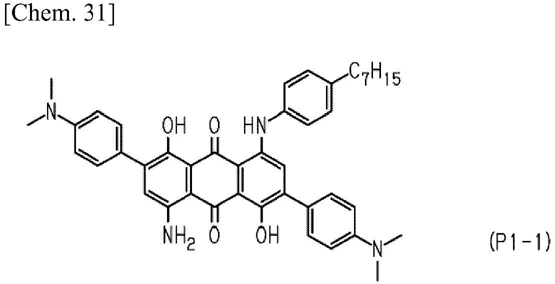

### <Pigment synthesis of Test Example 1-2>

An anthraquinone derivative of Test Example 1-2 was obtained by performing synthesis as in the pigment synthesis process of Test Example 1-1 described above, except that 4-dimethylaminophenylboronic acid was replaced with 4-dibutylaminophenylboronic acid. The anthraquinone derivative of Test Example 1-2 is a compound expressed by the following Formula (P1-2).

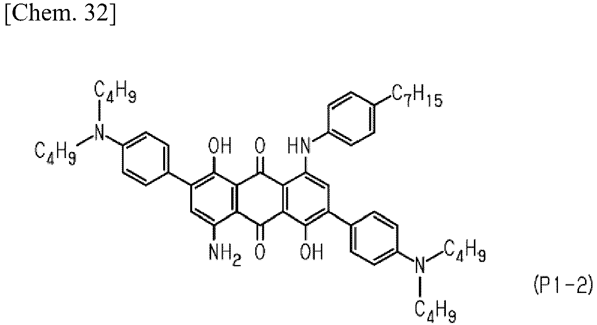

### <Pigment synthesis of Test Example 1-3>

An anthraquinone derivative of Test Example 1-3 was obtained by performing synthesis as in the pigment synthesis process of Test Example 1-1 described above, except that 4-dimethylaminophenylboronic acid was replaced with 4-monobutylaminophenylboronic acid. The anthraquinone derivative of Test Example 1-3 is a compound expressed by the following Formula (P1-3).

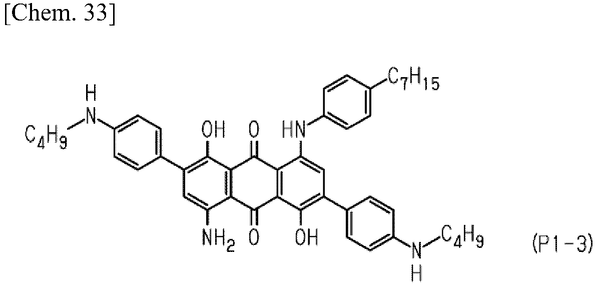

### <Pigment synthesis of Test Example 1-4>

An anthraquinone derivative of Test Example 1-4 was obtained by performing synthesis as in the pigment synthesis process of Test Example 1-1 described above, except that Precursor A2 was replaced with Precursor A3 and that 4-dimethylaminophenylboronic acid was replaced with 4-monobutylaminophenylboronic acid. The anthraquinone derivative of Test Example 1-4 is a compound expressed by the following Formula (P1-4).

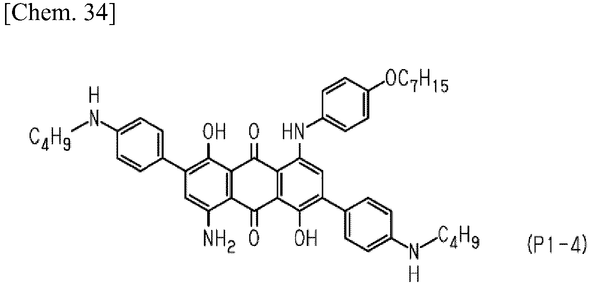

### <Pigment synthesis of Test Example 1-5>

An anthraquinone derivative of Test Example 1-5 was obtained by performing synthesis as in the pigment synthesis process of Test Example 1-1 described above, except that 4-dimethylaminophenylboronic acid was replaced with (4-piperidine-1-yl)phenylboronic acid. The anthraquinone derivative of Test Example 1-5 is a compound expressed by the following Formula (P1-5).

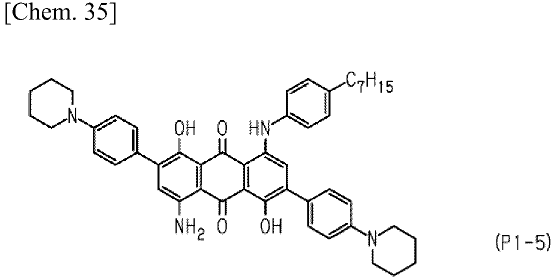

### <Pigment synthesis of Test Example 1-6>

An anthraquinone derivative of Test Example 1-6 was obtained by performing synthesis as in the pigment synthesis process of Test Example 1-1 described above, except that 4-dimethylaminophenylboronic acid was replaced with 4-aminophenylboronic acid. The anthraquinone derivative of Test Example 1-6 is a compound expressed by the following Formula (P1-6).

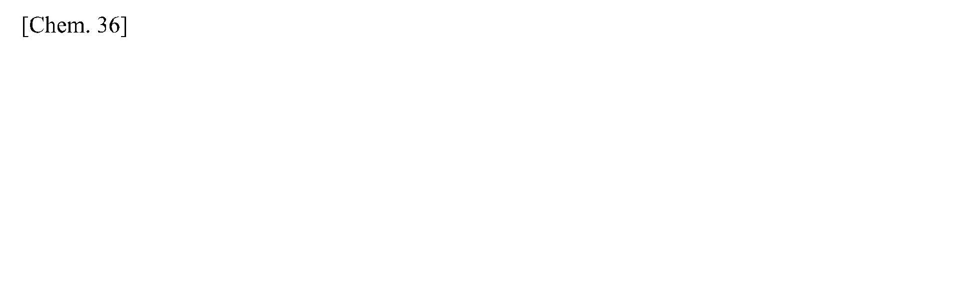

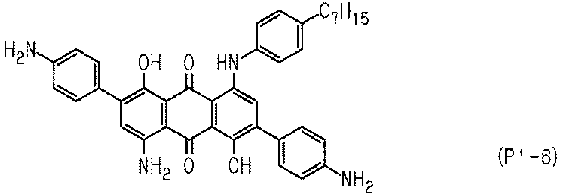

### <Pigment synthesis of Test Example 1-7>

An anthraquinone derivative of Test Example 1-7 was obtained by performing synthesis as in the pigment synthesis process of Test Example 1-1 described above, except that 4-dimethylaminophenylboronic acid was replaced with 4-acetamidephenylboronic acid. The anthraquinone derivative of Test Example 1-7 is a compound expressed by the following Formula (P1-7).

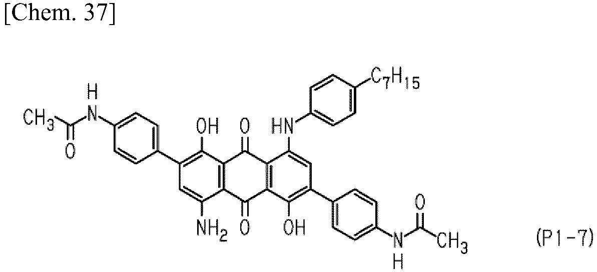

### <Pigment synthesis of Test Example 1-8>

An anthraquinone derivative of Test Example 1-8 was obtained by performing synthesis as in the pigment synthesis process of Test Example 1-1 described above, except that 4-dimethylaminophenylboronic acid was replaced with 4-hydroxyphenylboronic acid. The anthraquinone derivative of Test Example 1-8 is a compound expressed by the following Formula (P1-8).

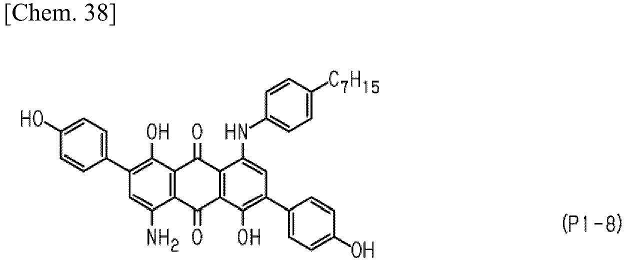

### <Pigment synthesis of Test Example 1-9>

In the pigment synthesis process of Test Example 1-1 described above, Precursor A2 was replaced with Precursor A3, and 4-dimethylaminophenylboronic acid was replaced with 1.2 equivalents of 4-monobutylaminophenylboronic acid, and the reaction solution was stirred at 80°C for 1 hour, to which 1.2 equivalents of 4-heptylphenylboronic acid were further added, followed by heating and stirring at 80°C for at least 2 hours. Subsequently, an anthraquinone derivative of Test Example 1-9 was obtained by performing extraction and purification as in the pigment synthesis process of Test Example 1-1 described above. The anthraquinone derivative of Test Example 1-9 is a compound expressed by the following Formula (P1-9).

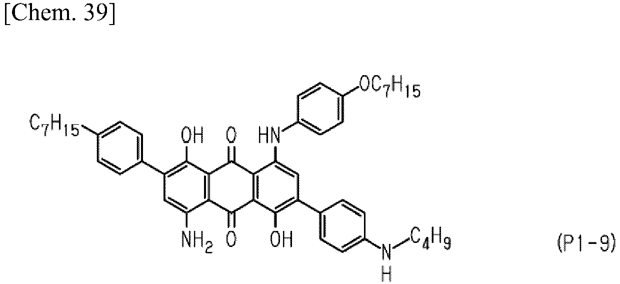

### <Pigment synthesis of Test Example 1-10>

An anthraquinone derivative of Test Example 1-10 was obtained by performing synthesis as in the pigment synthesis process of Test Example 1-1 described above, except that 4-dimethylaminophenylboronic acid was replaced with 4-heptyloxyphenylboronic acid. The anthraquinone derivative of Test Example 1-10 is a compound expressed by the following Formula (P1-10).

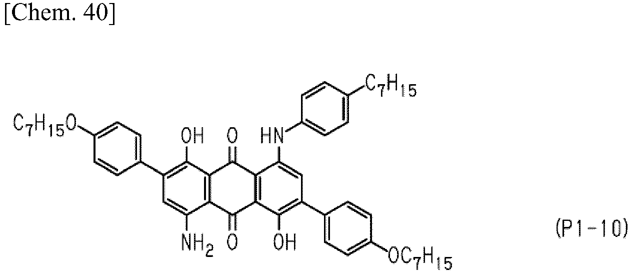

### <Pigment synthesis of Test Example 1-11>

An anthraquinone derivative of Test Example 1-11 was obtained by performing synthesis as in the pigment synthesis process of Test Example 1-1 described above, except that Precursor A2 was replaced with Precursor A3 and that 4-dimethylaminophenylboronic acid was replaced with 4-heptyloxyphenylboronic acid. The anthraquinone derivative of Test Example 1-11 is a compound expressed by the following Formula (P1-11).

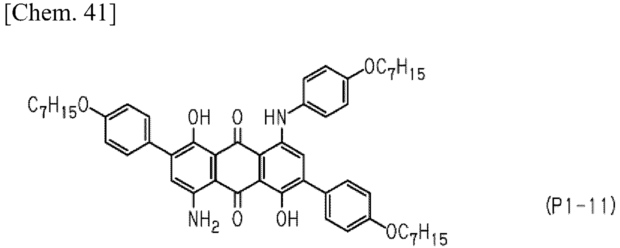

### <Pigment synthesis of Test Example 1-12>

4-heptyloxyphenol (8.65 parts by weight) and N-methyl-2-pyrrolidone (100 parts by weight) were placed in a two-necked round-bottom flask. To these materials, sodium hydride (55%, 1.7 parts by weight) was added gently in five separate doses, followed by stirring in a 60°C oil bath for 3 hours. To this solution, Precursor A3 (5.00 parts by weight) was added, followed by heating and stirring in a 130°C oil bath for 24 hours. After confirming completion of reaction using thin-layer chromatography, the reaction solution was returned to room temperature, and ethyl acetate and distilled water were added to the solution, followed by vigorous stirring at room temperature. After separating the two phases, the organic phase was sequentially washed with distilled water and saturated saline solution, and then dried with addition of anhydrous magnesium sulfate. Then, after filtering out the desiccant, the filtrate was concentrated under reduced pressure. The resultant residue was purified using silica gel column chromatography (eluent: hexane/dichloromethane = 1/1 to 1/2), and the resultant solid was washed with ethanol and then vacuum-dried at 60°C to obtain an anthraquinone derivative of Test Example 1-12. The anthraquinone derivative of Test Example 1-12 is a compound expressed by the following Formula (P1-12).

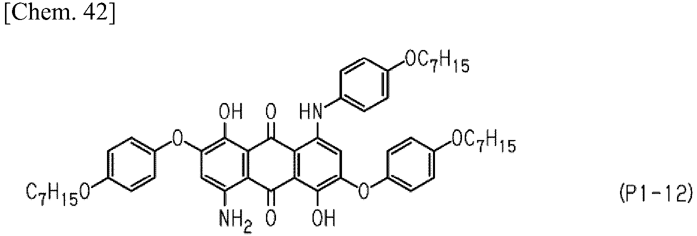

### <Pigment synthesis of Test Example 1-13>

An anthraquinone derivative of Test Example 1-13 was obtained by performing synthesis as in the pigment synthesis process of Test Example 1-12 described above, except that 4-heptyloxyphenol (8.65 parts by weight) was replaced with 4-butylaminophenol (6.86 parts by weight) and that Precursor A3 was replaced with Precursor A2. The anthraquinone derivative of Test Example 1-13 is a compound expressed by the following Formula (P1-13).

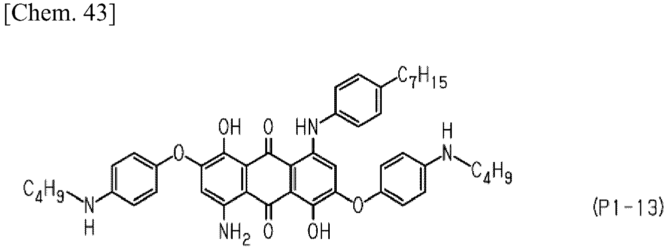

### (Test Examples 1-14 and 1-15)

### <Synthesis of Precursor B1>

1,5-diaminoanthraquinone (5.0 parts by weight), N,N-dimethylformamide (397 parts by weight), and pyridine (10.3 parts by weight) were placed in a two-necked round-bottom flask and purged with nitrogen. This solution was thoroughly cooled in an ice bath, to which acetyl chloride (19.8 parts by weight) was added dropwise over 45 minutes while being cooled with ice. The solution was then stirred for 10 minutes while being cooled with ice, and then stirred at room temperature for 24 hours. The precipitate was then recovered by suction filtration, and the recovered material was washed with diethyl ether and dried to obtain a brown crude product. Subsequently, the crude product (5.5 parts by weight) and ethyl acetate (54 parts by weight) were placed in a round-bottom flask, and heated to reflux temperature, followed by suspension washing for 30 minutes. Subsequently, the recovered material obtained by suction filtration was washed with diethyl ether and then dried at 60°C under reduced pressure to obtain Precursor B1 as a brown powder. Precursor B1 is a compound expressed by the following Formula (1-d).

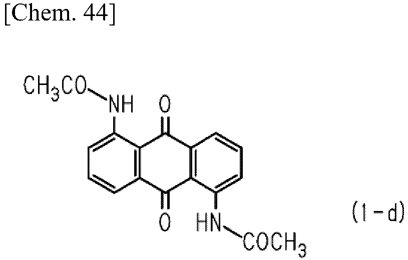

### <Synthesis of Precursor B2>

Concentrated sulfuric acid (48 parts by weight) was placed in a four-necked flask equipped with a thermometer and a mechanical stirrer, to which potassium nitrate (6.0 parts by weight) was added and dissolved completely, followed by cooling until the internal temperature of the four-necked flask reached 0°C. To this solution, Precursor B1 (3.0 parts by weight) was added in six separate doses, 0.5 parts by weight each, ensuring that the internal temperature of the four-necked flask did not exceed 5°C. After a while following addition of Precursor B1, a light brown precipitate formed. Subsequently, after stirring the mixture for 6 hours in a state in which the internal temperature of the four-necked flask was 0°C to 5°C, disappearance of the substrate was confirmed as a result of ¹H-NMR measurement (solvent: DMSO-d6), and therefore the stirring was stopped. This solution was added little by little to pure water cooled to 0°C to 5°C, the precipitate was recovered by suction filtration, and the recovered material was dried at 60°C under reduced pressure to obtain a crude product. The crude product was dissolved in nitrobenzene and stirred at 180°C for 30 minutes. After leaving the solution to cool naturally to room temperature, the precipitate recovered by filtration was dried at 60°C under reduced pressure to obtain Precursor B2 as a dull yellow powder. Precursor B2 is a compound expressed by the following Formula (1-e).

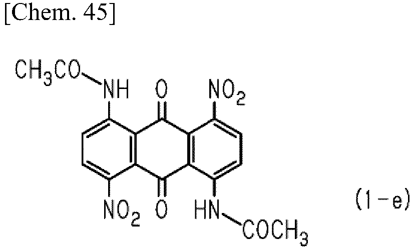

### <Synthesis of Precursor B3>

Pure water (5.5 parts by weight) was placed in a two-necked flask, and then concentrated sulfuric acid (40 parts by weight) was slowly added while being cooled with ice. To this solution, Precursor B2 (2.2 parts by weight) was added while being cooled with ice. This solution was stirred at 95°C for 2 hours, and then added to pure water (200 parts by weight) while being cooled with ice. Subsequently, the precipitate was recovered by suction filtration, and the recovered material was dried at 60°C under reduced pressure to obtain Precursor B3 as a red powder. Precursor B3 is a compound expressed by the following Formula (1-f).

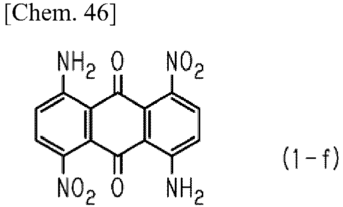

### <Synthesis of Precursor B4>

Precursor B3 (1.6 parts by weight) and N-bromosuccinimide (1.9 parts by weight) were placed in a two-necked flask and purged with nitrogen. To these materials, nitrogen-bubbled N,N-dimethylformamide (30 parts by weight) was added, followed by stirring at room temperature for 20 hours. This reaction solution was poured into methanol (200 parts by weight), followed by stirring at room temperature for 15 minutes. Subsequently, the precipitate was recovered by suction filtration, and the recovered material was dried at 60°C under reduced pressure to obtain Precursor B4 as a reddish-brown powder. Precursor B4 is a compound expressed by the following Formula (1-g).

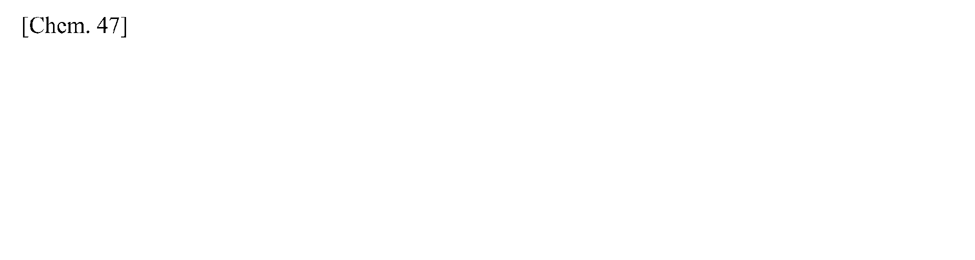

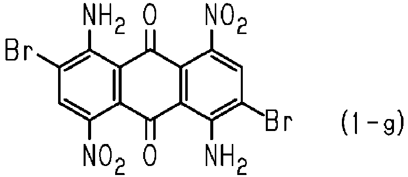

### <Synthesis of Precursor B5>

A round-bottom flask fitted with a Dimroth condenser was purged with nitrogen. In this flask, toluene (5 parts by weight), water (2.5 parts by weight), Precursor B4 (0.30 parts by weight), (4-piperidine-1-yl)phenylboronic acid (0.28 parts by weight), potassium carbonate (0.21 parts by weight), and tetrakistriphenylphosphine palladium (0.06 parts by weight) were placed, and heated and stirred at 80°C for at least 2 hours. After confirming completion of reaction using thin-layer chromatography, the reaction solution was returned to room temperature, to which distilled water was added to perform extraction with ethyl acetate. To the organic phase obtained, sodium sulfate was added, followed by drying, which was further followed by removing the solvent under reduced pressure using an evaporator. The obtained residue was purified using column chromatography (eluent: hexane/ethyl acetate = 10/1 to 5/1) to obtain Precursor B5. Precursor B5 is a compound expressed by the following Formula (1-h).

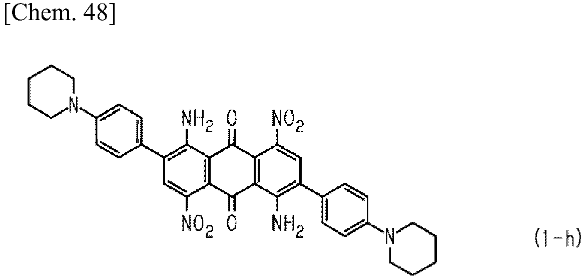

### <Synthesis of Precursor B6>

Precursor B5 (0.20 parts by weight) was placed in a two-necked flask and purged with nitrogen. To this material, nitrobenzene (4.8 parts by weight) and 4-heptylaniline (0.52 parts by weight) were added, followed by stirring at 200°C for 12 hours. The reaction solution was dried at 75°C under reduced pressure to distill away the nitrobenzene and obtain the residue. Methanol was poured into this residue, and the precipitated powder was recovered by suction filtration to obtain Precursor B6. Precursor B6 is a compound expressed by the following Formula (1-i).

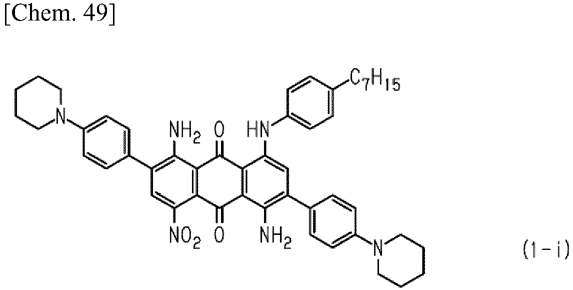

### <Synthesis of Precursor B7>

Precursor B7 was obtained by performing synthesis as in the synthesis process of Precursor B5 described above, except that (4-piperidine-1-yl)phenylboronic acid was replaced with 4-heptyloxyphenylboronic acid. Precursor B7 is a compound expressed by the following Formula (1-j).

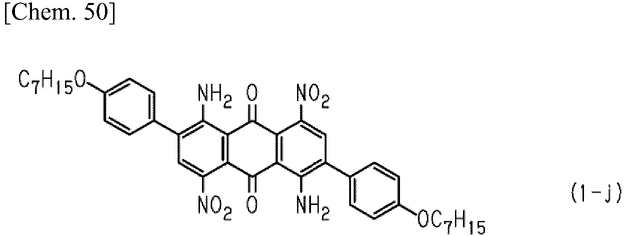

### <Synthesis of Precursor B8>

Precursor B8 was obtained by performing synthesis as in the synthesis process of Precursor B6 described above, except that Precursor B5 was replaced with Precursor B7. Precursor B8 is a compound expressed by the following Formula (1-k).

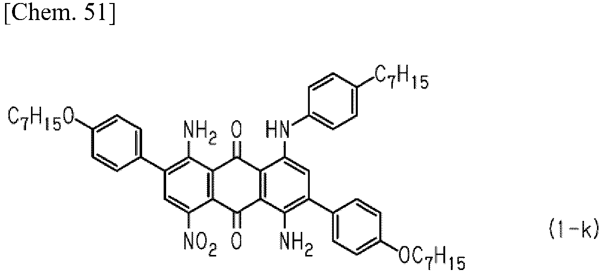

### <Pigment synthesis of Test Example 1-14>

Precursor B6 (0.20 parts by weight) was placed in a two-necked flask and purged with nitrogen. To this material, 2-propanol (3.1 parts by weight) was added, followed by heating to 80°C, to which sodium borohydride (0.22 parts by weight) was further added, and the mixture was stirred for 27 hours. This reaction solution was poured into cold water, and the precipitated powder was recovered by suction filtration and then purified using column chromatography to obtain an anthraquinone derivative of Test Example 1-14. The anthraquinone derivative of Test Example 1-14 is a compound expressed by the following Formula (P1-14).

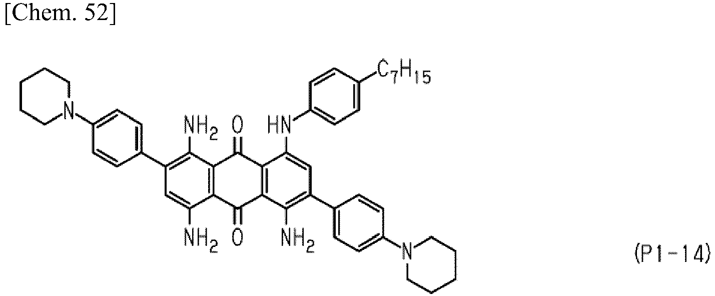

### <Pigment synthesis of Test Example 1-15>

An anthraquinone derivative of Test Example 1-15 was obtained by performing synthesis as in the pigment synthesis process of Test Example 1-14 described above, except that Precursor B6 was replaced with Precursor B8. The anthraquinone derivative of Test Example 1-15 is a compound expressed by the following Formula (P1-15).

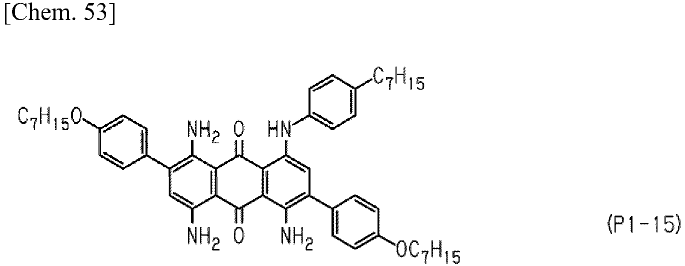

### (Evaluation Method)

### <Preparation of evaluation test specimens>

The following materials were mixed with the anthraquinone derivative of each test example to prepare a pigment-containing composition.

| | |
|---|---|
| Mixture of pentaerythritol tetraacrylate and isoboronyl acrylate (70 mass% of pentaerythritol tetraacrylate and 30 mass% of isoboronyl acrylate): | 45 parts by weight |
| Photopolymerization initiator (Omnirad TPO manufactured by IGM Resins B.V.) | 4.5 parts by weight |
| Anthraquinone derivative: | 1 part by weight |
| Methyl ethyl ketone | 50 parts by weight |

The pigment-containing composition was applied to a polyethylene terephthalate film as a transparent substrate having a thickness of 60 µm, and the coating was dried in an 80°C oven for 60 seconds. After that, UV light was applied to the coating at a dose of 150 mJ/cm² using a UV irradiation device (light source H-Bulb, manufactured by Fusion UV Systems Japan K.K.) to cure the coating. Test specimens for evaluation were prepared by adjusting the thickness so that the cured thickness was 8.0 µm.

### <Evaluation of absorption wavelength and coloring power>

For the evaluation test specimen of each test example, a UV-visible absorption spectrum was measured using an automated spectrophotometer (U-4100 manufactured by Hitachi, Ltd.), and a maximum absorption wavelength λmax and an absorbance at this wavelength were obtained.

When evaluating the coloring power, Test Example 1-10 corresponding to the conventional anthraquinone derivative was used as a reference. Specifically, the test specimens having an absorbance greater than that of Test Example 1-10 were evaluated to be Good and those having an absorbance lower than or equal to that of Test Example 1-10 were evaluated to be Poor.

### <Evaluation of light resistance>

The evaluation test specimen of each test example was subjected to light resistance testing using a xenon weather meter (X75 manufactured by Suga Test Instruments Co., Ltd.). In the light resistance testing, a UV-cut adhesive film absorbing light of 395 nm or less was attached to the evaluation test specimen, and the front surface of the test specimen was left to stand under conditions of 60 W/cm² (300 nm to 400 nm) xenon lamp illuminance, 45°C temperature, and 50%RH humidity for 120 hours.

Absorbance was measured for each evaluation test specimen before and after the light resistance testing using an automated spectrophotometer (U-4100 manufactured by Hitachi, Ltd.) to obtain the absorbance at the wavelength showing the maximum absorbance in the visible light range. Then, an absorbance change ratio ΔAbs before and after the light resistance testing was calculated. Specifically, an equation ΔAbs(%)={(Abs1-Abs2)/Abs1}×100 can be established, where Abs1 represents an absorbance measured before the light resistance testing, and Abs2 represents an absorbance measured after the light resistance testing.

When evaluating light resistance, test specimens having ΔAbs of 2% or less were evaluated to be Excellent, those having ΔAbs of higher than 2% and 10% or less were evaluated to be Good, and those having ΔAbs of higher than 10% were evaluated to be Poor.

### <Measurement of dichroic ratio>

For each test example, a liquid crystal composition was prepared by mixing the anthraquinone derivative with a cyanocyclohexylbenzene-based liquid crystal (ZLI-1840 manufactured by Merck) at a ratio of 1.0 mass%. Next, the liquid crystal composition was filled in a cell to prepare a host-guest liquid crystal element. The cell had a structure in which two glass plates with transparent electrodes, which had been subjected to homogeneous alignment treatment, were placed facing each other so that the alignment treatment surfaces were antiparallel. The alignment treatment was performed by applying a polyimide resin to the transparent electrodes, curing the resin, and then performing rubbing. The cell had a thickness of 10 µm.

The liquid crystal element was placed in the optical path of a spectrophotometer to measure an absorbance A *∥* when linearly polarized light parallel to the rubbing direction was applied to the liquid crystal element and an absorbance A⊥ when linearly polarized light perpendicular to the rubbing direction was applied to the liquid crystal element. Then, as a dichroic ratio, the ratio of the absorbance A*∥*to the absorbance A⊥ (A*∥*/A⊥) was calculated.

### (Evaluations)

Table 1 shows the structure, maximum absorption wavelength λmax, absorbance at the maximum absorption wavelength λmax, evaluation of coloring power, absorbance change ratio ΔAbs, evaluation of light resistance, and dichroic ratio of each anthraquinone derivative, for Test Examples 1-1 to 1-15. The structure of the anthraquinone derivative of each test example is expressed by the following Formula (I), and R, A, Y¹, Y² and Z of Table 1 correspond to R, A, Y¹, Y² and Z of Formula (I).

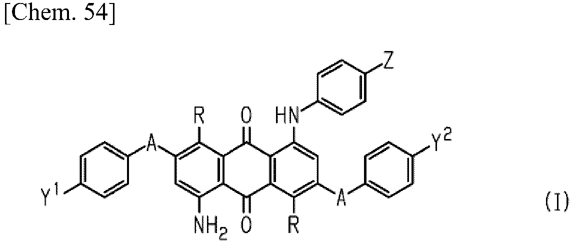

**[Table 1]**

| Test Ex. | Structure | | | | | λmax (nm) | Coloring power | | Light resistance | | Dichroic ratio |
|---|---|---|---|---|---|---|---|---|---|---|---|
| | R | A | Y¹ | Y² | Z | | Absorbance | Evaluation | ΔAbs (%) | Evaluation | |
| 1-1 | OH | Direct bond | N(CH₃)₂ | N(CH₃)₂ | C₇H₁₅ | 694 | 0.69 | Good | 2 | Excellent | 13.5 |
| 1-2 | OH | Direct bond | N(C₄H₉)₂ | N(C₄H₉)₂ | C₇H₁₅ | 701 | 0.68 | Good | 3 | Good | 10.2 |
| 1-3 | OH | Direct bond | NHC₄H₉ | NHC₄H₉ | C₇H₁₅ | 691 | 0.72 | Good | 1 | Excellent | 13.4 |
| 1-4 | OH | Direct bond | NHC₄H₉ | NHC₄H₉ | OC₇H₁₅ | 688 | 0.71 | Good | 1 | Excellent | 12.2 |
| 1-5 | OH | Direct bond | Piperidyl group | Piperidyl group | C₇H₁₅ | 692 | 0.65 | Good | 7 | Good | 12.1 |
| 1-6 | OH | Direct bond | NH₂ | NH₂ | C₇H₁₅ | 680 | 0.64 | Good | 3 | Good | 11.3 |
| 1-7 | OH | Direct bond | Acetamide group | Acetamide group | C₇H₁₅ | 683 | 0.55 | Good | 4 | Good | 10.5 |
| 1-8 | OH | Direct bond | OH | OH | C₇H₁₅ | 679 | 0.57 | Good | 4 | Good | 10.7 |
| 1-9 | OH | Direct bond | C₇H₁₅ | NHC₄H₉ | OC₇H₁₅ | 687 | 0.57 | Good | 1 | Excellent | 12.5 |
| 1-10 | OH | Direct bond | OC₇H₁₅ | OC₇H₁₅ | C₇H₁₅ | 681 | 0.52 | Poor | <1 | Excellent | 11.4 |
| 1-11 | OH | Direct bond | OC₇H₁₅ | OC₇H₁₅ | OC₇H₁₅ | 681 | 0.42 | Poor | <1 | Excellent | 12.2 |
| 1-12 | OH | O | OC₇H₁₅ | OC₇H₁₅ | OC₇H₁₅ | 632 | 0.23 | Poor | 43 | Poor | 12.2 |
| 1-13 | OH | O | NHC₄H₉ | NHC₄H₉ | C₇H₁₅ | 608 | 0.35 | Poor | 30 | Poor | 11.1 |
| 1-14 | NH₂ | Direct bond | Piperidyl group | Piperidyl group | C₇H₁₅ | 709 | 0.62 | Good | 2 | Excellent | 9.0 |
| 1-15 | NH₂ | Direct bond | OC₇H₁₅ | OC₇H₁₅ | C₇H₁₅ | 699 | 0.49 | Poor | <1 | Excellent | 8.6 |

As shown in Table 1, the anthraquinone derivatives of the test examples have a maximum absorption wavelength in the wavelength range of 600 nm or greater. Therefore, the anthraquinone derivatives of the test examples exhibit a blue color and can be used as cyanide pigments.

Furthermore, in Test Examples 1-1 to 1-11, 1-14 and 1-15, where substituted phenyl groups are directly bonded at the β-positions, extremely high light resistance is achieved compared to Test Examples 1-12 and 1-13, where ether bonds are present at the β-positions.

In Test Examples 1-1 to 1-9 and 1-14, where the para substituent of a phenyl group directly bonded at at least one β-position is an electron-donating group, high absorbance and good coloring power are achieved compared to Test Examples 1-10, 1-11 and 1-15, where the substituent in question is not an electron-donating group. In Test Examples 1-1 to 1-9 and 1-14, high absorbance is achieved even relative to Test Examples 1-12 and 1-13, where ether bonds are present at the β-positions.

Comparing Test Examples 1-3 with 1-9, a higher absorbance is achieved in the test example, where both of the two phenyl groups at the β-positions have electron-donating groups as substituents, than in the test example where only one of the substituents is an electron-donating group. Referring to Test Examples 1-1 to 1-8, it was confirmed that when the electron-donating group is an alkylamino group, piperidyl group, or amino group, the effect of improving absorbance is higher than when the electron-donating group is an acetamide group or hydroxyl group.

The test examples all achieved a good dichroic ratio, and it was confirmed that a high dichroic ratio of 10.0 or higher can be achieved especially when two hydroxyl groups are present at α-positions.

### [Second Example: Test examples corresponding to second embodiment]

In the Second Example, synthesis was performed using Precursors A1, A2 and A3 similar to those of the First Example.

### (Test Example 2-1)

### <Synthesis of Precursor C1>

Precursor A1 (1.0 part by weight) and p-aminobenzonitrile (2.42 parts by weight) were placed in a two-necked round-bottom flask and purged with nitrogen. To these materials, nitrobenzene (24 parts by weight) was added, followed by heating and stirring in a 190°C oil bath for 5 hours. The reaction solution, after being cooled to room temperature, was dried at 70°C under reduced pressure to remove the solvent. Methanol was poured into the resultant residue, and the precipitated powder was recovered by suction filtration to obtain Precursor C1 as a purple powder. Precursor C1 is a compound expressed by the following Formula (2-a).

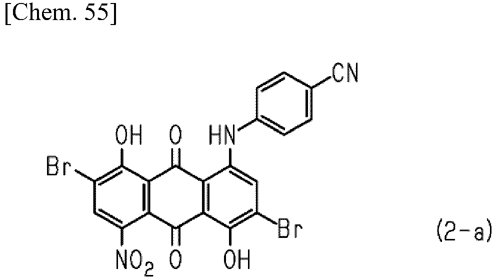

### <Synthesis of Precursor C2>

Precursor C1 (1.0 part by weight), (4-pentyloxy)phenylboronic acid (1.18 parts by weight), cesium carbonate (1.74 parts by weight), [1,1,-bis(diphenylphosphinoferrocene)]dichloropalladium(II) (0.131 parts by weight), and 2-dicyclohexylphosphino-2',6'-dimethoxybiphenyl (0.147 parts by weight) were place in a two-necked round-bottom flask and purged with nitrogen. To these materials, toluene (71 parts by weight), ethanol (7.2 parts by weight), and pure water (4.5 parts by weight) were added, followed by stirring in an 80°C oil bath for 2 hours. The reaction solution, after being cooled to room temperature, was separated with dichloromethane and pure water, and the organic phase was dried with sodium sulfate. Then, after filtering out the sodium sulfate and concentrating the remaining solution, the concentrated solution was purified using silica gel column chromatography (eluent: dichloromethane/hexane = 1/1) to obtain a crude product. The crude product was subjected to column purification to obtain Precursor C2 as a purple powder. The yield of Precursor C2 was 11%. Precursor C2 is a compound expressed by the following Formula (2-b).

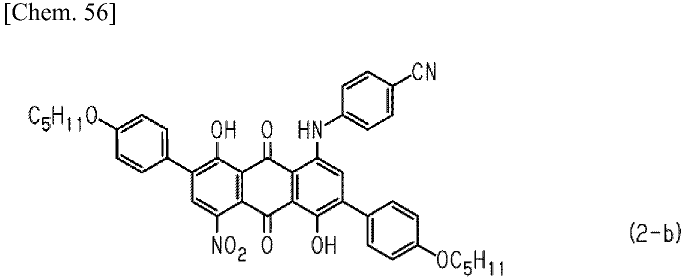

### <Pigment synthesis of Test Example 2-1>

Precursor C2 (1 part by weight), iron powder (0.325 parts by weight), and ammonium chloride (0.311 parts by weight) were placed in a two-necked round-bottom flask and purged with nitrogen. To these materials, toluene (27.9 parts by weight), ethanol (8.38 parts by weight), and pure water (5.31 parts by weight) were added, followed by heating and stirring in a 70°C oil bath for 1 hour. The reaction solution, after being cooled to room temperature, was filtered with Celite to remove insoluble components, then separated with dichloromethane and pure water, followed by washing with pure water and saturated saline, and the organic phase was dried with sodium sulfate. After filtering out the sodium sulfate and concentrating the remaining solution, the concentrated solution was purified using silica gel column chromatography (eluent: dichloromethane/hexane = 2/1 to 1/1) to obtain an anthraquinone derivative of Test Example 2-1 as a purple powder. The anthraquinone derivative of Test Example 2-1 is a compound expressed by the following Formula (P2-1).

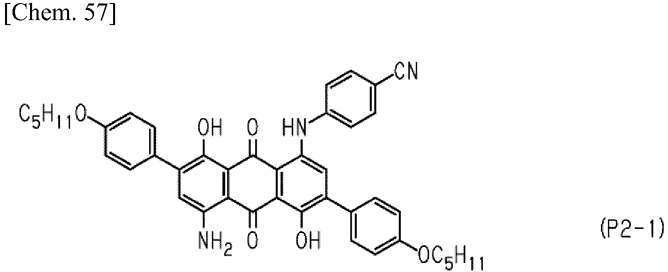

### (Test Example 2-2)

### <Synthesis of Precursor C3>

Precursor C3 was obtained by performing the synthesis as in the synthesis process of Precursor C1 described above, except that p-aminobenzonitrile (2.42 parts by weight) was replaced with p-aminobenzotrifluoride (3.3 parts by weight). Precursor C3 is a compound expressed by the following Formula (2-c).

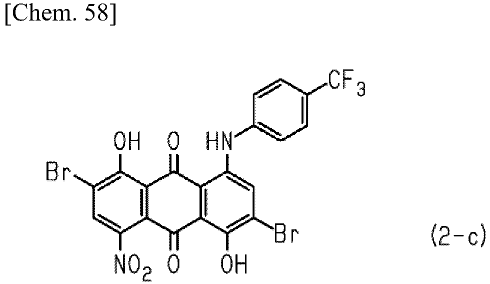

### <Synthesis of Precursor C4>

Precursor C4 was obtained by performing synthesis as in the synthesis process of Precursor C2 described above, except that Precursor C1 was replaced with Precursor C3. Precursor C4 is a compound expressed by the following Formula (2-d).

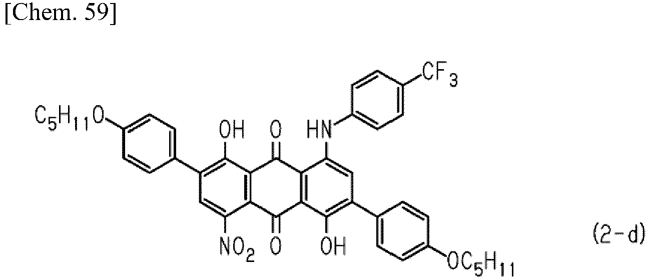

### <Pigment synthesis of Test Example 2-2>

An anthraquinone derivative of Test Example 2-2 was obtained by performing synthesis as in the pigment synthesis process of Test Example 2-1 described above, except that Precursor C2 was replaced with Precursor C4. The anthraquinone derivative of Test Example 2-2 is a compound expressed by the following Formula (P2-2).

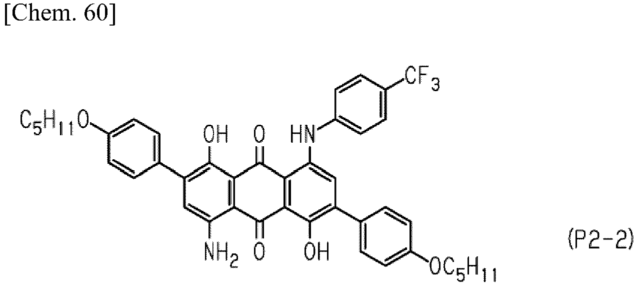

### (Test Example 2-3)

### <Pigment synthesis of Test Example 2-3>

Precursor C5 was obtained by performing synthesis as in the synthesis process of Precursor C2 described above, except that (4-pentyloxy)phenylboronic acid was replaced with 4-(trans-4-pentylcyclohexyl)phenylboronic acid. An anthraquinone derivative of Test Example 2-3 was obtained by performing synthesis as in the pigment synthesis process of Test Example 2-1 described above, except that Precursor C2 was replaced with Precursor C5. The anthraquinone derivative of Test Example 2-3 is a compound expressed by the following Formula (P2-3).

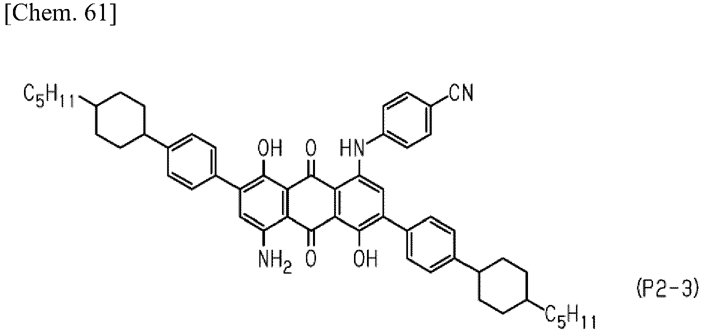

### (Test Example 2-4)

### <Pigment synthesis of Test Example 2-4>

Precursor C6 was obtained by performing synthesis as in the synthesis process of Precursor C2 described above, except that Precursor C1 was replaced with Precursor C3 and that (4-pentyloxy)phenylboronic acid was replaced with 4-(trans-4-pentylcyclohexyl)phenylboronic acid. Then, an anthraquinone derivative of Test Example 2-4 was obtained by performing synthesis as in the pigment synthesis process of Test Example 2-1 described above, except that Precursor C2 was replaced with Precursor C6. The anthraquinone derivative of Test Example 2-4 is a compound expressed by the following Formula (P2-4).

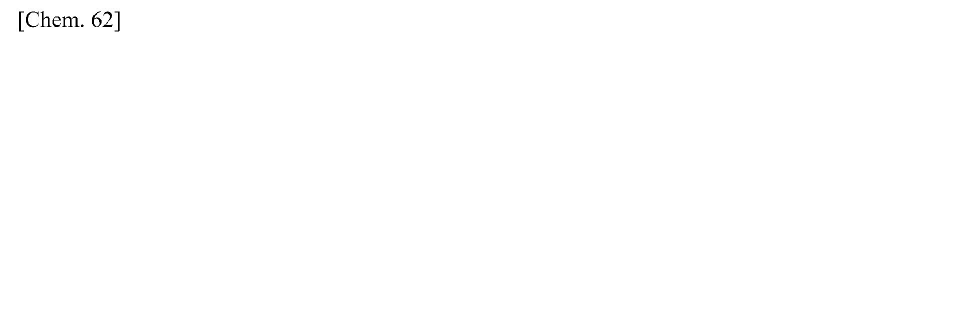

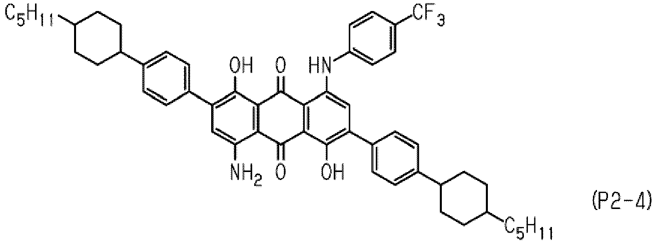

### (Test Example 2-5)

### <Pigment synthesis of Test Example 2-5>

Precursor C7 was obtained by performing synthesis as in the synthesis process of Precursor C2 described above, except that (4-pentyloxy)phenylboronic acid was replaced with 4-(dimethylamino)phenylboronic acid. Then, an anthraquinone derivative of Test Example 2-5 was obtained by performing synthesis as in the pigment synthesis process of Test Example 2-1 described above, except that Precursor C2 was replaced with Precursor C7. The anthraquinone derivative of Test Example 2-5 is a compound expressed by the following Formula (P2-5).

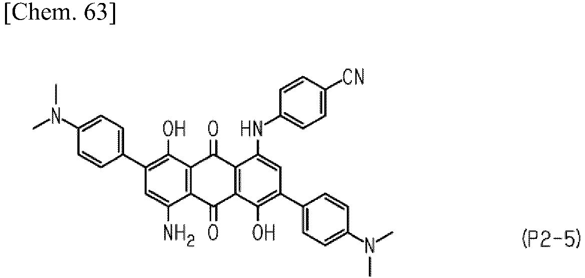

### (Test Example 2-6)

The pigment of Test Example 1-10 of the First Example was used as an anthraquinone derivative of Test Example 2-6. The anthraquinone derivative of Test Example 2-6 is a compound expressed by the following Formula (P2-6).

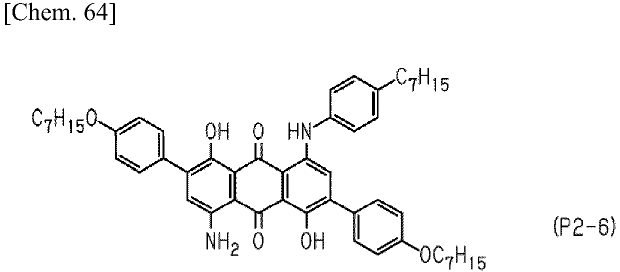

### (Test Example 2-7)

The pigment of Test Example 1-11 of the First Example was used as an anthraquinone derivative of Test Example 2-7. The anthraquinone derivative of Test Example 2-7 is a compound expressed by the following Formula (P2-7).

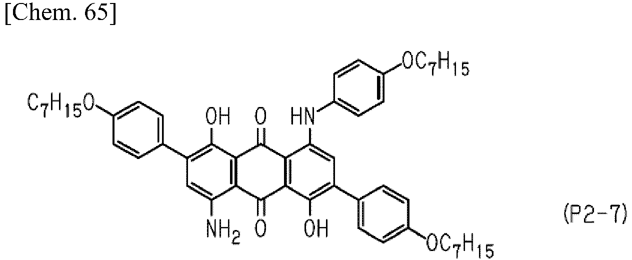

### (Test Example 2-8)

### <Pigment synthesis of Test Example 2-8>

An anthraquinone derivative of Test Example 2-8 was obtained by performing synthesis as in the pigment synthesis process of Test Example 1-1 of the First Example, except that 4-dimethylaminophenylboronic acid was replaced with 4-(trans-4-pentylcyclohexyl)phenylboronic acid. The anthraquinone derivative of Test Example 2-8 is a compound expressed by the following Formula (P2-8).

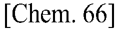

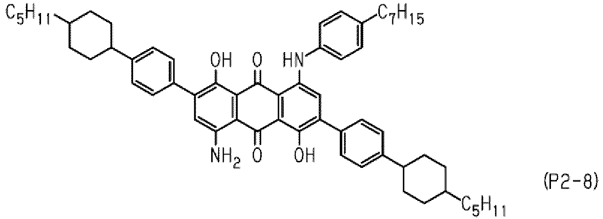

### (Test Example 2-9)

### <Pigment synthesis of Test Example 2-9>

An anthraquinone derivative of Test Example 2-9 was obtained by performing synthesis as in the pigment synthesis process of Test Example 1-1 of the First Example, except that Precursor A2 was replaced with Precursor A3 and that 4-dimethylaminophenylboronic acid was replaced with 4-(trans-4-pentylcyclohexyl)phenylboronic acid. The anthraquinone derivative of Test Example 2-9 is a compound expressed by the following Formula (P2-9).

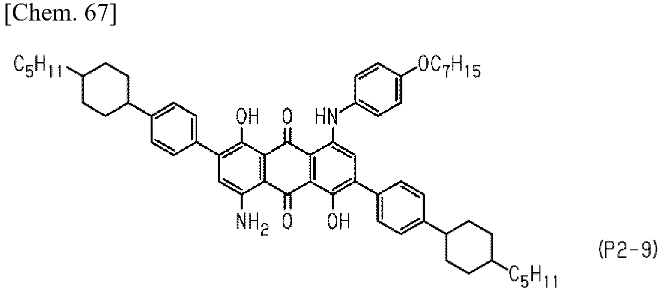

### (Test Example 2-10)

The pigment of Test Example 1-12 of the First Example was used as an anthraquinone derivative of Test Example 2-10. The anthraquinone derivative of Test Example 2-10 is a compound expressed by the following Formula (P2-10).

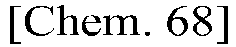

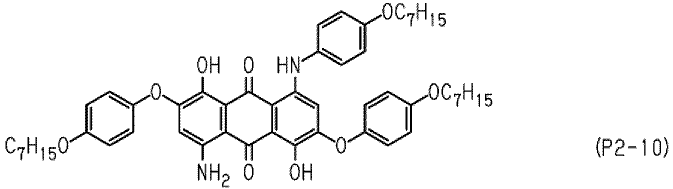

### (Test Example 2-11)

### <Synthesis of Precursor C8>

Precursor C8 was obtained by performing synthesis as in the pigment synthesis process of Test Example 1-12 of the First Example, except that Precursor A3 was replaced with Precursor C1. Precursor C8 is a compound expressed by the following Formula (2-e).

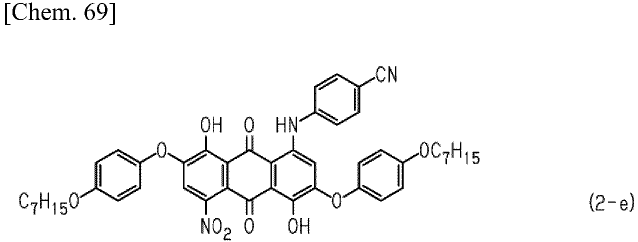

### <Pigment synthesis of Test Example 2-11>

An anthraquinone derivative of Test Example 2-11 was obtained by performing synthesis as in the pigment synthesis process of Test Example 2-1 described above, except that Precursor C2 was replaced with Precursor C8. The anthraquinone derivative of Test Example 2-11 is a compound expressed by the following Formula (P2-11).

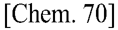

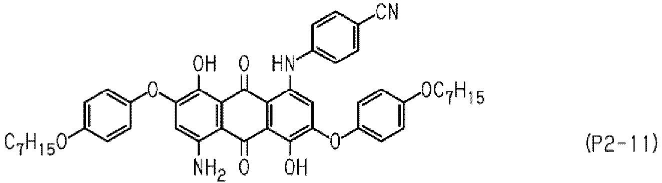

### (Evaluation method)

For the anthraquinone derivatives of Test Examples 2-1 to 2-11, evaluation test specimens were prepared as in the First Example, and absorption wavelength, coloring power, light resistance, and dichroic ratio were evaluated. When evaluating coloring power, Test Example 2-6 corresponding to the conventional anthraquinone derivative was used as a reference. Specifically, the test specimens having an absorbance higher than that of Test Example 2-6 were evaluated to be Good and those having an absorbance lower than or equal to that of Test Example 2-6 were evaluated to be Poor.

### (Evaluations)

Table 2 shows the structure, maximum absorption wavelength λmax, absorbance at the maximum absorption wavelength λmax, evaluation of coloring power, absorbance change ratio ΔAbs, evaluation of light resistance, and dichroic ratio of each anthraquinone derivative, for Test Examples 2-1 to 2-11. The structure of the anthraquinone derivative of each test example is expressed by the following Formula (I), and R, A, Y¹, Y² and Z of Table 2 correspond to R, A, Y¹, Y² and Z of Formula (I).

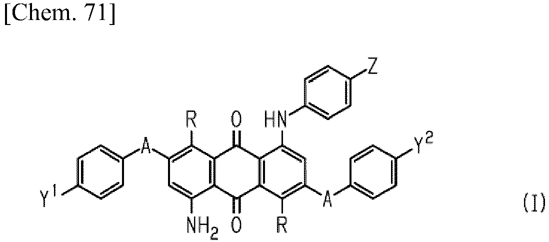

**[Table 2]**

| Test Ex. | Structure | | | | | λmax (nm) | Coloring power | | Light resistance | | Dichroic ratio |
|---|---|---|---|---|---|---|---|---|---|---|---|
| | R | A | Y¹ | Y² | Z | | Absorbance | Evaluation | ΔAbs (%) | Evaluation | |
| 2-1 | OH | Direct bond | OC₅H₁₁ | OC₅H₁₁ | CN | 674 | 0.60 | Good | <1 | Excellent | 8.4 |
| 2-2 | OH | Direct bond | OC₅H₁₁ | OC₅H₁₁ | CF₃ | 676 | 0.58 | Good | <1 | Excellent | 7.6 |
| 2-3 | OH | Direct bond | CYC₅H₁₁ | CyC₅H₁₁ | CN | 676 | 0.58 | Good | <1 | Excellent | 7.5 |
| 2-4 | OH | Direct bond | CYC₅H₁₁ | CyC₅H₁₁ | CF₃ | 676 | 0.58 | Good | <1 | Excellent | 7.6 |
| 2-5 | OH | Direct bond | N(CH₃)₂ | N(CH₃)₂ | CN | 706 | 0.75 | Good | <1 | Excellent | 9.0 |
| 2-6 | OH | Direct bond | OC₇H₁₅ | OC₇H₁₅ | C₇H₁₅ | 681 | 0.52 | Poor | <1 | Excellent | 11.4 |
| 2-7 | OH | Direct bond | OC₇H₁₅ | OC₇H₁₅ | OC₇H₁₅ | 681 | 0.42 | Poor | <1 | Excellent | 12.2 |
| 2-8 | OH | Direct bond | CYC₅H₁₁ | CYC₅H₁₁ | C₇H₁₅ | 683 | 0.45 | Poor | 3 | Good | 9.3 |
| 2-9 | OH | Direct bond | CYC₅H₁₁ | CYC₅H₁₁ | OC₇H₁₅ | 681 | 0.33 | Poor | <1 | Excellent | 10.0 |
| 2-10 | OH | O | OC₇H₁₅ | OC₇H₁₅ | OC₇H₁₅ | 632 | 0.23 | Poor | 43 | Poor | 12.2 |
| 2-11 | OH | O | OC₇H₁₅ | OC₇H₁₅ | CN | 601 | 0.52 | Poor | 50 | Poor | 9.8 |

As shown in Table 2, the anthraquinone derivatives of the test examples have a maximum absorption wavelength in the wavelength range of 600 nm or greater. Therefore, the anthraquinone derivatives of the test examples exhibit a blue color and can be used as cyanide pigments.

Furthermore, in Test Examples 2-1 to 2-9, where substituted phenyl groups are directly bonded at the β-positions, extremely high light resistance is achieved compared to Test Examples 2-10 and 2-11, where ether bonds are present at the β-positions.

In Test Examples 2-1 to 2-5, where the para substituent of the anilino group at the α-position is an electron-withdrawing group in the structure in which substituted phenyl groups are directly bonded at the β-positions, high absorbance and good coloring power are achieved compared to Test Examples 2-6 to 2-9, where the substituent in question is not an electron-withdrawing group. In Test Examples 2-1 to 2-5, high absorbance is achieved even relative to Test Examples 2-10 and 2-11, where ether bonds are present at the β-positions.

Referring to Test Examples 2-1 to 2-5, it was confirmed that in Test Example 2-5, where the phenyl groups at the β-positions have electron-donating groups as para substituents in addition to the para electron-withdrawing group being present at an α-position, particularly high absorbance is achieved compared to other test examples, where no such electron-donating groups are present.

It was confirmed that good dichroic ratio is achieved in all of the test examples.

### [Third Example: Test examples corresponding to third embodiment]

In the Third Example, synthesis was performed using Precursors A1 to A3 and B1 to B4 similar to those of the First Example.

### (Test Example 3-1)

The pigment of Test Example 2-8 of the Second Example was used as an anthraquinone derivative of Test Example 3-1. The anthraquinone derivative of Test Example 3-1 is a compound expressed by the following Formula (P3-1).

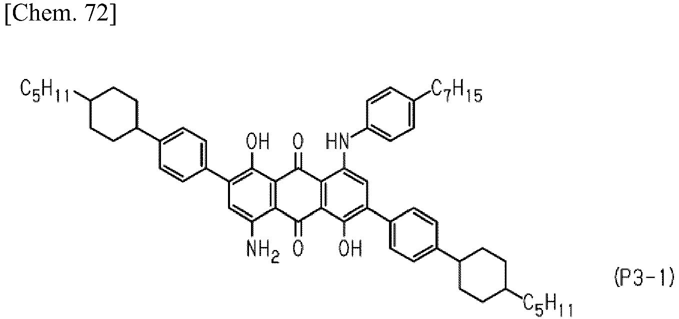

### (Test Example 3-2)

### <Pigment synthesis of Test Example 3-2>

An anthraquinone derivative of Test Example 3-2 was obtained by performing synthesis as in the pigment synthesis process of Test Example 1-1 of the First Example, except that 4-dimethylaminophenylboronic acid was replaced with 4-ethylcyclohexylphenylboronic acid. The anthraquinone derivative of Test Example 3-2 is a compound expressed by the following Formula (P3-2).

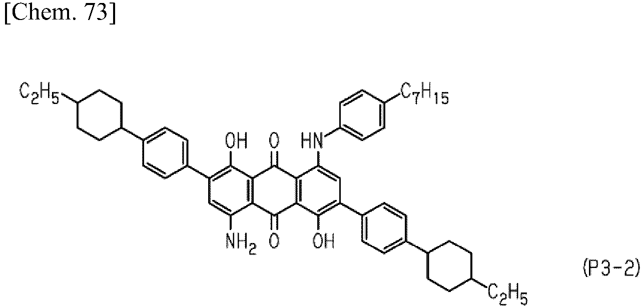

### (Test Example 3-3)

### <Pigment synthesis of Test Example 3-3>

An anthraquinone derivative of Test Example 3-3 was obtained by performing synthesis as in the pigment synthesis process of Test Example 1-1 of the First Example, except that 4-dimethylaminophenylboronic acid was replaced with 4-methylcyclohexylphenylboronic acid. The anthraquinone derivative of Test Example 3-3 is a compound expressed by the following Formula (P3-3).

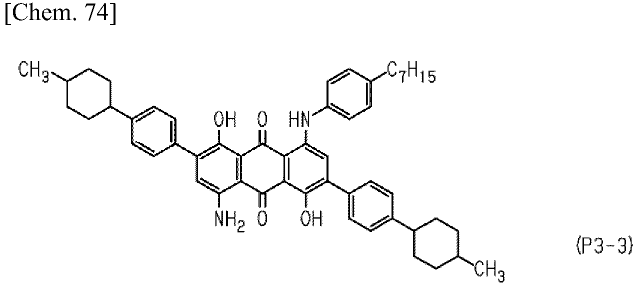

### (Test Example 3-4)

### <Pigment synthesis of Test Example 3-4>

An anthraquinone derivative of Test Example 3-4 was obtained by performing synthesis as in the pigment synthesis process of Test Example 1-1 of the First Example, except that 4-dimethylaminophenylboronic acid was replaced with cyclohexylphenylboronic acid. The anthraquinone derivative of Test Example 3-4 is a compound expressed by the following Formula (P3-4).

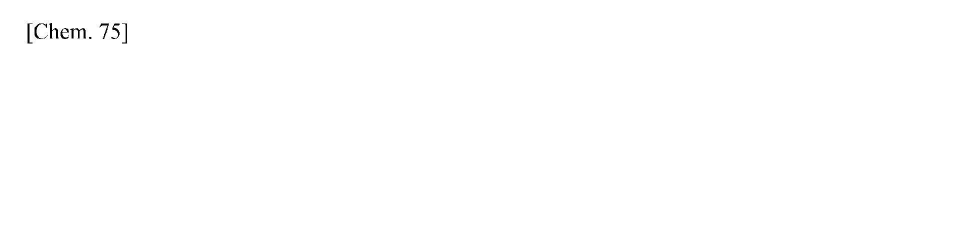

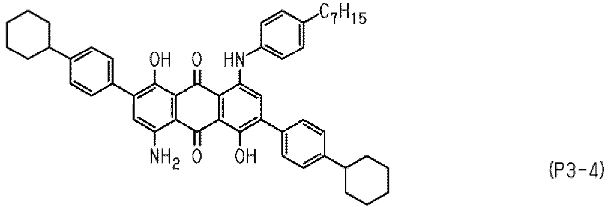

### (Test Example 3-5)

The pigment of Test Example 2-9 of the Second Example was used as an anthraquinone derivative of Test Example 3-5. The anthraquinone derivative of Test Example 3-5 is a compound expressed by the following Formula (P3-5).

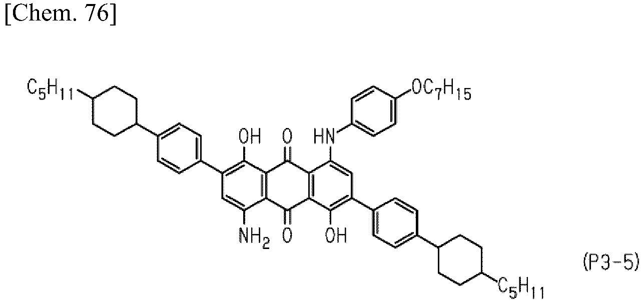

### (Test Example 3-6)

### <Pigment synthesis of Test Example 3-6>

In the pigment synthesis process of Test Example 1-1 of the First Example, 4-dimethylaminophenylboronic acid was replaced with 1.2 equivalents of 4-heptyloxyphenylboronic acid, and the reaction solution was stirred at 80°C for 1 hour, to which 1.2 equivalents of 4-pentylcyclohexylphenylboronic acid were further added, followed by heating and stirring at 80°C for at least 2 hours. Subsequently, an anthraquinone derivative of Test Example 3-6 was obtained by performing extraction and purification as in the pigment synthesis process of Test Example 1-1. The anthraquinone derivative of Test Example 3-6 is a compound expressed by the following Formula (P3-6).

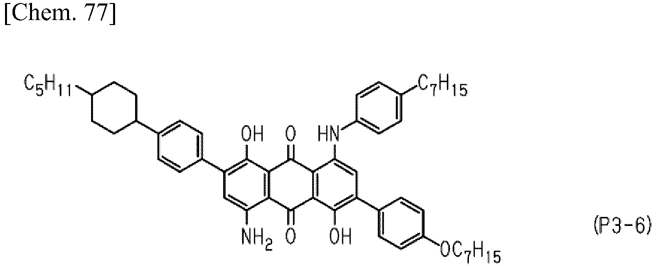

### (Test Example 3-7)

### <Pigment synthesis of Test Example 3-7>

In the pigment synthesis process of Test Example 1-1 of the First Example, 4-dimethylaminophenylboronic acid was replaced with 1.2 equivalents of 4-monobutylaminophenylboronic acid, and the reaction solution was stirred at 80°C for 1 hour, to which 1.2 equivalents of 4-pentylcyclohexylphenylboronic acid were further added, followed by heating and stirring at 80°C for at least 2 hours. Subsequently, an anthraquinone derivative of Test Example 3-7 was obtained by performing extraction and purification as in the pigment synthesis process of Test Example 1-1. The anthraquinone derivative of Test Example 3-7 is a compound expressed by the following Formula (P3-7).

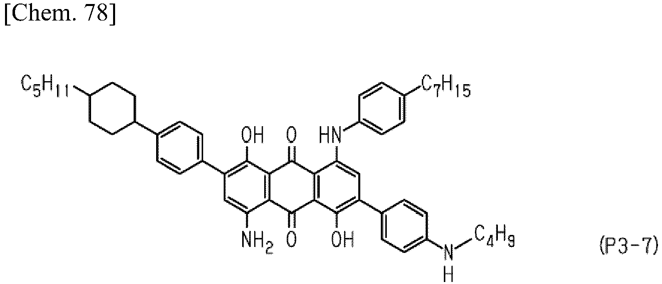

### (Test Example 3-8)

The pigment of Test Example 1-10 of the First Example was used as an anthraquinone derivative of Test Example 3-8. The anthraquinone derivative of Test Example 3-8 is a compound expressed by the following Formula (P3-8).

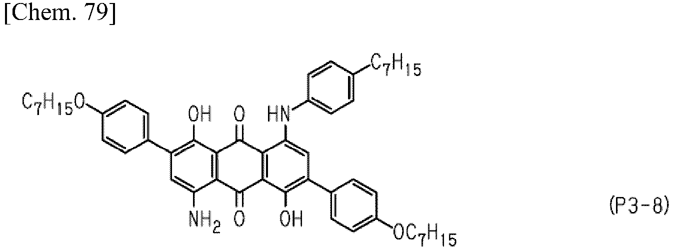

### (Test Example 3-9)

The pigment of Test Example 1-11 of the First Example was used as an anthraquinone derivative of Test Example 3-9. The anthraquinone derivative of Test Example 3-9 is a compound expressed by the following Formula (P3-9).

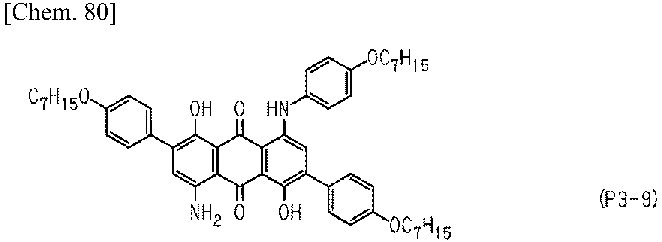

### (Test Example 3-10)

The pigment of Test Example 1-3 of the First Example was used as an anthraquinone derivative of Test Example 3-10. The anthraquinone derivative of Test Example 3-10 is a compound expressed by the following Formula (P3-10).

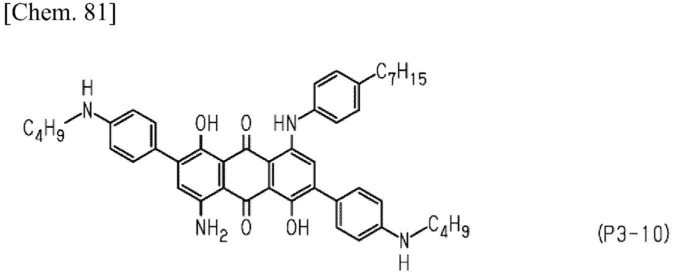

### (Test Example 3-11)

### <Pigment synthesis of Test Example 3-11>

An anthraquinone derivative of Test Example 3-11 was obtained by performing synthesis as in the synthesis process of Test Example 1-12 of the First Example, except that 4-heptyloxyphenol was replaced with 4-heptylcyclohexylphenol and that Precursor A3 was replaced with Precursor A2. The anthraquinone derivative of Test Example 3-11 is a compound expressed by the following Formula (P3-11).

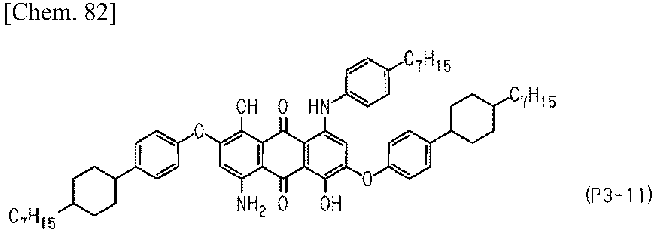

### (Test Example 3-12)

### <Synthesis of Precursor B9>

Precursor B9 was obtained by performing synthesis as in the synthesis process of Precursor B5 of the First Example, except that (4-piperidine-1-yl)phenylboronic acid was replaced with (4-pentylcyclohexyl)phenylboronic acid. Precursor B9 is a compound expressed by the following Formula (3-a).

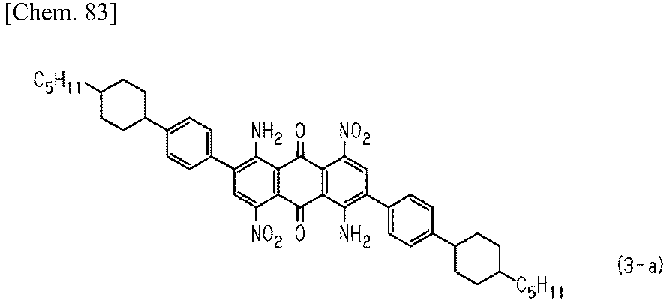

### <Synthesis of Precursor B10>

Precursor B10 was obtained by performing synthesis as in the synthesis process of Precursor B6 of the First Example, except that Precursor B5 was replaced with Precursor B9. Precursor B10 is a compound expressed by the following Formula (3-b).

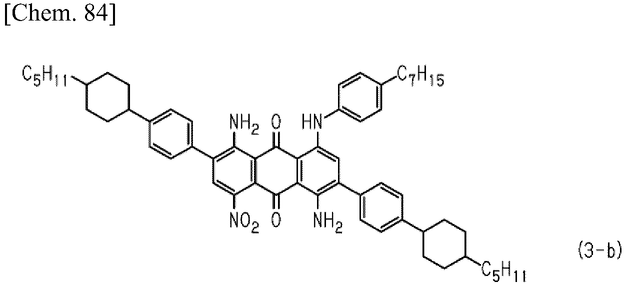

### <Pigment synthesis of Test Example 3-12>

An anthraquinone derivative of Test Example 3-12 was obtained by performing synthesis as in the pigment synthesis process of Test Example 1-14 of the First Example, except that Precursor B6 was replaced with Precursor B10. The anthraquinone derivative of Test Example 3-12 is a compound expressed by the following Formula (P3-12).

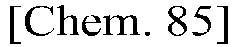

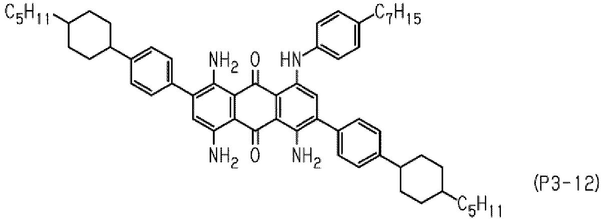

### (Test Example 3-13)

The pigment of Test Example 1-15 of the First Example was used as an anthraquinone derivative of Test Example 3-13. The anthraquinone derivative of Test Example 3-13 is a compound expressed by the following Formula (P3-13).

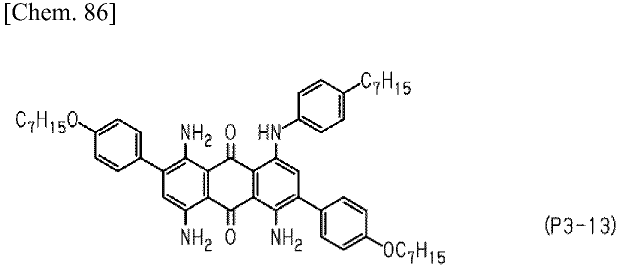

### (Evaluation method)

For the anthraquinone derivatives of Test Examples 3-1 to 3-13, evaluation test specimens were prepared as in the First Example, and absorption wavelength, light resistance, and dichroic ratio were evaluated.

A UV-visible absorption spectrum was normalized such that the absorbance at the maximum absorption wavelength was 1.0, and using the obtained spectrum, a sum of absorbances in the short-wavelength range of 380 nm or greater and 480 nm or less was calculated as a degree of secondary absorption. The sum of the absorbances is an integral value corresponding to the area in the range of 380 nm or greater and 480 nm or less in the normalized spectrum. It should be noted that absorption in the short-wavelength range of 380 nm or greater and 480 nm or less is defined to be a secondary absorption.

### (Evaluations)

Table 3 shows the structure, maximum absorption wavelength λmax, degree of secondary absorption, absorbance change ratio ΔAbs, evaluation of light resistance, and dichroic ratio of each anthraquinone derivative, for Test Examples 3-1 to 3-13. The structure of the anthraquinone derivative of each test example is expressed by the following Formula (I), and R, A, Y¹, Y² and Z of Table 3 correspond to R, A, Y¹, Y² and Z of Formula (I).

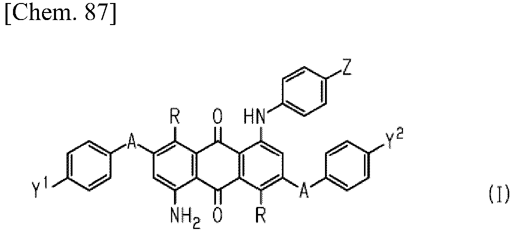

**[Table 3]**

| Test Ex. | Structure | | | | | λmax (nm) | Degree of secondary absorption | Light resistance | | Dichroic ratio |
|---|---|---|---|---|---|---|---|---|---|---|
| | R | A | Y¹ | Y² | Z | | | ΔAbs (%) | Evaluation | |
| 3-1 | OH | Direct bond | CyC₅H₁₁ | CyC₅H₁₁ | C₇H₁₅ | 683 | 13.73 | 3 | Good | 9.3 |
| 3-2 | OH | Direct bond | CyC₂H₅ | CyC₂H₅ | C₇H₁₅ | 684 | 13.47 | 2 | Excellent | 9.7 |
| 3-3 | OH | Direct bond | CyCH₃ | CyCH₃ | C₇H₁₅ | 683 | 13.21 | 2 | Excellent | 10.1 |
| 3-4 | OH | Direct bond | Cy | Cy | C₇H₁₅ | 681 | 13.56 | 2 | Excellent | 10.3 |
| 3-5 | OH | Direct bond | CyC₅H₁₁ | CyC₅H₁₁ | OC₇H₁₅ | 681 | 18.33 | <1 | Excellent | 10.0 |
| 3-6 | OH | Direct bond | CyC₅H₁₁ | OC₇H₁₅ | C₇H₁₅ | 683 | 15.58 | 1 | Excellent | 10.4 |
| 3-7 | OH | Direct bond | CyC₅H₁₁ | NHC₄H₉ | C₇H₁₅ | 689 | 16.34 | 6 | Good | 11.8 |
| 3-8 | OH | Direct bond | OC₇H₁₅ | OC₇H₁₅ | C₇H₁₅ | 681 | 17.80 | <1 | Excellent | 11.4 |
| 3-9 | OH | Direct bond | OC₇H₁₅ | OC₇H₁₅ | OC₇H₁₅ | 681 | 22.89 | <1 | Excellent | 12.2 |
| 3-10 | OH | Direct bond | NHC₄H₉ | NHC₄H₉ | C₇H₁₅ | 691 | 22.02 | 1 | Excellent | 13.4 |
| 3-11 | OH | O | CyC₇H₁₅ | CyC₇H₁₅ | C₇H₁₅ | 634 | 9.83 | 17 | Poor | 11.5 |
| 3-12 | NH₂ | Direct bond | CyC₅H₁₁ | CyC₅H₁₁ | C₇H₁₅ | 707 | 12.12 | 1 | Excellent | 8.0 |
| 3-13 | NH₂ | Direct bond | OC₇H₁₅ | OC₇H₁₅ | C₇H₁₅ | 699 | 17.90 | <1 | Excellent | 8.6 |

As shown in Table 3, the anthraquinone derivatives of the test examples have a maximum absorption wavelength in the wavelength range of 600 nm or greater. Therefore, the anthraquinone derivatives of the test examples exhibit a blue color and can be used as cyanide pigments.

In Test Examples 3-1 to 3-10, 3-12 and 3-13, where substituted phenyl groups are directly bonded at the β-positions, extremely high light resistance is achieved compared to Test Example 3-11, where ether bonds are present at the β-positions.

Comparing Test Examples 3-1 to 3-4 with Test Examples 3-8 and 3-10, it was confirmed that the degree of secondary absorption can be reduced due to the para substituents of the phenyl groups at the β-positions being substituted or unsubstituted cyclohexyl groups. Similarly, comparing Test Example 3-5 with Test Example 3-9 and comparing Test Example 3-12 with Test Example 3-13, it was also confirmed that the degree of secondary absorption is reduced due to the para substituents at the β-positions being cyclohexyl groups. Thus, it can be said that if the para substituents at the β-positions are cyclohexyl groups, an excellent blue hue with reduced yellowness can be produced in the anthraquinone derivative.

Comparing Test Example 3-6 with Test Example 3-8 and comparing Test Example 3-7 with Test Example 3-10, it was confirmed that the degree of secondary absorption is effectively suppressed even when only one of the para substituents at the β-position is a cyclohexyl group. Comparing Test Example 3-1 with Test Examples 3-6 and 3-7, it was confirmed that when both of the para substituents at the β-positions are cyclohexyl groups, the effect of suppressing the degree of secondary absorption is higher compared to the case where only one of them is a cyclohexyl group.

The test examples all achieved a good dichroic ratio, and it was confirmed that a high dichroic ratio can be achieved especially when two hydroxyl groups are present at α-positions.

### [Fourth Example: Test examples corresponding to fourth embodiment]

In the Fourth Example, synthesis was performed using precursors similar to those of the First to Third Examples.

### (Test Example 4-1)

### <Synthesis of Precursor B11>

4-heptyloxyphenol (0.45 parts by weight) and potassium carbonate (0.30 parts by weight) were placed in a two-necked flask and purged with nitrogen. To these materials, anhydrous N-methyl-2-pyrrolidone (20 parts by weight) was added, followed by stirring at 120°C for 3 hours. To this solution, Precursor B4 (0.50 parts by weight) was added, followed by stirring at 80°C for 7 hours. The reaction solution was returned to room temperature, to which water/dichloromethane was added to separate the solution. The organic phase obtained as a result of the separation was dried with sodium sulfate and then concentrated using an evaporator. Using silica gel column chromatography (eluent: hexane/dichloromethane = 1/4 with 1 mass% triethylamine added), the resultant material was purified and the recovered material was dried at 60°C under reduced pressure to thereby obtain Precursor B11 as a red powder. Precursor B11 is a compound expressed by the following Formula (4-a).

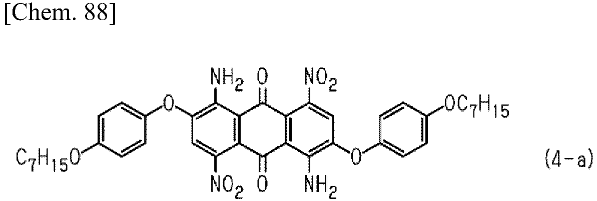

### <Synthesis of Precursor B12>

Precursor B11 (0.20 parts by weight) was placed in a two-necked flask and purged with nitrogen. To this material, nitrobenzene (4.8 parts by weight) and 4-heptylaniline (0.52 parts by weight) were added, followed by stirring at 200°C for 12 hours. The reaction solution was dried at 75°C under reduced pressure to distill away the nitrobenzene and obtain the residue. Methanol was poured into the residue, and the precipitated powder was recovered by suction filtration to obtain Precursor B12 as a purple powder. Precursor B12 is a compound expressed by the following Formula (4-b).

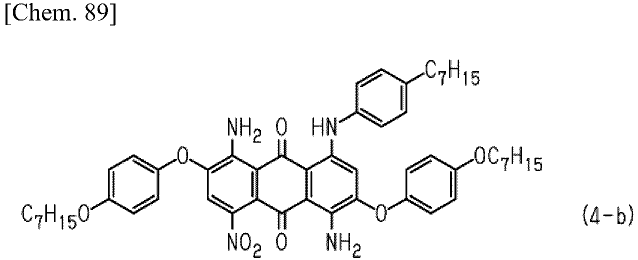

### <Pigment synthesis of Test Example 4-1>

An anthraquinone derivative of Test Example 4-1 was obtained by performing synthesis as in the pigment synthesis process of Test Example 1-14 of the First Example, except that Precursor B6 was replaced with Precursor B12. The anthraquinone derivative of Test Example 4-1 is a compound expressed by the following Formula (P4-1).

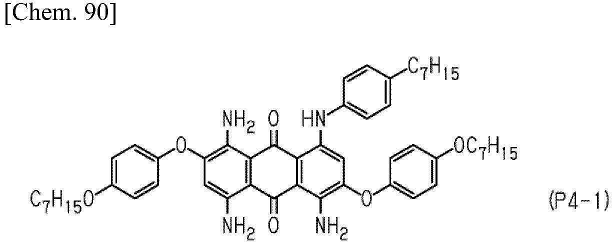

### (Test Example 4-2)

The pigment of Test Example 1-10 of the First Example was used as an anthraquinone derivative of Test Example 4-2. The anthraquinone derivative of Test Example 4-2 is a compound expressed by the following Formula (P4-2).

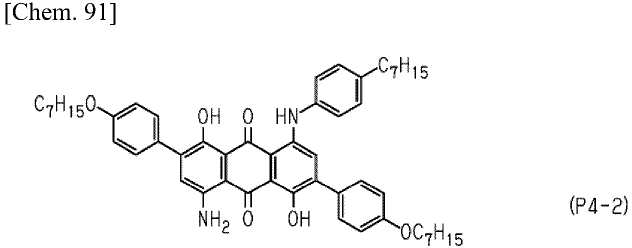

### (Test Example 4-3)

The pigment of Test Example 2-8 of the Second Example was used as an anthraquinone derivative of Test Example 4-3. The anthraquinone derivative of Test Example 4-3 is a compound expressed by the following Formula (P4-3).

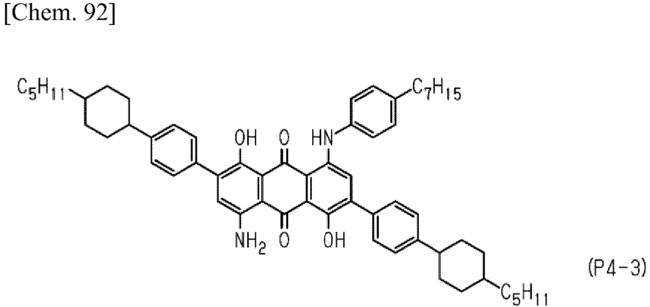

### (Test Example 4-4)

The pigment of Test Example 1-11 of the First Example was used as an anthraquinone derivative of Test Example 4-4. The anthraquinone derivative of Test Example 4-4 is a compound expressed by the following Formula (P4-4).

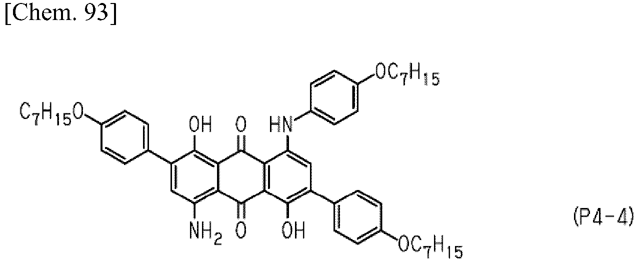

### (Test Example 4-5)

### <Pigment synthesis of Test Example 4-5>

In the pigment synthesis process of Test Example 1-1 of the First Example, 4-dimethylaminophenylboronic acid was replaced with 1.2 equivalents of 4-heptyloxyphenylboronic acid, and the reaction solution was stirred at 80°C for 1 hour, to which 1.2 equivalents of 4-pentyloxybiphenylboronic acid were further added, followed by heating and stirring at 80°C for at least 2 hours. Subsequently, an anthraquinone derivative of Test Example 4-5 was obtained by performing extraction and purification as in the pigment synthesis process of Test Example 1-1 described above. The anthraquinone derivative of Test Example 4-5 is a compound expressed by the following Formula (P4-5).

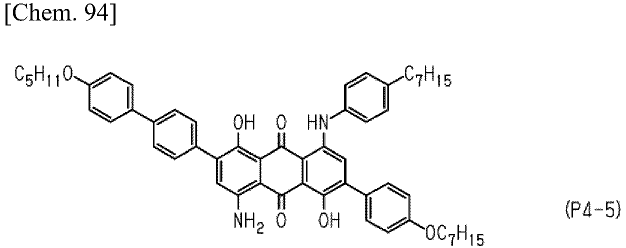

### (Test Example 4-6)

The pigment of Test Example 3-6 of the Third Example was used as an anthraquinone derivative of Test Example 4-6. The anthraquinone derivative of Test Example 4-6 is a compound expressed by the following Formula (P4-6).

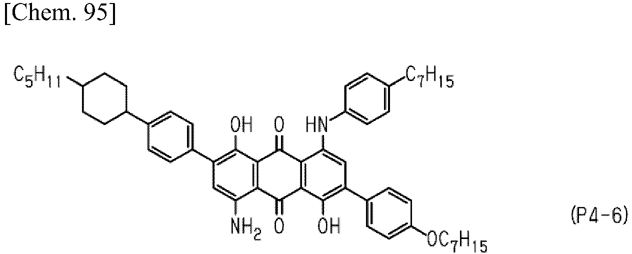

### (Test Example 4-7)

The pigment of Test Example 2-9 of the Second Example was used as an anthraquinone derivative of Test Example 4-7. The anthraquinone derivative of Test Example 4-7 is a compound expressed by the following Formula (P4-7).

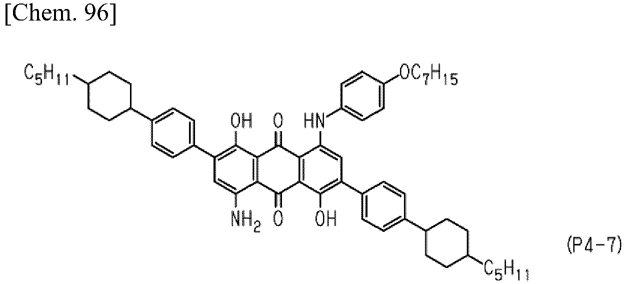

### (Test Example 4-8)

The pigment of Test Example 2-3 of the Second Example was used as an anthraquinone derivative of Test Example 4-8. The anthraquinone derivative of Test Example 4-8 is a compound expressed by the following Formula (P4-8).

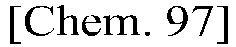

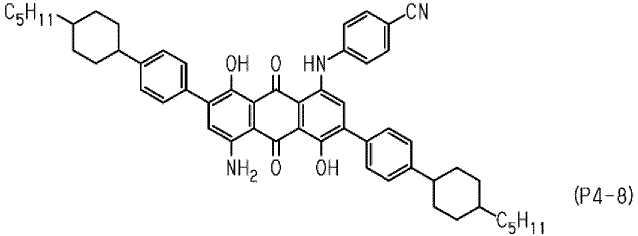

### (Test Example 4-9)

The pigment of Test Example 3-7 of the Third Example was used as an anthraquinone derivative of Test Example 4-9. The anthraquinone derivative of Test Example 4-9 is a compound expressed by the following Formula (P4-9).

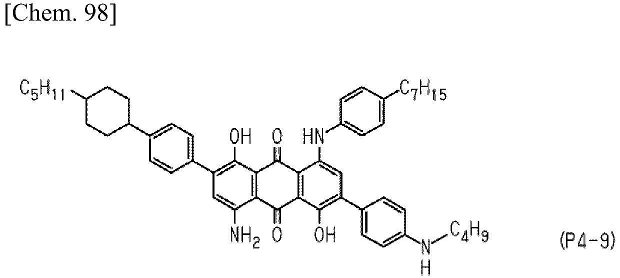

### (Test Example 4-10 to 4-12)

### <Synthesis of Precursor D1>

Toluene (100 parts by weight), ethanol (20 parts by weight), water (10 parts by weight), Precursor A1 (1 part by weight), 4-pentylcyclohexylphenylboronic acid (1.2 parts by weight), triethylamine (0.62 parts by weight), and tris(dibenzylideneacetone)dipalladium (0.2 parts by weight) were placed in a two-necked round-bottom flask, and these materials were heated and stirred at 80°C for at least 2 hours. After confirming completion of reaction using thin-layer chromatography, the reaction solution was returned to room temperature, to which distilled water was added to perform extraction with ethyl acetate. To the organic phase obtained, sodium sulfate was added, followed by drying, which was further followed by removing the solvent under reduced pressure using an evaporator. The resultant solid was purified using column chromatography (eluent: hexane/dichloromethane = 1/1 to 2/3) to obtain Precursor D1 as a dark red solid. Precursor D1 is a compound expressed by the following Formula (4-c).

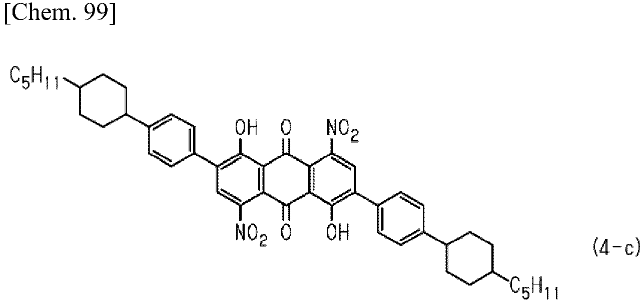

### <Synthesis of Precursor D2>

Precursor D2 was obtained by performing synthesis as in the synthesis process of Precursor D1 described above, except that 4-pentylcyclohexylphenylboronic acid was replaced with 4-heptyloxyphenylboronic acid. Precursor D2 is a compound expressed by the following Formula (4-d).

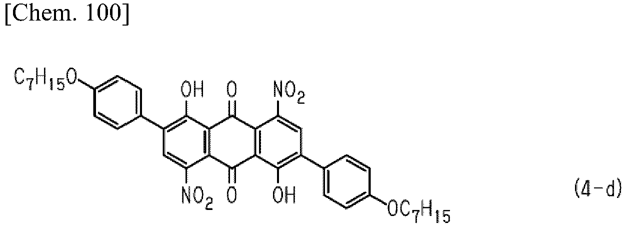

### <Synthesis of Precursor D3>

Precursor D3 was obtained by performing synthesis as in the synthesis process of Precursor D1 described above, except that 4-pentylcyclohexylphenylboronic acid was replaced with 4-monobutylaminophenylboronic acid. Precursor D3 is a compound expressed by the following Formula (4-e).

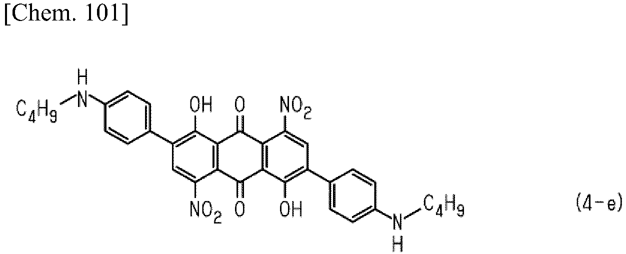

### <Pigment synthesis of Test Example 4-10>

Precursor D1 (1.0 part by weight) was placed in a two-necked round-bottom flask, and the inside of the system was purged with nitrogen, to which tetrahydrofuran (18.0 parts by weight) was added. Next, using another flask, 4-methoxybenzenethiol (3.3 parts by weight) was mixed with pyridine (1.6 parts by weight) and the mixture was stirred at room temperature for 30 minutes to prepare a solution which was then added to the solution containing Precursor D1 in the two-necked round-bottom flask, followed by heating and stirring at 50°C. After confirming completion of reaction using thin-layer chromatography, the reaction solution was left to stand to cool to room temperature, to which dilute hydrochloric acid (mixture of 35% hydrochloric acid (20.0 parts by weight) and pure water (80.0 parts by weight)) were added, and the precipitated solid was recovered by filtration. The recovered solid (1.0 part by weight) and a zinc powder (0.5 parts by weight) were placed in a two-necked flask and the inside of the system was purged with nitrogen. To these materials, dichloromethane (200.0 parts by weight) and acetic acid (18.0 parts by weight) were added, followed by stirring at room temperature. After confirming completion of reaction using thin-layer chromatography, the reaction mixture was filtered, and pure water was added to the filtrate to perform extraction with dichloromethane. To the organic phase obtained, sodium sulfate was added, followed by drying, which was further followed by removing the solvent under reduced pressure using an evaporator. The obtained residue was purified using column chromatography (eluent: hexane/dichloromethane = 1/1 to 1/3) to obtain an anthraquinone derivative of Test Example 4-10 as a deep blue solid. The anthraquinone derivative of Test Example 4-10 is a compound expressed by the following Formula (P4-10).

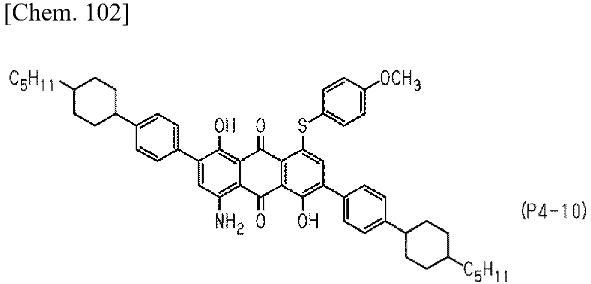

### <Pigment synthesis of Test Example 4-11>

An anthraquinone derivative of Test Example 4-11 was obtained by performing synthesis as in the pigment synthesis process of Test Example 4-10 described above, except that Precursor D1 was replaced with Precursor D2. The anthraquinone derivative of Test Example 4-11 is a compound expressed by the following Formula (P4-11).

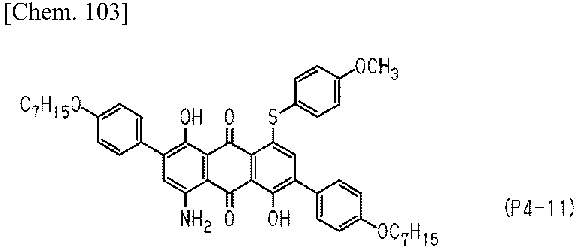

### <Pigment synthesis of Test Example 4-12>

An anthraquinone derivative of Test Example 4-12 was obtained by performing synthesis as in the pigment synthesis process of Test Example 4-10 described above, except that Precursor D1 was replaced with Precursor D3. The anthraquinone derivative of Test Example 4-12 is a compound expressed by the following Formula (P4-12).

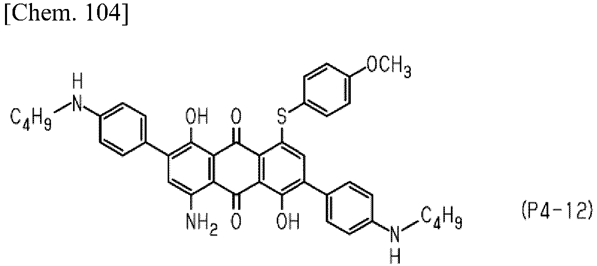

### (Test Example 4-13)

### <Pigment synthesis of Test Example 4-13>

4-heptyloxyphenol (0.36 parts by weight) and potassium carbonate (0.24 parts by weight) were placed in a two-necked flask and purged with nitrogen, to which anhydrous N-methyl-2-pyrrolidone (10 parts by weight) was added, followed by stirring at 120°C for 3 hours. To this solution, Precursor A2 (0.50 parts by weight) was added, followed by stirring at 120°C for 7 hours. The reaction solution was returned to room temperature, to which water/dichloromethane was added to separate the solution. The organic phase obtained was dried with sodium sulfate and then concentrated using an evaporator. Then, the resultant material was purified using silica gel column chromatography and dried at 60°C under reduced pressure to obtain an anthraquinone derivative of Test Example 4-13 as a blue powder. The anthraquinone derivative of Test Example 4-13 is a compound expressed by the following Formula (P4-13).

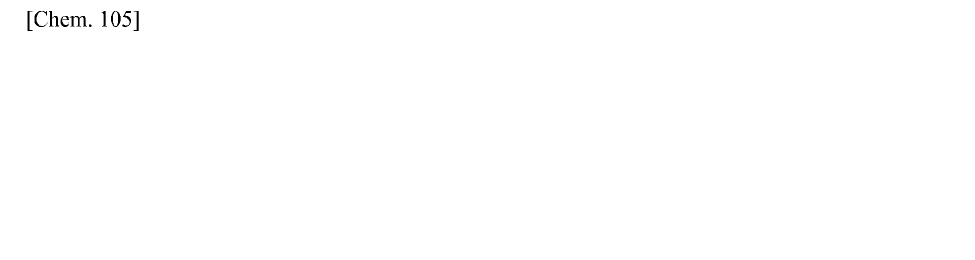

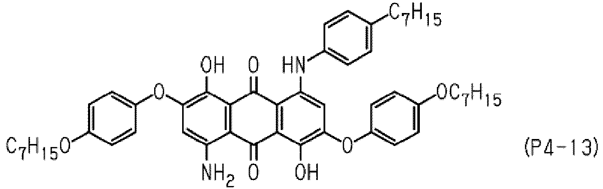

### (Test Example 4-14)

### <Pigment synthesis of Test Example 4-14>

An anthraquinone derivative of Test Example 4-14 was obtained by performing synthesis as in the pigment synthesis process of Test Example 4-13 described above, except that 4-heptyloxyphenol was replaced with 4-heptylphenol. The anthraquinone derivative of Test Example 4-14 is a compound expressed by the following Formula (P4-14).

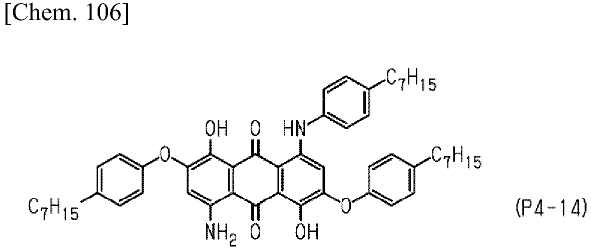

### (Test Example 4-15)

### <Pigment synthesis of Test Example 4-15>

An anthraquinone derivative of Test Example 4-15 was obtained by performing synthesis as in the pigment synthesis process of Test Example 4-13 described above, except that 4-heptyloxyphenol was replaced with 4-heptylcyclohexylphenol. The anthraquinone derivative of Test Example 4-15 is a compound expressed by the following Formula (P4-15). The anthraquinone derivative of Test Example 4-15 is the same compound as the anthraquinone derivative of Test Example 3-11.

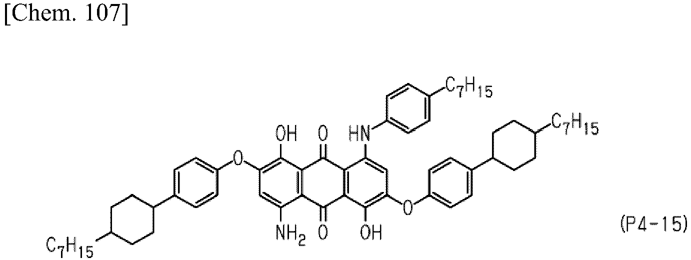

### (Evaluation method)

### <Evaluation of absorption wavelength and light resistance>

For the anthraquinone derivatives of Test Examples 4-1 to 4-15, evaluation test specimens were prepared as in the First Example, and absorption wavelength and light resistance were evaluated.

### <Calculation of total energy difference ΔE>

For the anthraquinone derivatives of Test Examples 4-1 to 4-15, a total energy difference ΔE before and after the reaction expressed by Reaction Formula (4-2) above was calculated. The total energy difference ΔE was calculated using the quantum chemistry calculation program GAMESS, with B3LYP used as the functional and 6-31G(d) used for the basis functions, through the following procedure.
(1) The anthraquinone derivative of each test example was structurally optimized using an SCF calculation to obtain a total molecular energy Ep in the optimized structure.
(2) Hydrogen was added to the anthraquinone skeleton of the structurally optimized anthraquinone derivative to obtain an initial structure of a hydrogen adduct, which was structurally optimized using an SCF calculation. Then, for the hydrogen adduct obtained, a total molecular energy Ek in the optimized structure was obtained.

Hydrogen adducts can have four types of stereoisomers depending on the positional relationship between the substituents at the β-positions. Of the four stereoisomers, the most stable structure, where the energy is most stable, is considered to be the most likely to be produced. Therefore, a most stable structure was adopted as a three-dimensional structure of the hydrogen adduct. The most stable structure was searched for by performing a relaxed scan calculation in which the sites where the substituents were bonded at the β-positions were subjected to dihedral rotation.

(3) A total energy difference ΔE was calculated based on a formula ΔE=Ek-Ep.

### (Evaluations)

Table 4 shows the structure, maximum absorption wavelength λmax, total energy difference ΔE, absorbance change ratio ΔAbs, and evaluation of light resistance of each anthraquinone derivative, for Test Examples 4-1 to 4-15. The structure of the anthraquinone derivative of each test example is expressed by the following Formula (II), and R, A, X, Y¹, Y² and Z of Table 4 correspond to R, A, X, Y¹, Y² and Z of Formula (II).

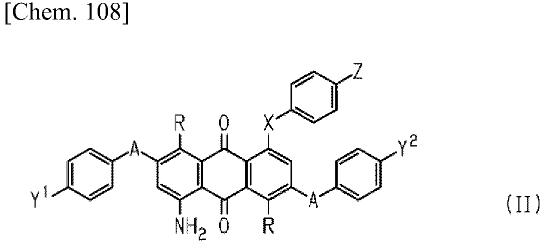

**[Table 4]**

| Test Ex. | Structure | | | | | | λmax (nm) | ΔE | Light resistance | |
|---|---|---|---|---|---|---|---|---|---|---|
| | R | A | X | Y¹ | Y² | Z | | | ΔAbs (%) | Evaluation |
| 4-1 | NH₂ | O | NH | OC₇H₁₅ | OC₇H₁₅ | C₇H₁₅ | 656 | -11.1 | <1 | Excellent |
| 4-2 | OH | Direct bond | NH | OC₇H₁₅ | OC₇H₁₅ | C₇H₁₅ | 681 | -11.9 | <1 | Excellent |
| 4-3 | OH | Direct bond | NH | CyC₅H₁₁ | CyC₅H₁₁ | C₇H₁₅ | 683 | -12.8 | 3 | Good |
| 4-4 | OH | Direct bond | NH | OC₇H₁₅ | OC₇H₁₅ | OC₇H₁₅ | 681 | -11.9 | <1 | Excellent |
| 4-5 | OH | Direct bond | NH | PhOC₅H₁₁ | OC₇H₁₅ | C₇H₁₅ | 684 | -12.3 | 4 | Good |
| 4-6 | OH | Direct bond | NH | CyC₅H₁₁ | OC₇H₁₅ | C₇H₁₅ | 683 | -12.3 | 1 | Excellent |
| 4-7 | OH | Direct bond | NH | CyC₅H₁₁ | CyC₅H₁₁ | OC₇H₁₅ | 681 | -12.9 | <1 | Excellent |
| 4-8 | OH | Direct bond | NH | CyC₅H₁₁ | CyC₅H₁₁ | CN | 676 | -12.6 | <1 | Excellent |
| 4-9 | OH | Direct bond | NH | CyC₅H₁₁ | NHC₄H₉ | C₇H₁₅ | 689 | -11.9 | 6 | Good |
| 4-10 | OH | Direct bond | S | CyC₅H₁₁ | CyC₅H₁₁ | OCH₃ | 592 | -9.6 | <1 | Excellent |
| 4-11 | OH | Direct bond | S | OC₇H₁₅ | OC₇H₁₅ | OCH₃ | 638 | -8.7 | <1 | Excellent |
| 4-12 | OH | Direct bond | S | NHC₄H₉ | NHC₄H₉ | OCH₃ | 650 | -8.9 | <1 | Excellent |
| 4-13 | OH | O | NH | OC₇H₁₅ | OC₇H₁₅ | C₇H₁₅ | 632 | -17.3 | 30 | Poor |
| 4-14 | OH | O | NH | C₇H₁₅ | C₇H₁₅ | C₇H₁₅ | 633 | -18.4 | 54 | Poor |
| 4-15 | OH | O | NH | CyC₇H₁₅ | CyC₇H₁₅ | C₇H₁₅ | 634 | -18.3 | 17 | Poor |

As shown in Table 4, the anthraquinone derivatives of the test examples have a maximum absorption wavelength in the wavelength range of 580 nm or greater, and therefore can be used as cyanide pigments.

In Test Examples 4-1 to 4-12, where the total energy difference ΔE is -16 kcal/mol or more, high light resistance is achieved. However, in Test Examples 4-13 to 4-15, where the total energy difference ΔE is less than -16 kcal/mol, light resistance is deteriorated. As can be seen from the results shown in Table 4, structures without any hydroxyl group at an α-position, or structures with substituents directly bonded at the β-positions, increase the total energy difference ΔE and achieve higher light resistance.

### [Fifth Example: Test examples corresponding to fifth embodiment]

In the Fifth Example, synthesis was performed using precursors similar to those of the First to Fourth Examples.

### (Test Example 5-1)

The pigment of Test Example 1-10 of the First Example was used as an anthraquinone derivative of Test Example 5-1. The anthraquinone derivative of Test Example 5-1 is a compound expressed by the following Formula (P5-1).

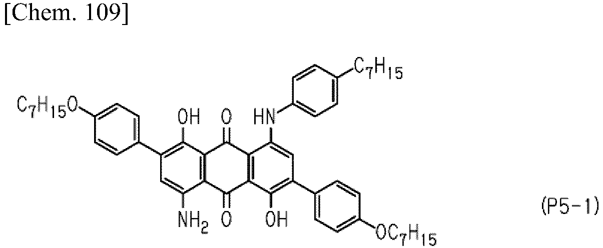

### (Test Example 5-2)

The pigment of Test Example 1-11 of the First Example was used as an anthraquinone derivative of Test Example 5-2. The anthraquinone derivative of Test Example 5-2 is a compound expressed by the following Formula (P5-2).

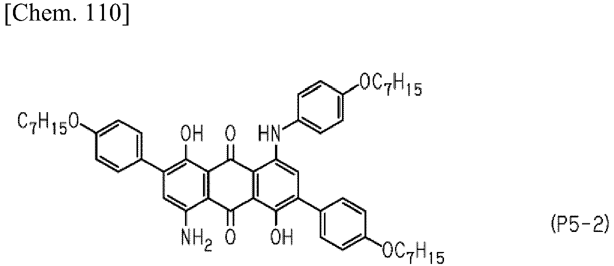

### (Test Example 5-3)

### <Pigment synthesis of Test Example 5-3>

In the pigment synthesis process of Test Example 1-1 of the First Example, Precursor A2 was replaced with Precursor A3, to which 1.2 equivalents of 4-heptyloxyphenylboronic acid were added in place of 4-dimethylaminophenylboronic acid, and the reaction solution was stirred at 80°C for 1 hour, to which 1.2 equivalents of 4-(trans-4-pentylcyclohexyl)phenylboronic acid were further added, followed by heating and stirring at 80°C for at least 2 hours. Subsequently, an anthraquinone derivative of Test Example 5-3 was obtained by performing extraction and purification as in the pigment synthesis process of Test Example 1-1. The anthraquinone derivative of Test Example 5-3 is a compound expressed by the following Formula (P5-3).

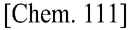

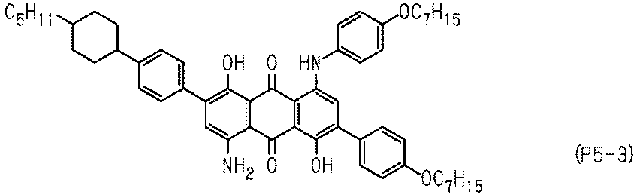

### (Test Example 5-4)

The pigment of Test Example 1-2 of the First Example was used as an anthraquinone derivative of Test Example 5-4. The anthraquinone derivative of Test Example 5-4 is a compound expressed by the following Formula (P5-4).

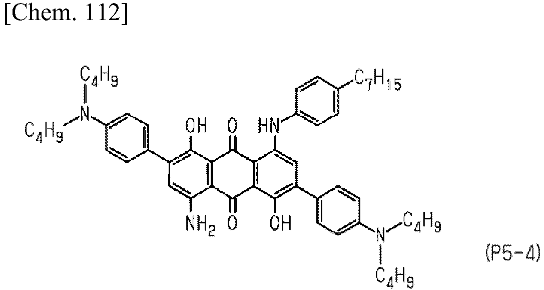

### (Test Example 5-5)

### <Pigment synthesis of Test Example 5-5>

An anthraquinone derivative of Test Example 5-5 was obtained by performing synthesis as in the pigment synthesis process of Test Example 4-10 of the Fourth Example, except that Precursor D1 was replaced with Precursor D2 and that 4-methoxybenzenethiol was replaced with 4-heptyloxybenzenethiol. The anthraquinone derivative of Test Example 5-5 is a compound expressed by the following Formula (P5-5).

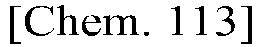

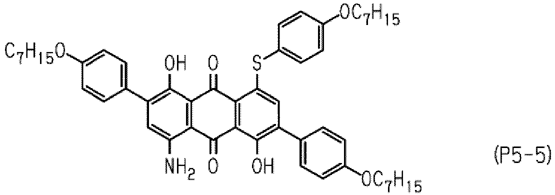

### (Test Example 5-6)

The pigment of Test Example 4-11 of the Fourth Example was used as an anthraquinone derivative of Test Example 5-6. The anthraquinone derivative of Test Example 5-6 is a compound expressed by the following Formula (P5-6).

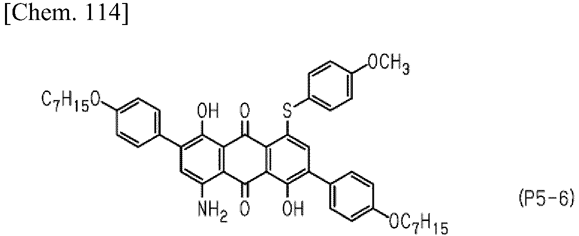

### (Test Example 5-7)

### <Pigment synthesis of Test Example 5-7>

An anthraquinone derivative of Test Example 5-7 was obtained by performing synthesis as in the pigment synthesis process of Test Example 4-10 of the Fourth Example, except that 4-methoxybenzenethiol was replaced with 4-heptyloxybenzenethiol. The anthraquinone derivative of Test Example 5-7 is a compound expressed by the following Formula (P5-7).

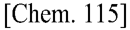

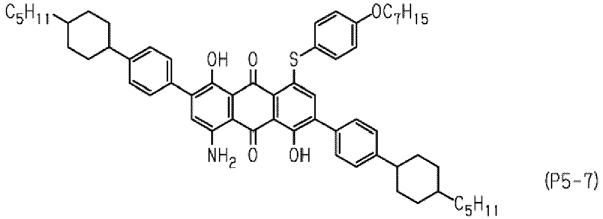

### (Test Example 5-8)

### <Pigment synthesis of Test Example 5-8>

An anthraquinone derivative of Test Example 5-8 was obtained by performing synthesis as in the pigment synthesis process of Test Example 4-10 of the Fourth Example, except that Precursor D1 was replaced with Precursor D2 and that 4-methoxybenzenethiol was replaced with 4-cyanobenzenethiol. The anthraquinone derivative of Test Example 5-8 is a compound expressed by the following Formula (P5-8).

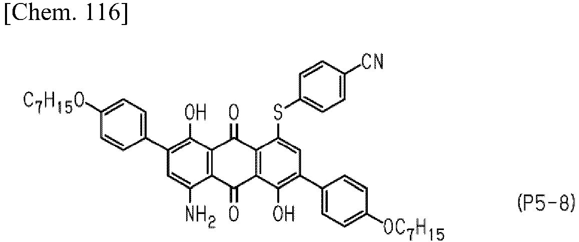

### (Test Example 5-9)

### <Synthesis of Precursor B13>

Precursor B13 was obtained by performing synthesis as in the synthesis process of Precursor A2 of the First Example, except that Precursor A1 was replaced with Precursor B4. Precursor B13 is a compound expressed by the following Formula (5-a).

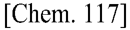

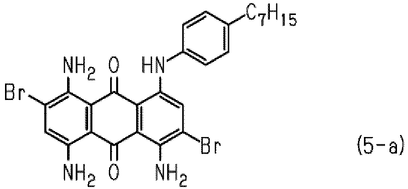

### <Pigment synthesis of Test Example 5-9>

An anthraquinone derivative of Test Example 5-9 was obtained by performing synthesis as in the pigment synthesis process of Test Example 1-1 of the First Example, except that Precursor A2 was replaced with Precursor B13 and that 4-dimethylaminophenylboronic acid was replaced with 4-heptyloxyphenylboronic acid. The anthraquinone derivative of Test Example 5-9 is a compound expressed by the following Formula (P5-9). The anthraquinone derivative of Test Example 5-9 is the same compound as the anthraquinone derivative of Test Example 1-15.

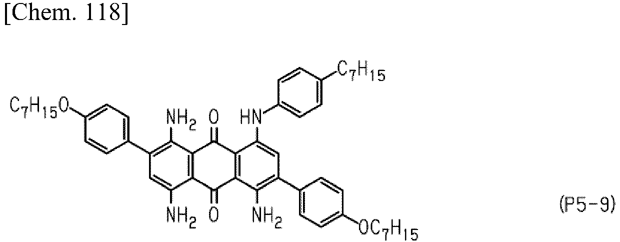

### (Test Example 5-10)

The pigment of Test Example 4-1 of the Fourth Example was used as an anthraquinone derivative of Test Example 5-10. The anthraquinone derivative of Test Example 5-10 is a compound expressed by the following Formula (P5-10).

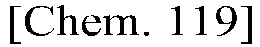

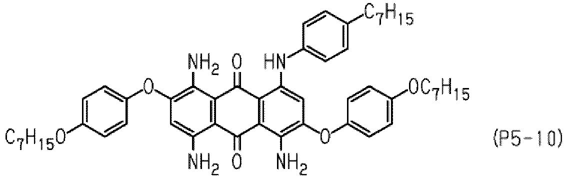

### (Test Example 5-11)

The pigment of Test Example 4-13 of the Fourth Example was used as an anthraquinone derivative of Test Example 5-11. The anthraquinone derivative of Test Example 5-11 is a compound expressed by the following Formula (P5-11).

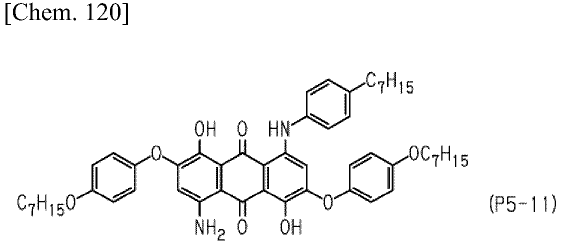

### (Test Example 5-12)

The pigment of Test Example 4-14 of the Fourth Example was used as an anthraquinone derivative of Test Example 5-12. The anthraquinone derivative of Test Example 5-12 is a compound expressed by the following Formula (P5-12).

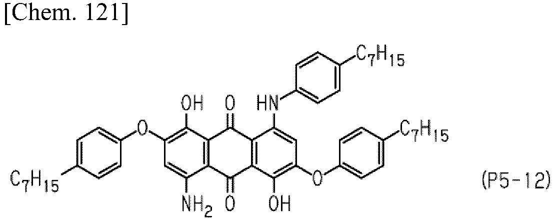

### (Evaluation method)

### <Evaluation of absorption wavelength and light resistance>

For the anthraquinone derivatives of Test Examples 5-1 to 5-12, evaluation test specimens were prepared as in the First Example, and absorption wavelength and light resistance were evaluated.

### <Thermogravimetric measurements>

The anthraquinone derivatives of Test Examples 5-1 to 5-12 were subjected to weight measurements using a differential heat/thermogravimetry simultaneous measurement device (STA7200RV manufactured by Hitachi High-Tech Science Corporation) to measure the weight of the samples after heating from 30°C to 550°C under the conditions of gas flow rate: 200 mL/min and heating rate: 10°C/min. Air was used as the gas. The 10% weight loss temperature was obtained with reference to the weight at the start of temperature rise.

### (Evaluations)

Table 5 shows the structure, maximum absorption wavelength λmax, 10% weight loss temperature, absorbance change ratio ΔAbs, and evaluation of light resistance of each anthraquinone derivative, for Test Examples 5-1 to 5-12. The structure of the anthraquinone derivative of each test example is expressed by the following Formula (II), and R, A, X, Y¹, Y² and Z of Table 5 correspond to R, A, X, Y¹, Y² and Z of Formula (II).

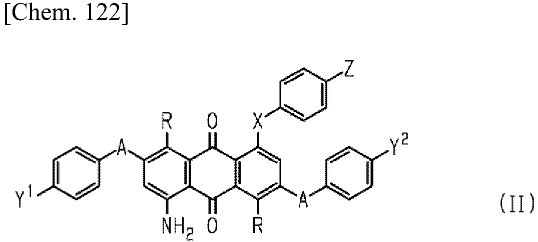

**[Table 5]**

| Test Ex, | Structure | | | | | | λmax (nm) | 10% weight loss temp. (°C) | Light resistance | |
|---|---|---|---|---|---|---|---|---|---|---|
| | R | A | X | Y¹ | Y² | Z | | | ΔAbs (%) | Evaluation |
| 5-1 | OH | Direct bond | NH | OC₇H₁₅ | OC₇H₁₅ | C₇H₁₅ | 681 | 400 | <1 | Excellent |
| 5-2 | OH | Direct bond | NH | OC₇H₁₅ | OC₇H₁₅ | OC₇H₁₅ | 681 | 408 | <1 | Excellent |
| 5-3 | OH | Direct bond | NH | CyC₅H₁₁ | OC₇H₁₅ | OC₇H₁₅ | 683 | 411 | 1 | Excellent |
| 5-4 | OH | Direct bond | NH | N(C₄H₉)₂ | N(C₄H₉)₂ | C₇H₁₅ | 701 | 356 | 3 | Good |
| 5-5 | OH | Direct bond | S | OC₇H₁₅ | OC₇H₁₅ | OC₇H₁₅ | 638 | 365 | 2 | Excellent |
| 5-6 | OH | Direct bond | S | OC₇H₁₅ | OC₇H₁₅ | OCH₃ | 638 | 369 | <1 | Excellent |
| 5-7 | OH | Direct bond | S | CyC₅H₁₁ | CyC₅H₁₁ | OC₇H₁₅ | 592 | 388 | 2 | Excellent |
| 5-8 | OH | Direct bond | S | OC₇H₁₅ | OC₇H₁₅ | CN | 629 | 352 | 5 | Good |
| 5-9 | NH₂ | Direct bond | NH | OC₇H₁₅ | OC₇H₁₅ | C₇H₁₅ | 699 | 397 | <1 | Excellent |
| 5-10 | NH₂ | O | NH | OC₇H₁₅ | OC₇H₁₅ | C₇H₁₅ | 656 | 366 | <1 | Excellent |
| 5-11 | OH | O | NH | OC₇H₁₅ | OC₇H₁₅ | C₇H₁₅ | 632 | 344 | 30 | Poor |
| 5-12 | OH | O | NH | C₇H₁₅ | C₇H₁₅ | C₇H₁₅ | 633 | 337 | 54 | Poor |

As shown in Table 5, the anthraquinone derivatives of the test examples have a maximum absorption wavelength in the wavelength range of 580 nm or greater, and therefore can be used as cyanide pigments.

In Test Examples 5-1 to 5-10, where the 10% weight loss temperature is 350°C or higher, high light resistance is achieved. However, in Test Examples 5-11 and 5-12, where the 10% weight loss temperature is less than 350°C, light resistance is deteriorated.

As can be seen from the results shown in Table 5, in the structures without any hydroxyl group at an α-position, or the structures with substituents directly bonded at the β-positions, the 10% weight loss temperature is increased and higher light resistance is achieved. Furthermore, it was confirmed that structures with X representing -NH- tend to have a higher 10% weight loss temperature and higher light resistance than the structures with X representing a sulfur atom. Also, it was confirmed that when Y¹ and Y² are electron-donating groups such as alkylamino groups, the 10% weight loss temperature and light resistance tend to decrease.

### [Sixth Example: Test examples corresponding to sixth embodiment]

In the Sixth Example, synthesis was performed using precursors similar to those of the First to Fifth Examples.

### (Test Example 6-1)

The pigment of Test Example 1-11 of the First Example was used as an anthraquinone derivative of Test Example 6-1. The anthraquinone derivative of Test Example 6-1 is a compound expressed by the following Formula (P6-1).

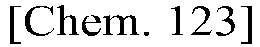

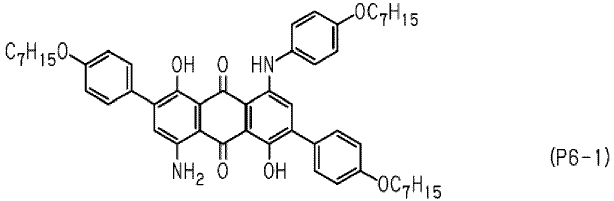

### (Test Example 6-2)

The pigment of Test Example 2-8 of the Second Example was used as an anthraquinone derivative of Test Example 6-2. The anthraquinone derivative of Test Example 6-2 is a compound expressed by the following Formula (P6-2).

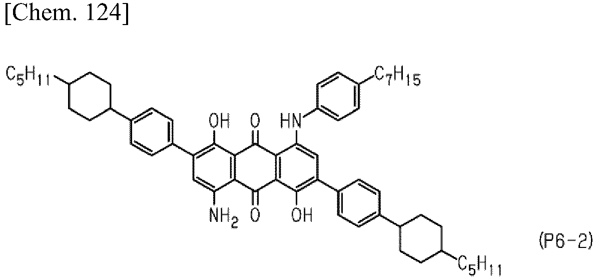

### (Test Example 6-3)

The pigment of Test Example 1-3 of the First Example was used as an anthraquinone derivative of Test Example 6-3. The anthraquinone derivative of Test Example 6-3 is a compound expressed by the following Formula (P6-3).

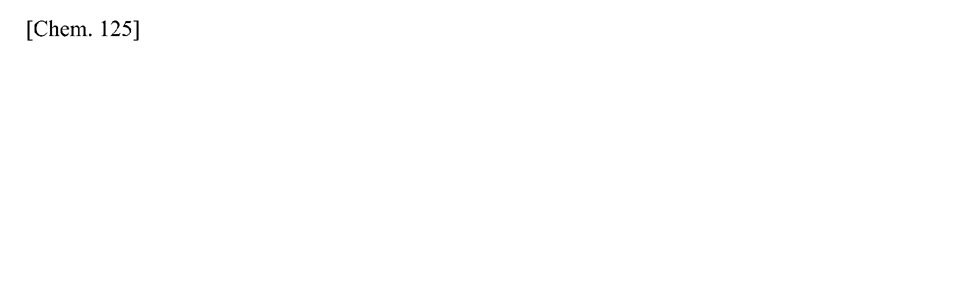

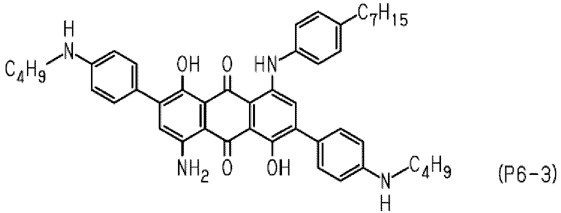

### (Test Example 6-4)

The pigment of Test Example 1-2 of First Example was used as an anthraquinone derivative of Test Example 6-4. The anthraquinone derivative of Test Example 6-4 is a compound expressed by the following Formula (P6-4).

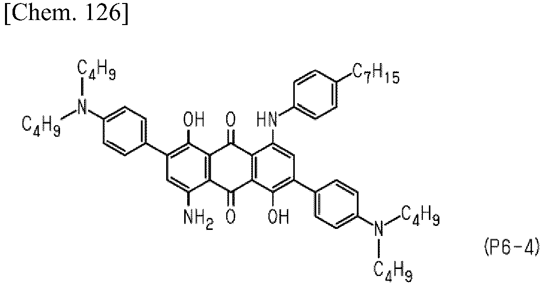

### (Test Example 6-5)

### <Pigment synthesis of Test Example 6-5>

In the pigment synthesis process of Test Example 1-1 of the First Example, 4-dimethylaminophenylboronic acid was replaced with 1.2 equivalents of 4-(trans-4-pentylcyclohexyl)phenylboronic acid, and the reaction solution was stirred at 80°C for 1 hour, to which 1.2 equivalents of 4-pentyloxybiphenylboronic acid were further added, followed by heating and stirring at 80°C for at least 2 hours. Subsequently, an anthraquinone derivative of Test Example 6-5 was obtained by performing extraction and purification as in the pigment synthesis process of Test Example 1-1 described above. The anthraquinone derivative of Test Example 6-5 is a compound expressed by the following Formula (P6-5).

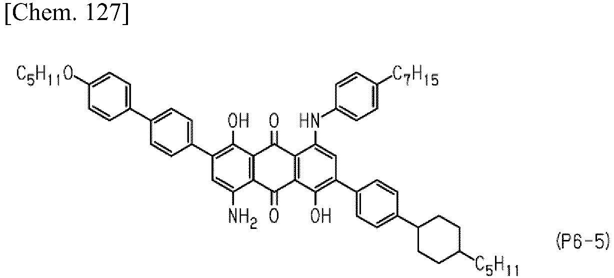

### (Test Example 6-6)

### <Pigment synthesis of Test Example 6-6>

In the pigment synthesis process of Test Example 1-1 of the First Example, 4-dimethylaminophenylboronic acid was replaced with 1.2 equivalents of 4-(trans-4-pentylcyclohexyl)phenylboronic acid, and the reaction solution was stirred at 80°C for 1 hour, to which 1.2 equivalents of 4-monobutylaminophenylboronic acid were further added, followed by heating and stirring at 80°C for at least 2 hours. Subsequently, an anthraquinone derivative of Test Example 6-6 was obtained by performing extraction and purification as in the pigment synthesis process of Test Example 1-1 described above. The anthraquinone derivative of Test Example 6-6 is a compound expressed by the following Formula (P6-6).

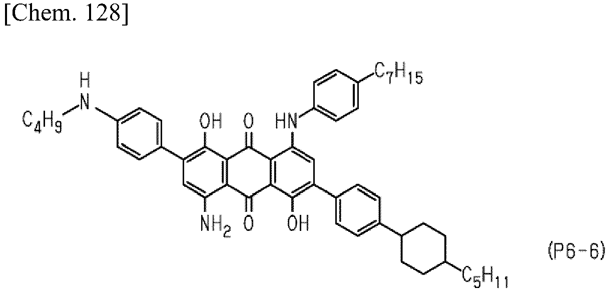

### (Test Example 6-7)

The pigment of Test Example 5-5 of the Fifth Example was used as an anthraquinone derivative of Test Example 6-7. The anthraquinone derivative of Test Example 6-7 is a compound expressed by the following Formula (P6-7).

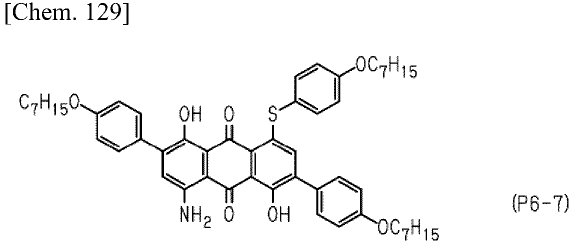

### (Test Example 6-8)

The pigment of Test Example 4-12 of the Fourth Example was used as an anthraquinone derivative of Test Example 6-8. The anthraquinone derivative of Test Example 6-8 is a compound expressed by the following Formula (P6-8).

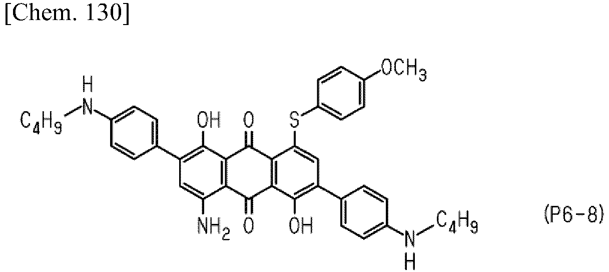

### (Test Example 6-9)

The pigment of Test Example 5-7 of the Fifth Example was used as an anthraquinone derivative of Test Example 6-9. The anthraquinone derivative of Test Example 6-9 is a compound expressed by the following Formula (P6-9).

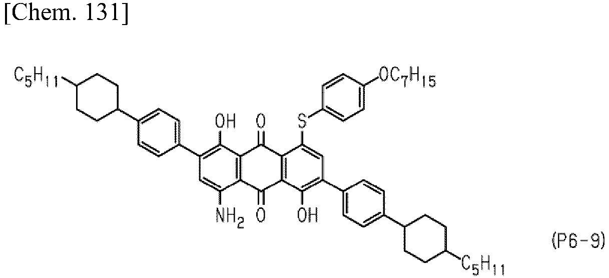

### (Test Example 6-10)

### <Synthesis of Precursor B14>

Precursor B14 was obtained by performing synthesis as in the synthesis process of Precursor A2 of the First Example, except that Precursor A1 was replaced with Precursor B4 and that 4-heptylaniline was replaced with 4-heptyloxyaniline. Precursor B14 is a compound expressed by the following Formula (6-a).

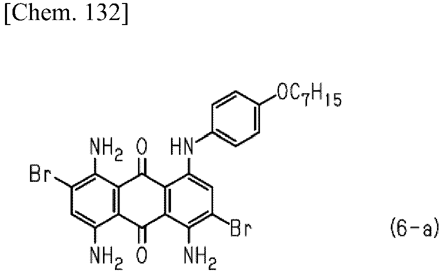

### <Pigment synthesis of Test Example 6-10>

An anthraquinone derivative of Test Example 6-10 was obtained by performing synthesis as in the pigment synthesis process of Test Example 1-1 of the First Example, except that Precursor A2 was replaced with Precursor B14 and that 4-dimethylaminophenylboronic acid was replaced with 4-heptyloxyphenylboronic acid. The anthraquinone derivative of Test Example 6-10 is a compound expressed by the following Formula (P6-10).

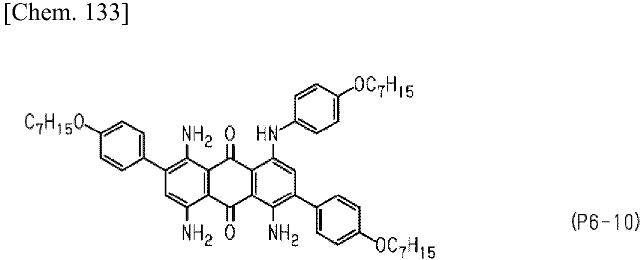

### (Test Example 6-11)

### <Pigment synthesis of Test Example 6-11>

An anthraquinone derivative of Test Example 6-11 was obtained by performing synthesis as in the pigment synthesis process of Test Example 4-13 of the Fourth Example, except that 4-heptyloxyphenol was replaced with 4-(trans-4-pentylcyclohexyl)phenol. The anthraquinone derivative of Test Example 6-11 is a compound expressed by the following Formula (P6-11).

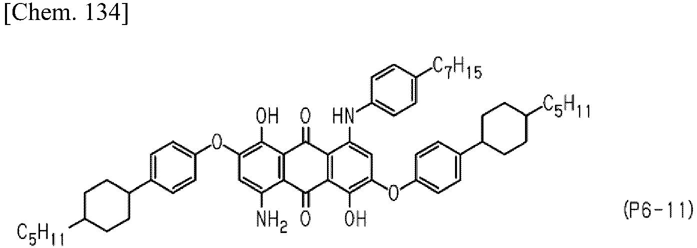

### (Test Example 6-12)

### <Synthesis of Precursor D4>

Precursor D4 was obtained by performing synthesis as in the synthesis process of Precursor B11 of the Fourth Example, except that Precursor B4 was replaced with Precursor A1. Precursor D4 is a compound expressed by the following Formula (6-b).

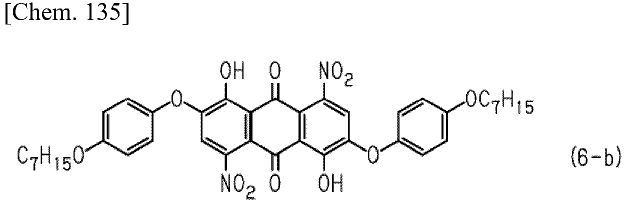

### <Pigment synthesis of Test Example 6-12>

An anthraquinone derivative of Test Example 6-12 was obtained by performing synthesis as in the pigment synthesis process of Test Example 4-10 of the Fourth Example, except that Precursor D1 was replaced with Precursor D4 and that 4-methoxybenzenethiol was replaced with 4-heptyloxybenzenethiol. The anthraquinone derivative of Test Example 6-12 is a compound expressed by the following Formula (P6-12).

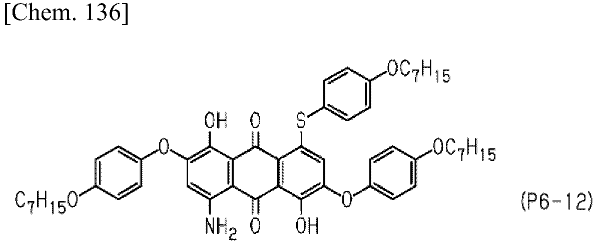

### (Test Example 6-13)

### <Pigment synthesis of Test Example 6-13>

An anthraquinone derivative of Test Example 6-13 was obtained by performing synthesis as in the pigment synthesis process of Test Example 4-13 of the Fourth Example, except that 4-heptyloxyphenol was replaced with 4-monobutylaminophenol. The anthraquinone derivative of Test Example 6-13 is a compound expressed by the following Formula (P6-13). The anthraquinone derivative of Test Example 6-13 is the same compound as the anthraquinone derivative of Test Example 1-13.

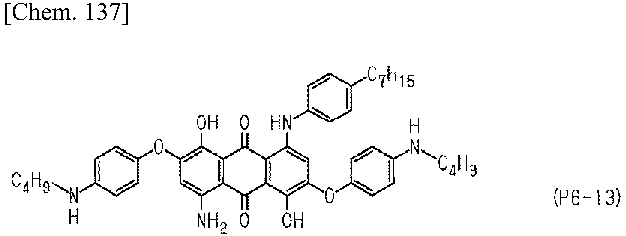

### (Evaluation method)

### <Evaluation of absorption wavelength and coloring power>

For the anthraquinone derivatives of Test Examples 6-1 to 6-13, evaluation test specimens were prepared as in the First Example.

For the evaluation test specimen of each test example, a UV-visible absorption spectrum was measured using an automated spectrophotometer (U-4100 manufactured by Hitachi, Ltd.). Then, for wavelengths in the measurement range of 360 nm or greater and 800 nm or less, a maximum absorption wavelength λmax and an absorbance at this wavelength were obtained.

When evaluating coloring power, test specimens having an absorbance of 0.40 or more were evaluated to be Good, and those having an absorbance of less than 0.40 were evaluated to be Poor.

### <Calculation of transition dipole moment>

For the anthraquinone derivatives of Test Examples 6-1 to 6-13, a transition dipole moment was calculated using the time-dependent density functional theory. Specifically, using the quantum chemical calculation program GAMESS, a transition dipole moment of each anthraquinone derivative in a vacuum was calculated using B3LYP as the functional and 6-31G(d) as the basis function. The transition dipole moment is a vector constituted of x, y and z components. A one-half power of the square sum of the components was defined to be the magnitude of the transition dipole moment, µ (µ=(x²+y²+z²)^{1/2}).

### (Evaluations)

Table 6 shows the structure, components of transition dipole moment, magnitude µ of transition dipole moment, maximum absorption wavelength λmax, absorbance, and evaluation of coloring power of each anthraquinone derivative, for Test Examples 6-1 to 6-13. The structure of the anthraquinone derivative of each test example is expressed by the following Formula (II), and R, A, X, Y¹, Y² and Z of Table 6 correspond to R, A, X, Y¹, Y² and Z of Formula (II).

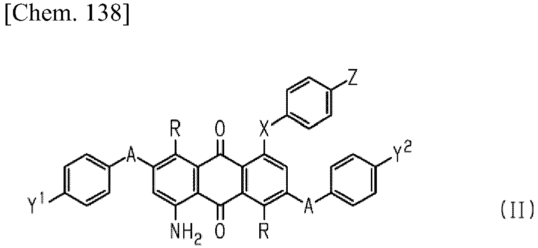

**[Table 6]**

| Test Ex. | Structure | | | | | | Transition dipole moment (D) | | | | λmax (nm) | Coloring power | |
|---|---|---|---|---|---|---|---|---|---|---|---|---|---|
| | R | A | X | Y¹ | Y² | Z | x | y | z | µ | | Absorbance | Evaluation |
| 6-1 | OH | Direct bond | NH | OC₇H₁₅ | OC₇H₁₅ | OC₇H₁₅ | 3.16 | -1.37 | -0.08 | 3.45 | 681 | 0.42 | Good |
| 6-2 | OH | Direct bond | NH | CyC₅H₁₁ | CyC₅H₁₁ | C₇H₁₅ | 3.21 | 1.42 | 0.08 | 3.51 | 683 | 0.45 | Good |
| 6-3 | OH | Direct bond | NH | NHC₄H₉ | NHC₄H₉ | C₇H₁₅ | 3.86 | -0.21 | 0.01 | 3.87 | 691 | 0.72 | Good |
| 6-4 | OH | Direct bond | NH | N(C₄H₉)₂ | N(C₄H₉)₂ | C₇H₁₅ | 4.21 | 0.03 | -0.08 | 4.21 | 701 | 0.68 | Good |
| 6-5 | OH | Direct bond | NH | PhOC₅H₁₁ | CyC₅H₁₁ | C₇H₁₅ | -3.43 | 1.42 | 0.08 | 3.71 | 685 | 0.57 | Good |
| 6-6 | OH | Direct bond | NH | NHC₄H₉ | CyC₅H₁₁ | C₇H₁₅ | -3.50 | -1.34 | -0.07 | 3.75 | 688 | 0.59 | Good |
| 6-7 | OH | Direct bond | S | OC₇H₁₅ | OC₇H₁₅ | OC₇H₁₅ | 3.45 | 0.68 | 0.20 | 3.52 | 638 | 0.43 | Good |
| 6-8 | OH | Direct bond | S | NHC₄H₉ | NHC₄H₉ | OCH₃ | 3.77 | 0.47 | 0.05 | 3.80 | 650 | 0.71 | Good |
| 6-9 | OH | Direct bond | S | CyC₅H₁₁ | CyC₅H₁₁ | OC₇H₁₅ | 3.31 | 0.86 | 0.22 | 3.43 | 592 | 0.43 | Good |
| 6-10 | NH₂ | Direct bond | NH | OC₇H₁₅ | OC₇H₁₅ | OC₇H₁₅ | -3.21 | 1.13 | 0.09 | 3.40 | 706 | 0.45 | Good |
| 6-11 | OH | O | NH | CyC₅H₁₁ | CyC₅H₁₁ | C₇H₁₅ | 3.19 | 0.02 | -0.16 | 3.19 | 632 | 0.36 | Poor |
| 6-12 | OH | O | S | OC₇H₁₅ | OC₇H₁₅ | OC₇H₁₅ | 3.06 | 0.28 | 0.18 | 3.07 | 570 | 0.30 | Poor |
| 6-13 | OH | O | NH | NHC₄H₉ | NHC₄H₉ | C₇H₁₅ | 3.23 | 0.35 | -0.20 | 3.25 | 608 | 0.35 | Poor |

As shown in Table 6, in Test Examples 6-1 to 6-10, where the magnitude µ of the transition dipole moment is 3.30 D or more, higher absorbance is achieved and good coloring power is exhibited, compared to Test Examples 6-11 to 6-13, where the magnitude µ of the transition dipole moment is less than 3.30 D. Furthermore, the anthraquinone derivatives of Test Examples 6-1 to 6-10 also tend to have a high maximum absorption wavelength which is in the wavelength range of 580 nm or greater, and therefore can be used as cyanide pigments.

As can be seen from the results shown in Table 6, direct bonds of the substituents at the β-positions increase the transition dipole moment. Furthermore, in Test Examples 6-1 to 6-10, it was confirmed that when Y¹ and Y² are electron-donating groups such as alkylamino groups, the transition dipole moment tends to increase and a high absorbance tends to be obtained.

### [Seventh Example: Test examples corresponding to seventh embodiment]

In the Seventh Example, synthesis was performed using precursors similar to those of the First to Sixth Examples.

### (Test Example 7-1)

The pigment of Test Example 1-11 of the First Example was used as an anthraquinone derivative of Test Example 7-1. The anthraquinone derivative of Test Example 7-1 is a compound expressed by the following Formula (P7-1).

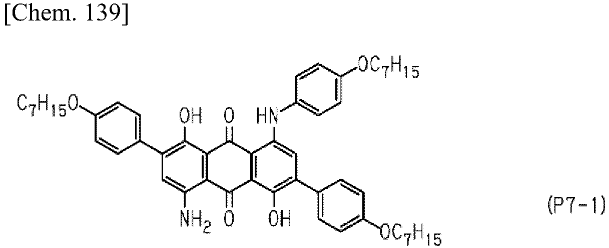

### (Test Example 7-2)

The pigment of Test Example 2-8 of the Second Example was used as an anthraquinone derivative of Test Example 7-2. The anthraquinone derivative of Test Example 7-2 is a compound expressed by the following Formula (P7-2).

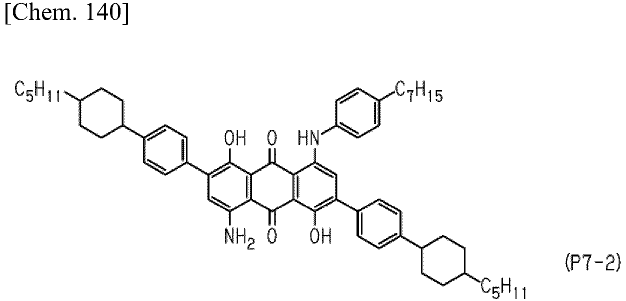

### (Test Example 7-3)

The pigment of Test Example 1-3 of the First Example was used as an anthraquinone derivative of Test Example 7-3. The anthraquinone derivative of Test Example 7-3 is a compound expressed by the following Formula (P7-3).

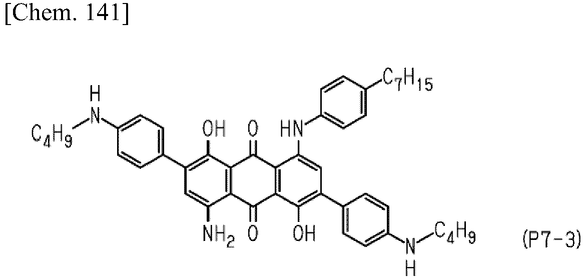

### (Test Example 7-4)

The pigment of Test Example 1-2 of the First Example was used as an anthraquinone derivative of Test Example 7-4. The anthraquinone derivative of Test Example 7-4 is a compound expressed by the following Formula (P7-4).

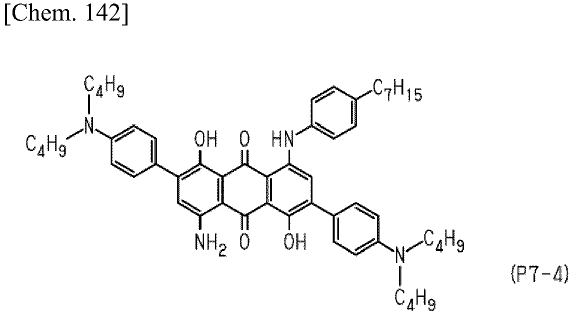

### (Test Example 7-5)

### <Synthesis of Precursor A4>

Precursor A4 was obtained by performing synthesis as in the synthesis process of Precursor A2 of the First Example, except that 4-heptylaniline was replaced with 4-cyanoaniline. Precursor A4 is a compound expressed by the following Formula (7-a).

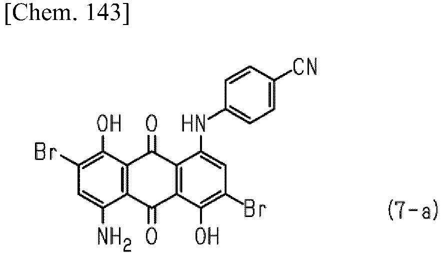

### <Pigment synthesis of Test Example 7-5>

An anthraquinone derivative of Test Example 7-5 was obtained by performing synthesis as in the pigment synthesis process of Test Example 1-1 of the First Example, except that Precursor A2 was replaced with Precursor A4 and that 4-dimethylaminophenylboronic acid was replaced with 4-heptyloxyphenylboronic acid. The anthraquinone derivative of Test Example 7-5 is a compound expressed by the following Formula (P7-5).

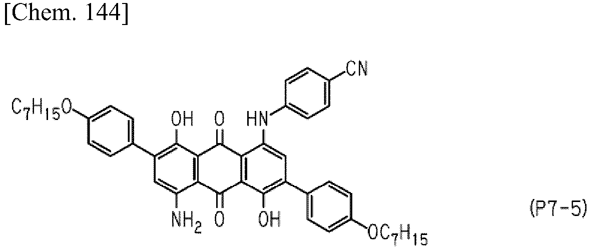

### (Test Example 7-6)

### <Pigment synthesis of Test Example 7-6>

An anthraquinone derivative of Test Example 7-6 was obtained by performing synthesis as in the pigment synthesis process of Test Example 1-1 of the First Example, except that Precursor A2 was replaced with Precursor A4 and that 4-dimethylaminophenylboronic acid was replaced with 4-pentylcyclohexylphenylboronic acid. The anthraquinone derivative of Test Example 7-6 is a compound expressed by the following Formula (P7-6). The anthraquinone derivative of Test Example 7-6 is the same compound as the anthraquinone derivative of Test Example 2-3.

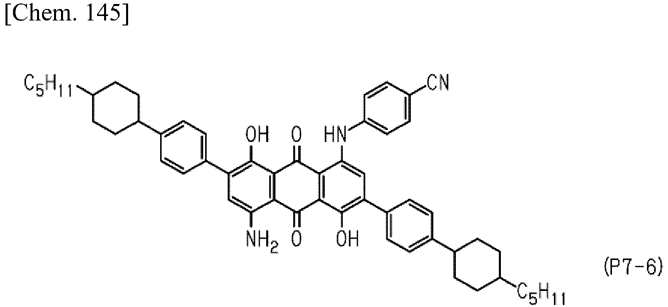

### (Test Example 7-7)

The pigment of Test Example 5-5 of the Fifth Example was used as an anthraquinone derivative of Test Example 7-7. The anthraquinone derivative of Test Example 7-7 is a compound expressed by the following Formula (P7-7).

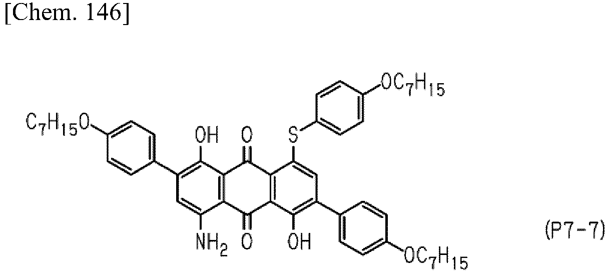

### (Test Example 7-8)

The pigment of Test Example 5-7 of the Fifth Example was used as an anthraquinone derivative of Test Example 7-8. The anthraquinone derivative of Test Example 7-8 is a compound expressed by the following Formula (P7-8).

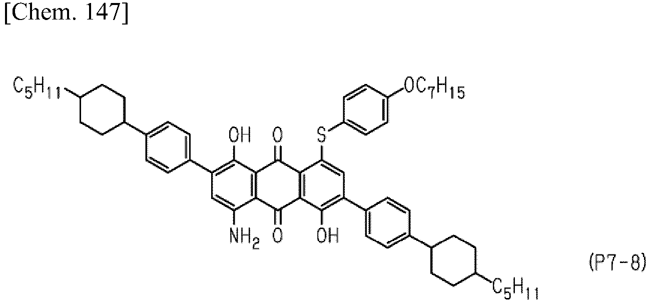

### (Test Example 7-9)

### <Pigment synthesis of Test Example 7-9>

An anthraquinone derivative of Test Example 7-9 was obtained by performing synthesis as in the pigment synthesis process of Test Example 4-10 of the Fourth Example, except that Precursor D1 was replaced with Precursor D3 and that 4-methoxybenzenethiol was replaced with 4-heptyloxybenzenethiol. The anthraquinone derivative of Test Example 7-9 is a compound expressed by the following Formula (P7-9).

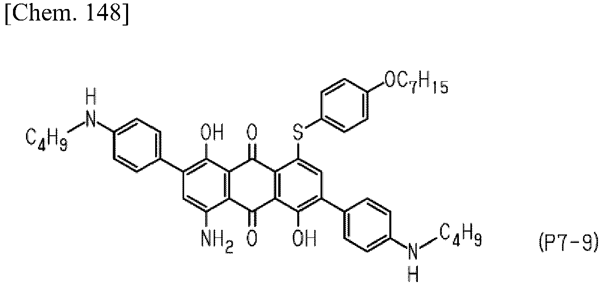

### (Test Example 7-10)

### <Pigment synthesis of Test Example 7-10>

An anthraquinone derivative of Test Example 7-10 was obtained by performing synthesis as in the pigment synthesis process of Test Example 4-13 of the Fourth Example, except that Precursor A2 was replaced with Precursor A3. The anthraquinone derivative of Test Example 7-10 is a compound expressed by the following Formula (P7-10). The anthraquinone derivative of Test Example 7-10 is the same compound as the anthraquinone derivative of Test Example 1-12.

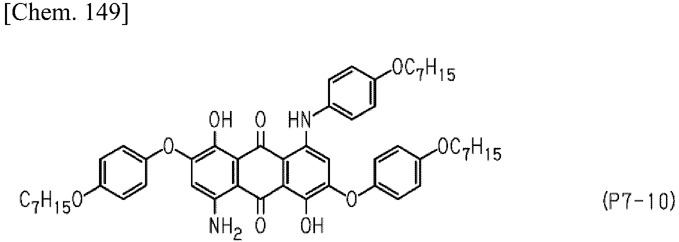

### (Test Example 7-11)

The pigment of Test Example 6-11 of the Sixth Example was used as an anthraquinone derivative of Test Example 7-11. The anthraquinone derivative of Test Example 7-11 is a compound expressed by the following Formula (P7-11).

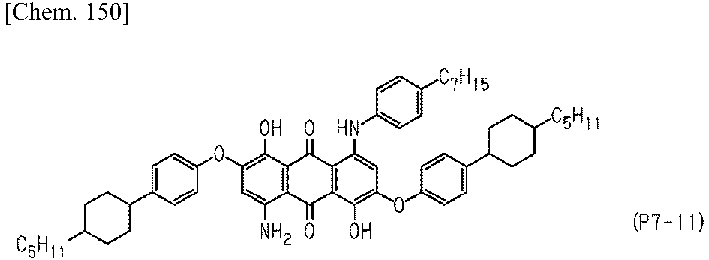

### (Test Example 7-12)

The pigment of Test Example 6-12 of the Sixth Example was used as an anthraquinone derivative of Test Example 7-12. The anthraquinone derivative of Test Example 7-12 is a compound expressed by the following Formula (P7-12).

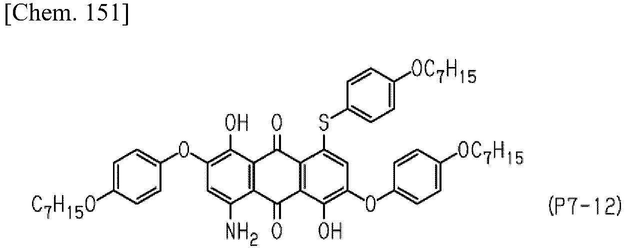

### (Test Example 7-13)

### <Synthesis of Precursor D5>

Precursor D5 was obtained by performing synthesis as in the synthesis process of Precursor B11 of the Fourth Example, except that Precursor B4 was replaced with Precursor A1 and that 4-heptyloxyphenol was replaced with 4-pentylcyclohexylphenol. Precursor D5 is a compound expressed by the following Formula (7-b).

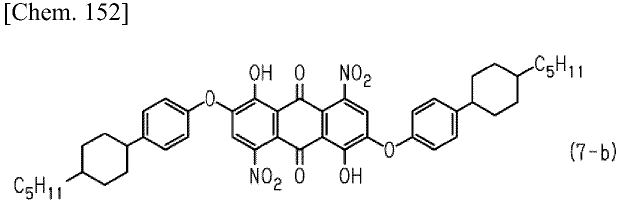

### <Pigment synthesis of Test Example 7-13>

An anthraquinone derivative of Test Example 7-13 was obtained by performing synthesis as in the pigment synthesis process of Test Example 4-10 of the Fourth Example, except that Precursor D1 was replaced with Precursor D5 and that 4-methoxybenzenethiol was replaced with 4-heptyloxybenzenethiol. The anthraquinone derivative of Test Example 7-13 is a compound expressed by the following Formula (P7-13).

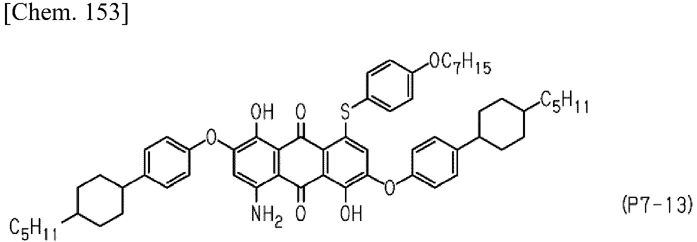

### (Evaluation method)

### <Evaluation of absorption wavelength and coloring power>

For the anthraquinone derivatives of Test Examples 7-1 to 7-13, evaluation test specimens were prepared as in the First Example. Then, an absorption maximum wavelength λmax and an absorbance at this wavelength were obtained as in the Sixth Example, and coloring power was evaluated.

### <Calculation of molecular orbital coefficient>

For the anthraquinone derivatives of Test Examples 7-1 to 7-13, molecular orbital coefficients were calculated using the density functional theory. Specifically, using the quantum chemical calculation program GAMESS, the anthraquinone derivatives were subjected to structural optimization to obtain molecular orbital coefficients in the optimized structures with B3LYP used as the functional and 6-31G(d) used for the basis functions. For carbon atoms C¹¹, C¹², C²¹ and C²² in the above Formula (7-1), square roots M₁₁, M₁₂, M₂₁ and M₂₂ of the square sums of the coefficients corresponding to the orbitals on the carbon atoms were calculated, and an average Mv of M₁₁, M₁₂, M₂₁ and M₂₂ was obtained. Under the present calculation conditions, 15 orbitals are assigned to each carbon atom, and molecular orbital coefficients are calculated for the respective orbitals.

### (Evaluations)

Table 7 shows the structure, square root of square sum of molecular orbital coefficients, average Mv, maximum absorption wavelength λmax, absorbance, and evaluation of coloring power of each anthraquinone derivative, for Test Examples 7-1 to 7-13. The structure of the anthraquinone derivative of each test example is expressed by the following Formula (II), and R, A, X, Y¹, Y² and Z of Table 7 correspond to R, A, X, Y¹, Y² and Z of Formula (II).

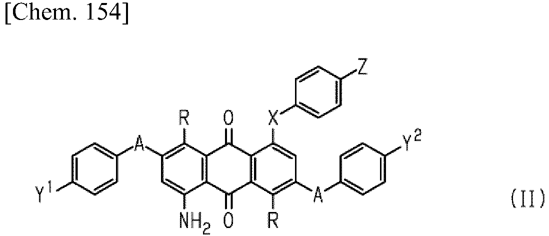

**[Table 7]**

| Test Ex. | Structure | | | | | | Square root of square sum of molecular orbital coefficients | | | | | λmax (nm) | Coloring power | |
|---|---|---|---|---|---|---|---|---|---|---|---|---|---|---|
| | R | A | X | Y¹ | Y² | Z | M₁₁ | M₁₂ | M₂₁ | M₂₂ | Mv | | Absorbance | Evaluation |
| 7-1 | OH | Direct bond | NH | OC₇H₁₅ | OC₇H₁₅ | OC₇H₁₅ | 0.047 | 0.048 | 0.043 | 0.041 | 0.045 | 681 | 0.42 | Good |
| 7-2 | OH | Direct bond | NH | CyC₅H₁₁ | CyC₅H₁₁ | C₇H₁₅ | 0.037 | 0.044 | 0.034 | 0.039 | 0.039 | 683 | 0.45 | Good |
| 7-3 | OH | Direct bond | NH | NHC₄H₉ | NHC₄H₉ | C₇H₁₅ | 0.085 | 0.070 | 0.082 | 0.066 | 0.076 | 691 | 0.72 | Good |
| 7-4 | OH | Direct bond | NH | N(C₄H₉)₂ | N(C₄H₉)₂ | C₇H₁₅ | 0.111 | 0.076 | 0.110 | 0.074 | 0.093 | 701 | 0.68 | Good |
| 7-5 | OH | Direct bond | NH | OC₇H₁₅ | OC₇H₁₅ | CN | 0.064 | 0.060 | 0.069 | 0.063 | 0.064 | 675 | 0.59 | Good |
| 7-6 | OH | Direct bond | NH | CyC₅H₁₁ | CyC₅H₁₁ | CN | 0.044 | 0.050 | 0.045 | 0.051 | 0.047 | 676 | 0.58 | Good |
| 7-7 | OH | Direct bond | S | OC₇H₁₅ | OC₇H₁₅ | OC₇H₁₅ | 0.077 | 0.072 | 0.088 | 0.081 | 0.079 | 638 | 0.43 | Good |
| 7-8 | OH | Direct bond | S | CyC₅H₁₁ | CyC₅H₁₁ | OC₇H₁₅ | 0.052 | 0.059 | 0.055 | 0.062 | 0.057 | 592 | 0.42 | Good |
| 7-9 | OH | Direct bond | S | NHC₄H₉ | NHC₄H₉ | OC₇H₁₅ | 0.125 | 0.095 | 0.169 | 0.128 | 0.129 | 650 | 0.71 | Good |
| 7-10 | OH | O | NH | OC₇H₁₅ | OC₇H₁₅ | OC₇H₁₅ | 0.011 | 0.005 | 0.014 | 0.005 | 0.009 | 632 | 0.23 | Poor |
| 7-11 | OH | O | NH | CyC₅H₁₁ | CyC₅H₁₁ | C₇H₁₅ | 0.011 | 0.004 | 0.014 | 0.005 | 0.009 | 632 | 0.36 | Poor |
| 7-12 | OH | O | S | OC₇H₁₅ | OC₇H₁₅ | OC₇H₁₅ | 0.014 | 0.008 | 0.012 | 0.009 | 0.011 | 570 | 0.30 | Poor |
| 7-13 | OH | O | S | CyC₅H₁₁ | CyC₅H₁₁ | OC₇H₁₅ | 0.014 | 0.007 | 0.029 | 0.029 | 0.020 | 576 | 0.26 | Poor |

As shown in Table 7, in Test Examples 7-1 to 7-9, where the average Mv is 0.03 or more, higher absorbance is achieved and good coloring power is exhibited, compared to Test Examples 7-10 to 7-13, where the average Mv is less than 0.03. The anthraquinone derivatives of Test Examples 7-1 to 7-9 have a maximum absorption wavelength in the wavelength range of 580 nm or greater, and therefore can be used as cyanide pigments.

As can be seen from the results shown in Table 7, direct bonds of the substituents at the β-positions increase the average Mv. Furthermore, comparing between Test Examples 7-1 and 7-5 and between Test Examples 7-2 and 7-6, it was confirmed that when Z is an electron-withdrawing group such as a cyano group, the average Mv tends to increase and a high absorbance tends to be achieved. Furthermore, as can be seen from Test Examples 7-3, 7-4 and 7-9, it was confirmed that when Y¹ and Y² are electron-donating groups such as alkylamino groups, the average Mv tends to increase and a high absorbance tends to be achieved.

### [Eighth Example: Test examples corresponding to eighth embodiment]

In the Eighth Example, synthesis was performed using precursors similar to those of the First to Seventh Examples.

### (Test Example 8-1)

The pigment of Test Example 4-1 of the Fourth Example was used as an anthraquinone derivative of Test Example 8-1. The anthraquinone derivative of Test Example 8-1 is a compound expressed by the following Formula (P8-1).

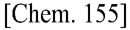

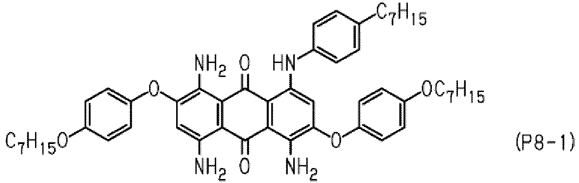

### (Test Example 8-2)

### <Synthesis of Precursor B15>

Precursor B15 was obtained by performing synthesis as in the synthesis process of Precursor B11 of the Fourth Example, except that 4-heptyloxyphenol was replaced with 4-heptylphenol. Precursor B15 is a compound expressed by the following Formula (8-a).

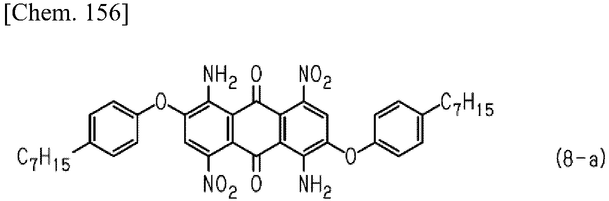

### <Synthesis of Precursor B16>

Precursor B16 was obtained by performing synthesis as in the synthesis process of Precursor B12 of the Fourth Example, except that Precursor B11 was replaced with Precursor B15. Precursor B16 is a compound expressed by the following Formula (8-b).

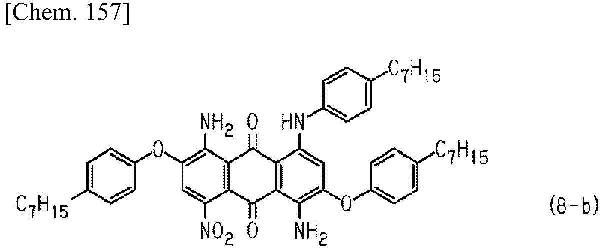

### <Pigment synthesis of Test Example 8-2>

An anthraquinone derivative of Test Example 8-2 was obtained by performing synthesis as in the pigment synthesis process of Test Example 1-14 of the First Example, except that Precursor B6 was replaced with Precursor B16. The anthraquinone derivative of Test Example 8-2 is a compound expressed by the following Formula (P8-2).

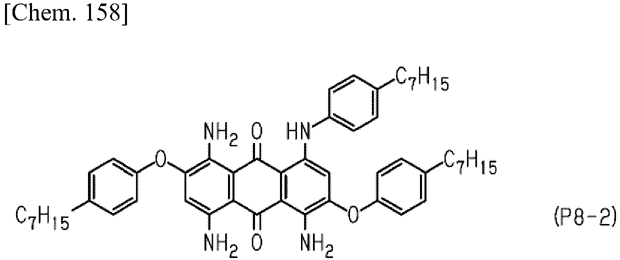

### (Test Example 8-3)

### <Synthesis of Precursor B17>

Precursor B17 was obtained by performing synthesis as in the synthesis process of Precursor B12 of the Fourth Example, except that 4-heptylaniline was replaced with N,N-dimethyl-1,4-phenylenediamine. Precursor B17 is a compound expressed by the following Formula (8-c).

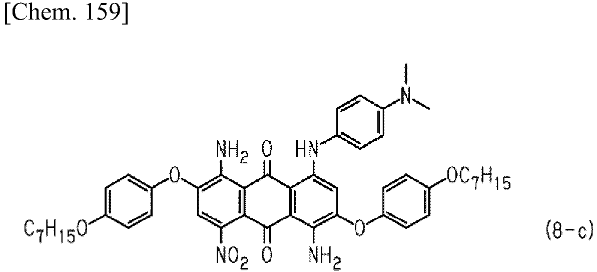

### <Pigment synthesis of Test Example 8-3>

An anthraquinone derivative of Test Example 8-3 was obtained by performing synthesis as in the pigment synthesis process of Test Example 1-14 of the First Example, except that Precursor B6 was replaced with Precursor B17. The anthraquinone derivative of Test Example 8-3 is a compound expressed by the following Formula (P8-3).

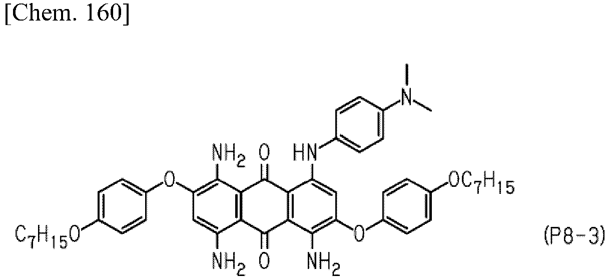

### (Test Example 8-4)

### <Synthesis of Precursor B18>

Precursor B18 was obtained by performing synthesis as in the synthesis process of Precursor B12 of the Fourth Example, except that 4-heptylaniline was replaced with 4-aminobenzonitrile. Precursor B18 is a compound expressed by the following Formula (8-d).

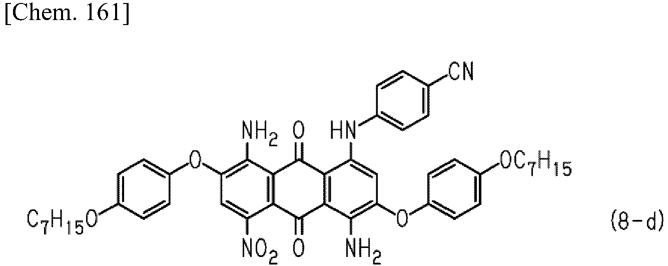

### <Pigment synthesis of Test Example 8-4>

An anthraquinone derivative of Test Example 8-4 was obtained by performing synthesis as in the pigment synthesis process of Test Example 1-14 of the First Example, except that Precursor B6 was replaced with Precursor B18. The anthraquinone derivative of Test Example 8-4 is a compound expressed by the following Formula (P8-4).

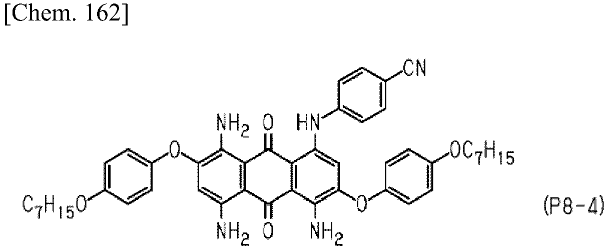

### (Test Example 8-5)

The pigment of Test Example 1-15 of the First Example was used as an anthraquinone derivative of Test Example 8-5. The anthraquinone derivative of Test Example 8-5 is a compound expressed by the following Formula (P8-5).

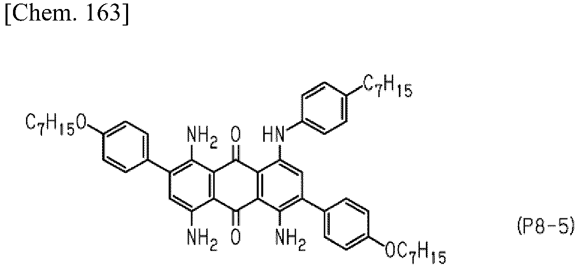

### (Test Example 8-6)

The pigment of Test Example 3-12 of the Third Example was used as an anthraquinone derivative of Test Example 8-6. The anthraquinone derivative of Test Example 8-6 is a compound expressed by the following Formula (P8-6).

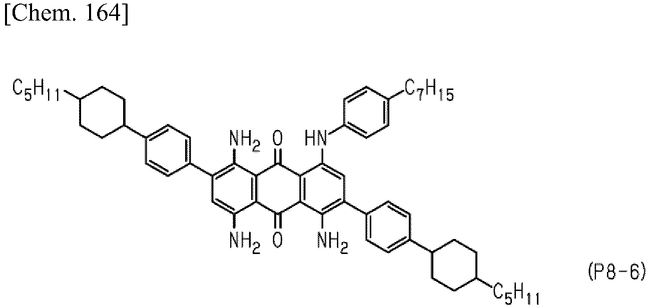

### (Test Example 8-7)

The pigment of Test Example 1-14 of the First Example was used as an anthraquinone derivative of Test Example 8-7. The anthraquinone derivative of Test Example 8-7 is a compound expressed by the following Formula (P8-7).

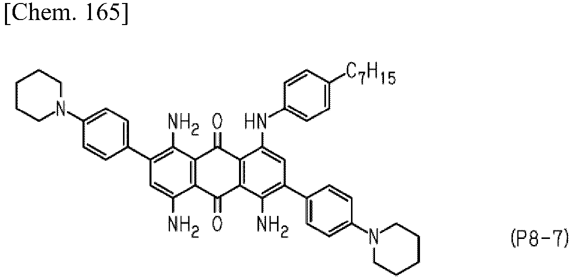

### (Test Example 8-8)

### <Synthesis of Precursor B19>

Precursor B4 (0.20 parts by weight), 4-heptyloxyaniline (1.2 parts by weight), and copper powder (0.053 parts by weight) were placed in a two-necked flask and purged with nitrogen. To these materials, N-methyl-2-pyrrolidone (3.0 parts by weight) was added, followed by stirring at 180°C for 10 hours. The reaction solution was subjected to extraction with pure water/dichloromethane, and the organic phase obtained was dried with sodium sulfate and filtered, and then concentrated using an evaporator. The resultant material was purified using silica gel column chromatography (eluent: hexane/ethyl acetate = 8/1) to obtain Precursor B19. Precursor B19 is a compound expressed by the following Formula (8-e).

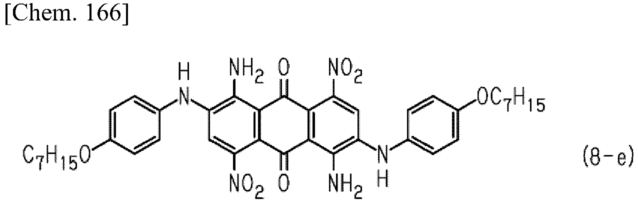

### <Synthesis of Precursor B20>

Precursor B20 was obtained by performing synthesis as in the synthesis process of Precursor B12 of the Fourth Example, except that Precursor B11 was replaced with Precursor B19. Precursor B20 is a compound expressed by the following Formula (8-f).

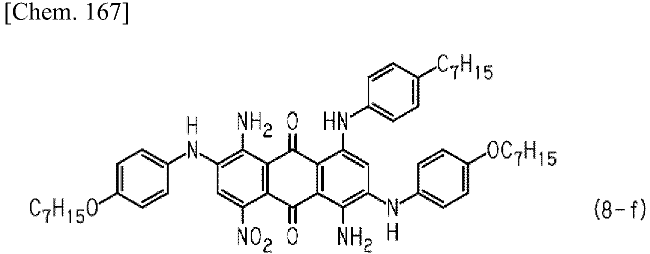

### <Pigment synthesis of Test Example 8-8>

An anthraquinone derivative of Test Example 8-8 was obtained by performing synthesis as in the pigment synthesis process of Test Example 1-14 of the First Example, except that Precursor B6 was replaced with Precursor B20. The anthraquinone derivative of Test Example 8-8 is a compound expressed by the following Formula (P8-8).

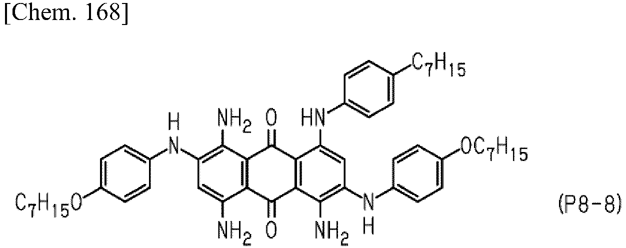

### (Test Example 8-9)

The pigment of Test Example 4-13 of the Fourth Example was used as an anthraquinone derivative of Test Example 8-9. The anthraquinone derivative of Test Example 8-9 is a compound expressed by the following Formula (P8-9).

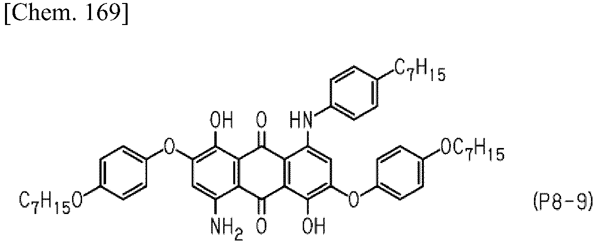

### (Test Example 8-10)

The pigment of Test Example 2-8 of the Second Example was used as an anthraquinone derivative of Test Example 8-10. The anthraquinone derivative of Test Example 8-10 is a compound expressed by the following Formula (P8-10).

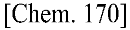

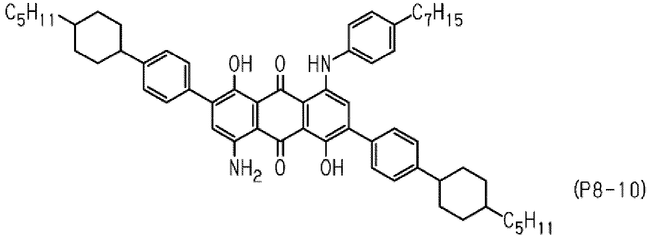

### (Test Example 8-11)

### <Pigment synthesis of Test Example 8-11>

An anthraquinone derivative of Test Example 8-11 was obtained by performing synthesis as in the pigment synthesis process of Test Example 1-1 of the First Example, except that 4-dimethylaminophenylboronic acid was replaced with (4-piperidyl-1-yl)phenylboronic acid. The anthraquinone derivative of Test Example 8-11 is a compound expressed by the following Formula (P8-11).

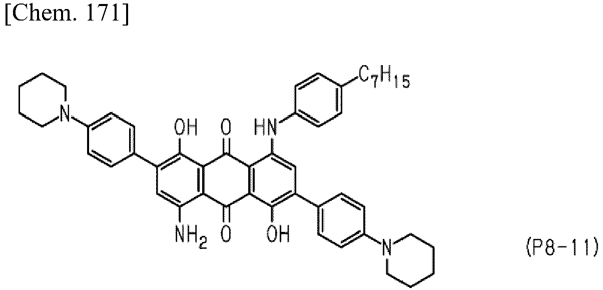

### (Test Example 8-12)

### <Pigment synthesis of Test Example 8-12>

An anthraquinone derivative of Test Example 8-12 was obtained by performing synthesis as in the synthesis process of Precursor B19 described above, except that Precursor B4 was replaced with Precursor A2. The anthraquinone derivative of Test Example 8-12 is a compound expressed by the following Formula (P8-12).

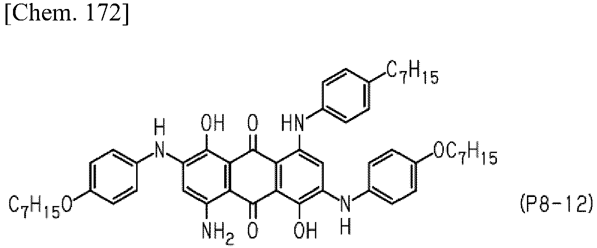

### (Evaluation method)

For the anthraquinone derivatives of Test Examples 8-1 to 8-12, evaluation test specimens were prepared as in the First Example, and respective maximum absorption wavelengths λmax were obtained. Furthermore, the evaluation test specimens were subjected to light resistance testing as in the First Example to calculate respective absorbance change ratios ΔAbs before and after the light resistance testing. When evaluating light resistance, test specimens having ΔAbs of 2% or less were evaluated to be Good, and those having ΔAbs exceeding 2% were evaluated to be Poor.

### (Evaluations)

Table 8 shows the structure, maximum absorption wavelength λmax, absorbance change ratio ΔAbs, and evaluation of light resistance of each anthraquinone derivative, for Test Examples 8-1 to 8-12. The structure of the anthraquinone derivative of each test example is expressed by the following Formula (I), and R, A, Y¹, Y² and Z of Table 8 correspond to R, A, Y¹, Y² and Z of Formula (I).

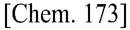

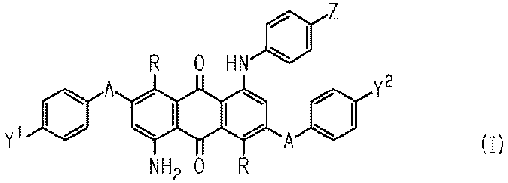

**[Table 8]**

| Test Ex. | Structure | | | | | λmax (nm) | Light resistance | |
|---|---|---|---|---|---|---|---|---|
| | R | A | Y¹ | Y² | Z | | ΔAbs (%) | Evaluation |
| 8-1 | NH₂ | O | OC₇H₁₅ | OC₇H₁₅ | C₇H₁₅ | 656 | 1.0 | Good |
| 8-2 | NH₂ | O | C₇H₁₅ | C₇H₁₅ | C₇H₁₅ | 659 | 1.8 | Good |
| 8-3 | NH₂ | O | OC₇H₁₅ | OC₇H₁₅ | N(CH₃)₂ | 671 | 1.5 | Good |
| 8-4 | NH₂ | O | OC₇H₁₅ | OC₇H₁₅ | CN | 636 | 1.4 | Good |
| 8-5 | NH₂ | Direct bond | OC₇H₁₅ | OC₇H₁₅ | C₇H₁₅ | 699 | 0.8 | Good |
| 8-6 | NH₂ | Direct bond | CyC₅H₁₁ | CyC₅H₁₁ | C₇H₁₅ | 707 | 1.0 | Good |
| 8-7 | NH₂ | Direct bond | Piperidyl group | Piperidyl group | C₇H₁₅ | 709 | 1.8 | Good |
| 8-8 | NH₂ | NH | OC₇H₁₅ | OC₇H₁₅ | C₇H₁₅ | 629 | 0.8 | Good |
| 8-9 | OH | O | OC₇H₁₅ | OC₇H₁₅ | C₇H₁₅ | 632 | 29.6 | Poor |
| 8-10 | OH | Direct bond | CyC₅H₁₁ | CyC₅H₁₁ | C₇H₁₅ | 683 | 3.2 | Poor |
| 8-11 | OH | Direct bond | Piperidyl group | Piperidyl group | C₇H₁₅ | 692 | 7.0 | Poor |
| 8-12 | OH | NH | OC₇H₁₅ | OC₇H₁₅ | C₇H₁₅ | 605 | 25.0 | Poor |

As shown in Table 8, the anthraquinone derivatives of Test Examples 8-1 to 8-8 have a maximum absorption wavelength in the wavelength range of 600 nm or greater. Therefore, the anthraquinone derivatives of Test Examples 8-1 to 8-8 exhibit a blue color and can be used as cyanide pigments. High light resistance is exhibited in Test Examples 8-1 to 8-8.

The anthraquinone derivatives of Test Examples 8-9 to 8-12 have a hydroxyl group at an α-position. In Test Examples 8-9 and 8-12, where ether bonds or secondary amines are present at the β-positions, the absorbance change ratio ΔAbs is 25% or more and light resistance is extremely low. In Test Examples 8-10 and 8-11, where substituted phenyl groups are directly bonded at the β-positions, light resistance is significantly improved compared to Test Examples 8-9 and 8-12.

In Test Examples 8-1 to 8-8, where no hydroxyl group is present at an α-position, even higher light resistance is achieved than in Test Examples 8-10 and 8-11, regardless of whether direct bonds are present at the β-positions. Therefore, as can be seen, the high light resistance of the anthraquinone derivatives of Test Examples 8-1 to 8-8 is achieved as a result of the structure in which the α-position substituents are an amino group and an anilino group.

### [Ninth Example: Test examples corresponding to ninth embodiment]

In the Ninth Example, synthesis was performed using precursors similar to those of the First to Eighth Examples.

### <Synthesis of Precursor D6>

Precursor D6 was obtained by performing synthesis as in the synthesis process of Precursor D1 of the Fourth Example, except that 4-pentylcyclohexylphenylboronic acid was replaced with 4-heptyloxyphenylboronic acid and that Precursor A1 was replaced with Precursor B4. Precursor D6 is a compound expressed by the following Formula (9-a). Precursor D6 is the same compound as that of Precursor B7.

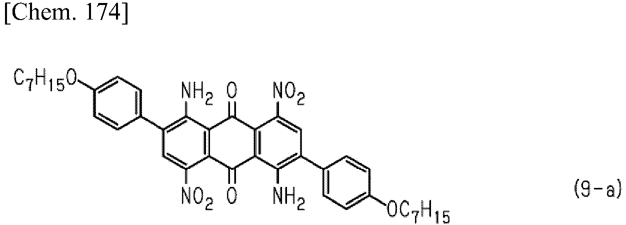

### <Synthesis of Precursor D7>

Precursor D7 was obtained by performing synthesis as in the synthesis process of Precursor D1 of the Fourth Example, except that 4-pentylcyclohexylphenylboronic acid was replaced with 4-(dimethylamino)phenylboronic acid. Precursor D7 is a compound expressed by the following Formula (9-b).

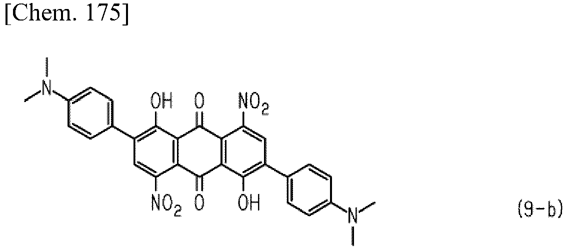

### <Synthesis of Precursor D8>

In the synthesis process of Precursor D1 of the Fourth Example, in place of adding 1.2 parts by weight of 4-pentylcyclohexylphenylboronic acid, 0.7 parts by weight of 4-pentylcyclohexylphenylboronic acid were added, and the reaction solution was stirred for 1 hour, to which 0.5 parts by weight of 4-monobutylaminophenylboronic acid were further added, followed by heating and stirring at 80°C for at least 2 hours. After that, Precursor D8 was obtained by performing extraction and purification as in the synthesis process of Precursor D1. Precursor D8 is a compound expressed by the following Formula (9-c).

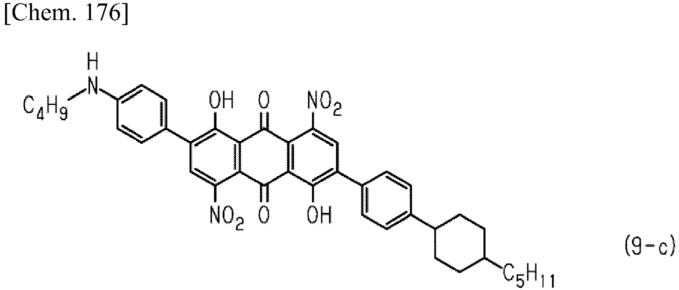

### (Test Example 9-1)

### <Pigment synthesis of Test Example 9-1>

An anthraquinone derivative of Test Example 9-1 was obtained by performing synthesis as in the pigment synthesis process of Test Example 4-10 of the Fourth Example, except that Precursor D1 was replaced with Precursor D2 and that 4-methoxybenzenethiol was replaced with 4-heptylbenzenethiol. The anthraquinone derivative of Test Example 9-1 is a compound expressed by the following Formula (P9-1).

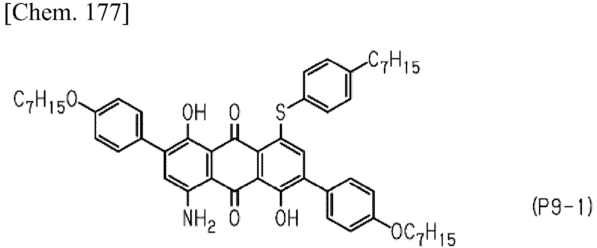

### (Test Example 9-2)

The pigment of Test Example 5-5 of the Fifth Example was used as an anthraquinone derivative of Test Example 9-2. The anthraquinone derivative of Test Example 9-2 is a compound expressed by the following Formula (P9-2).

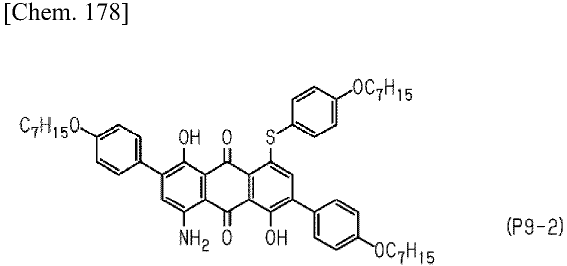

### (Test Example 9-3)

The pigment of Test Example 4-11 of the Fourth Example was used as an anthraquinone derivative of Test Example 9-3. The anthraquinone derivative of Test Example 9-3 is a compound expressed by the following Formula (P9-3).

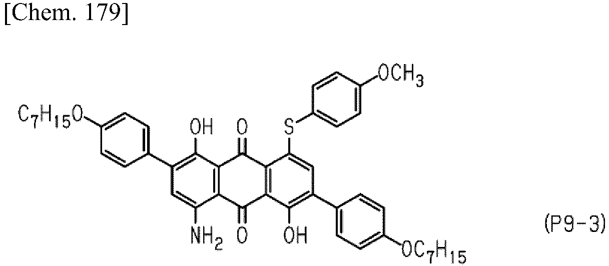

### (Test Example 9-4)

The pigment of Test Example 5-8 of the Fifth Example was used as an anthraquinone derivative of Test Example 9-4. The anthraquinone derivative of Test Example 9-4 is a compound expressed by the following Formula (P9-4).

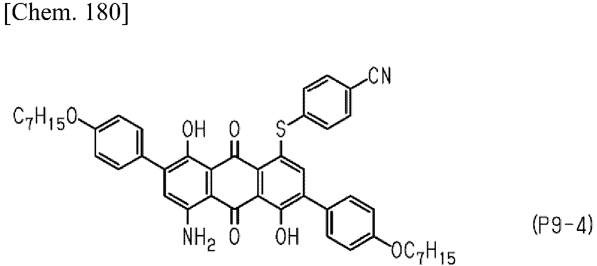

### (Test Example 9-5)

### <Pigment synthesis of Test Example 9-5>

An anthraquinone derivative of Test Example 9-5 was obtained by performing synthesis as in the pigment synthesis process of Test Example 4-10 of the Fourth Example, except that Precursor D1 was replaced with Precursor D6 and that 4-methoxybenzenethiol was replaced with 4-heptylbenzenethiol. The anthraquinone derivative of Test Example 9-5 is a compound expressed by the following Formula (P9-5).

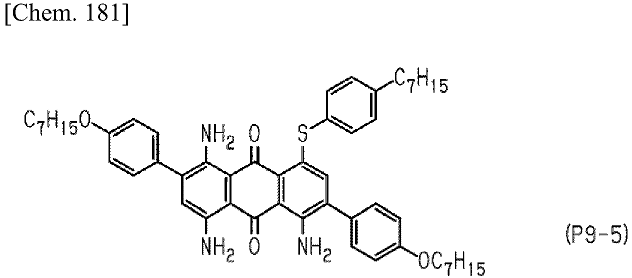

### (Test Example 9-6)

The pigment of Test Example 5-7 of the Fifth Example was used as an anthraquinone derivative of Test Example 9-6. The anthraquinone derivative of Test Example 9-6 is a compound expressed by the following Formula (P9-6).

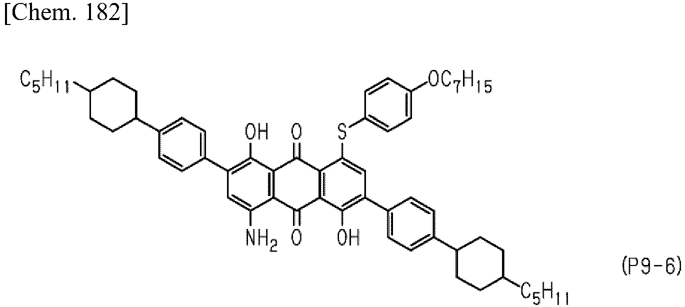

### (Test Example 9-7)

### <Pigment synthesis of Test Example 9-7>

An anthraquinone derivative of Test Example 9-7 was obtained by performing synthesis as in the pigment synthesis process of Test Example 4-10 of the Fourth Example, except that Precursor D1 was replaced with Precursor D7 and that 4-methoxybenzenethiol was replaced with 4-heptyloxybenzenethiol. The anthraquinone derivative of Test Example 9-7 is a compound expressed by the following Formula (P9-7).

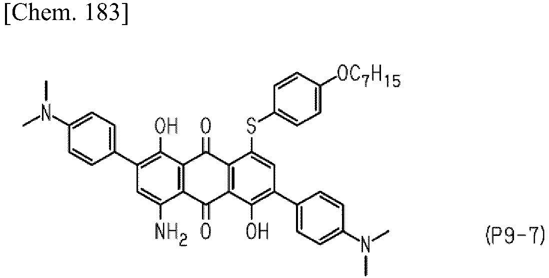

### (Test Example 9-8)

### <Pigment synthesis of Test Example 9-8>

An anthraquinone derivative of Test Example 9-8 was obtained by performing synthesis as in the pigment synthesis process of Test Example 4-10 of the Fourth Example, except that Precursor D1 was replaced with Precursor D7 and that 4-methoxybenzenethiol was replaced with 4-cyanobenzenethiol. The anthraquinone derivative of Test Example 9-8 is a compound expressed by the following Formula (P9-8).

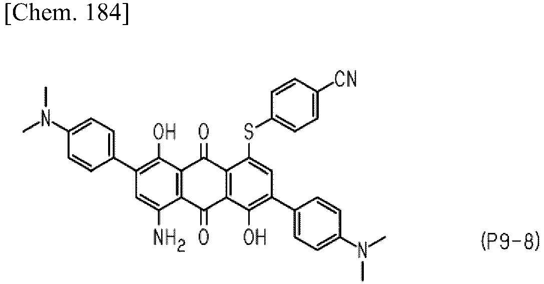

### (Test Example 9-9)

The pigment of Test Example 7-9 of the Seventh Example was used as an anthraquinone derivative of Test Example 9-9. The anthraquinone derivative of Test Example 9-9 is a compound expressed by the following Formula (P9-9).

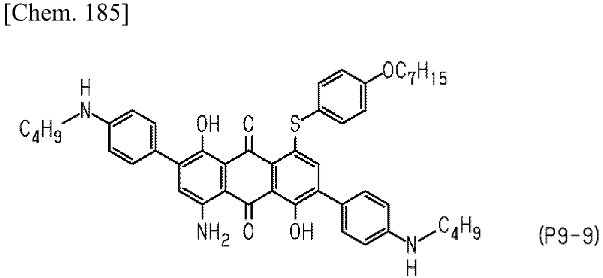

### (Test Example 9-10)

### <Pigment synthesis of Test Example 9-10>

An anthraquinone derivative of Test Example 9-10 was obtained by performing synthesis as in the pigment synthesis process of Test Example 4-10 of the Fourth Example, except that Precursor D1 was replaced with Precursor D8 and that 4-methoxybenzenethiol was replaced with 4-heptyloxybenzenethiol. The anthraquinone derivative of Test Example 9-10 is a compound expressed by the following Formula (P9-10).

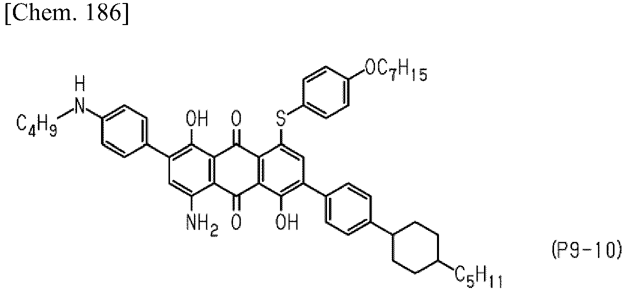

### (Test Example 9-11)

### <Pigment synthesis of Test Example 9-11>

An anthraquinone derivative of Test Example 9-11 was obtained by performing synthesis as in the pigment synthesis process of Test Example 1-1 of the First Example, except that 4-dimethylaminophenylboronic acid was replaced with 4-heptyloxyphenylboronic acid. The anthraquinone derivative of Test Example 9-11 is a compound expressed by the following Formula (P9-11).

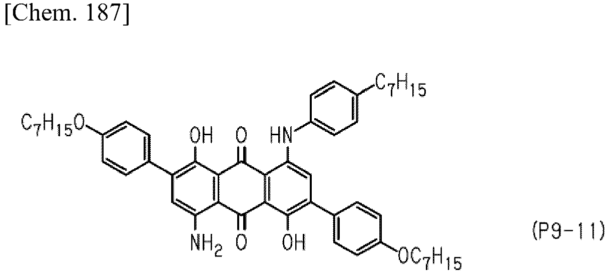

### (Test Example 9-12)

The pigment of Test Example 6-12 of the Sixth Example was used as an anthraquinone derivative of Test Example 9-12. The anthraquinone derivative of Test Example 9-12 is a compound expressed by the following Formula (P9-12).

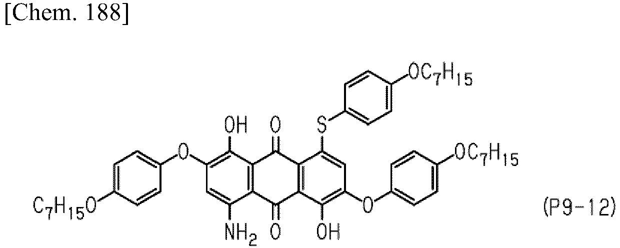

### (Evaluation method)

For the anthraquinone derivatives of Test Examples 9-1 to 9-12, evaluation test specimens were prepared as in the First Example, and respective maximum absorption wavelengths λmax were obtained.

When evaluating the absorption wavelength, test specimens having a maximum absorption wavelength λmax of 580 nm or greater and 670 nm or less were evaluated to have a Good blue hue and those having a maximum absorption wavelength λmax of less than 580 nm or greater than 670 nm were evaluated to have a Poor blue hue.

As in the First Example, the evaluation test specimens were subjected light resistance testing to evaluate light resistance.

### (Evaluations)

Table 9 shows the structure, maximum absorption wavelength λmax, evaluation of absorption wavelength, absorbance change ratio ΔAbs, and evaluation of light resistance of each anthraquinone derivative, for Test Examples 9-1 to 9-10. The structure of the anthraquinone derivative of each test example is expressed by the following Formula (III), and R, Y¹, Y² and Z of Table 9 correspond to R, Y¹, Y² and Z of Formula (III).

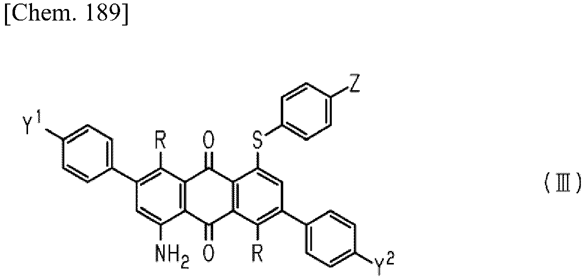

**[Table 9]**

| Test Ex. | Structure | | | | Absorption wavelength | | Light resistance | |
|---|---|---|---|---|---|---|---|---|
| | R | Y¹ | Y² | Z | λmax (nm) | Evaluation | ΔAbs (%) | Evaluation |
| 9-1 | OH | OC₇H₁₅ | OC₇H₁₅ | C₇H₁₅ | 637 | Good | 3 | Good |
| 9-2 | OH | OC₇H₁₅ | OC₇H₁₅ | OC₇H₁₅ | 638 | Good | 2 | Excellent |
| 9-3 | OH | OC₇H₁₅ | OC₇H₁₅ | OCH₃ | 638 | Good | <1 | Excellent |
| 9-4 | OH | OC₇H₁₅ | OC₇H₁₅ | CN | 629 | Good | 5 | Good |
| 9-5 | NH₂ | OC₇H₁₅ | OC₇H₁₅ | C₇H₁₅ | 656 | Good | 1 | Excellent |
| 9-6 | OH | CyC₅H₁₁ | CyC₅H₁₁ | OC₇H₁₅ | 592 | Good | 2 | Excellent |
| 9-7 | OH | N(CH₃)₂ | N(CH₃)₂ | OC₇H₁₅ | 665 | Good | 4 | Good |
| 9-8 | OH | N(CH₃)₂ | N(CH₃)₂ | CN | 669 | Good | 6 | Good |
| 9-9 | OH | NHC₄H₉ | NHC₄H₉ | OC₇H₁₅ | 650 | Good | 1 | Excellent |
| 9-10 | OH | NHC₄H₉ | CyC₅H₁₁ | OC₇H₁₅ | 654 | Good | 2 | Excellent |

Table 10 shows the structure, maximum absorption wavelength λmax, evaluation of absorption wavelength, absorbance change ratio ΔAbs, and evaluation of light resistance of each anthraquinone derivative, for Test Examples 9-11 and 9-12. The structure of the anthraquinone derivative of each test example is expressed by the following Formula (II), and R, A, X, Y¹, Y² and Z of Table 10 correspond to R, A, X, Y¹, Y² and Z of Formula (II).

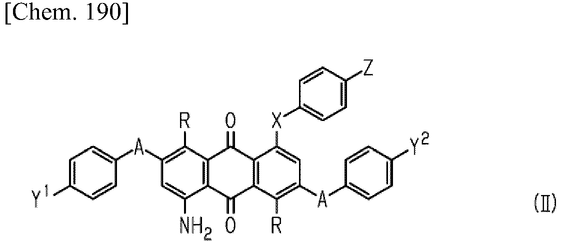

**[Table 10]**

| Test Ex. | Structure | | | | | | Absorption wavelength | | Light resistance | |
|---|---|---|---|---|---|---|---|---|---|---|
| | R | A | X | Y¹ | Y² | Z | λmax (nm) | Evaluation | ΔAbs (%) | Evaluation |
| 9-11 | OH | Direct bond | NH | OC₇H₁₅ | OC₇H₁₅ | C₇H₁₅ | 681 | Poor | 1 | Excellent |
| 9-12 | OH | O | S | OC₇H₁₅ | OC₇H₁₅ | OC₇H₁₅ | 570 | Poor | 48 | Poor |

As shown in Tables 9 and 10, in Test Examples 9-1 to 9-11, where the substituted phenyl groups were directly bonded at the β-positions of the anthraquinone skeleton, good light resistance is achieved, whereas in Test Example 9-12, where ether bonds were present at the β-positions, light resistance is extremely poor. Therefore, it was confirmed that light resistance can be improved as a result of having a structure in which substituted phenyl groups are directly bonded at the β-positions.

Furthermore, in Test Examples 9-1 to 9-10, where substituted phenyl groups are directly bonded at the β-positions and a substituted phenylthio group is present at an α-position, the maximum absorption wavelength λmax is achieved in the range of 580 nm or greater and 670 nm or less. On the other hand, in Test Example 9-11, where substituted phenyl groups are directly bonded at the β-positions and a substituted anilino group is present at the α-position, the maximum absorption wavelength λmax exceeds 680 nm, whereas in Test Example 9-12, where ether bonds are present at the β-positions and a substituted phenylthio group is present at an α-position, the maximum absorption wavelength λmax is 570 nm or less.

Therefore, it was confirmed that by having both the structure with direct bonds of substituted phenyl groups being at the β-positions, which contributes significantly to the shifting of the maximum absorption wavelength λmax toward longer wavelengths, and the structure with a substituted phenylthio group being at an α-position, which contributes less to the shifting toward longer wavelengths, the maximum absorption wavelength λmax can be achieved in a suitable range, thereby achieving both of high light resistance and a good blue hue.

## Claims

1. An anthraquinone derivative expressed by the following Formula (9-1): 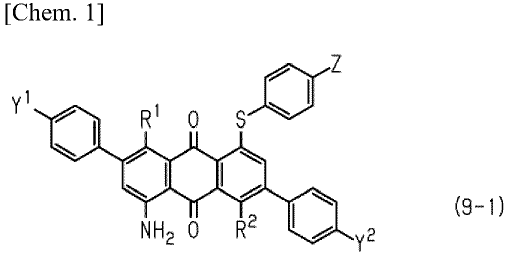 where
R¹ and R² each independently represent an amino group or a hydroxyl group, and Y¹, Y² and Z each independently represent a hydrogen atom, an alkyl group having 1 to 10 carbon atoms, an alkoxy group having 1 to 10 carbon atoms, a cyano group, a nitro group, a halogen atom, a halogenated alkyl group having 1 to 10 carbon atoms, an amino group, an alkylamino group, a piperidyl group, an aryl group, or a cyclohexyl group, and the aryl group and the cyclohexyl group may have a substituent.

2. An anthraquinone derivative expressed by the following Formula (9-2): 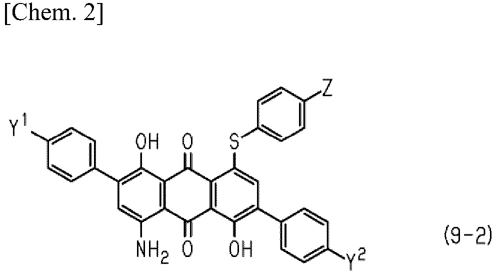 where
Y1^{,} Y² and Z each independently represent a hydrogen atom, an alkyl group having 1 to 10 carbon atoms, an alkoxy group having 1 to 10 carbon atoms, a cyano group, a nitro group, a halogen atom, a halogenated alkyl group having 1 to 10 carbon atoms, an amino group, an alkylamino group, a piperidyl group, an aryl group, or a cyclohexyl group, and the aryl group and the cyclohexyl group may have a substituent.

3. An anthraquinone derivative expressed by the following Formula (1-1): 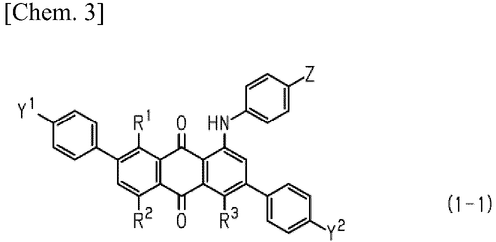 where
R¹, R² and R³ each independently represent an amino group or a hydroxyl group, at least one of R¹, R² and R³ representing a hydroxyl group;
at least one of Y¹ and Y² represents an electron-donating group, the electron-donating group being an alkylamino group, an amino group, or a piperidyl group, and if only one of Y¹ and Y² represents an electron-donating group, the other of Y¹ and Y² represents a hydrogen atom, an alkyl group having 1 to 10 carbon atoms, an alkoxy group having 1 to 10 carbon atoms, an aryl group, or a cyclohexyl group, and the aryl group and the cyclohexyl group may have a substituent; and
Z represents a hydrogen atom, an alkyl group having 1 to 10 carbon atoms, an alkoxy group having 1 to 10 carbon atoms, a cyano group, a nitro group, a halogen atom, an amino group, or an alkylamino group.

4. An anthraquinone derivative expressed by the following Formula (1-1): 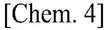 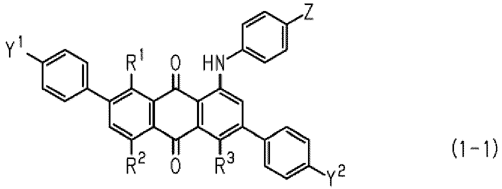 where
R¹, R² and R³ each independently represent an amino group or a hydroxyl group;
at least one of Y¹ and Y² represents an electron-donating group, the electron-donating group being an acetamide group or a hydroxyl group, and if only one of Y¹ and Y² represents an electron-donating group, the other of Y¹ and Y² represents a hydrogen atom, an alkyl group having 1 to 10 carbon atoms, an alkoxy group having 1 to 10 carbon atoms, an aryl group, or a cyclohexyl group, and the aryl group and the cyclohexyl group may have a substituent; and
Z represents a hydrogen atom, an alkyl group having 1 to 10 carbon atoms, an alkoxy group having 1 to 10 carbon atoms, a cyano group, a nitro group, a halogen atom, an amino group, or an alkylamino group.

5. The anthraquinone derivative according to claim 3 or 4, wherein
in Formula (1-1), R¹ and R³ each represent a hydroxyl group and R² represents an amino group.

6. An anthraquinone derivative expressed by the following Formula (2-1): 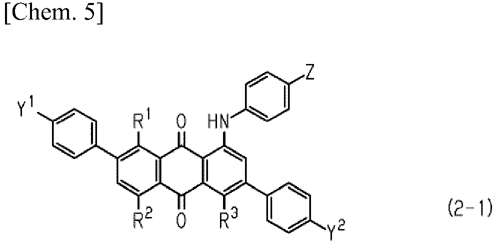 where
R¹, R² and R³ each independently represent an amino group or a hydroxyl group, at least one of R¹, R² and R³ representing a hydroxyl group;
Y¹and Y² each independently represent a hydrogen atom, an alkyl group having 1 to 10 carbon atoms, an alkoxy group having 1 to 10 carbon atoms, an aryl group, a cyano group, a nitro group, a halogen atom, an amino group, an alkylamino group, or a cyclohexyl group, and the aryl group and the cyclohexyl group may have a substituent; and
Z represents an electron-withdrawing group, the electron-withdrawing group being a cyano group or a halogenated alkyl group.

7. An anthraquinone derivative expressed by the following Formula (2-1): 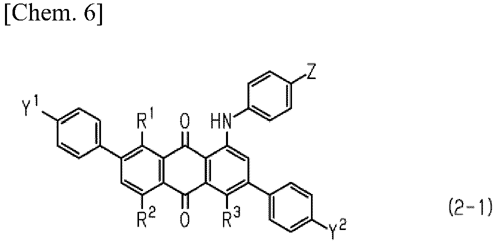 where
R¹, R² and R³ each independently represent an amino group or a hydroxyl group;
Y¹and Y² each independently represent a hydrogen atom, an alkyl group having 1 to 10 carbon atoms, an alkoxy group having 1 to 10 carbon atoms, an aryl group, a cyano group, a nitro group, a halogen atom, an amino group, an alkylamino group, or a cyclohexyl group, and the aryl group and the cyclohexyl group may have a substituent; and
Z represents an electron-withdrawing group, the electron-withdrawing group being an aldehyde group, an acetyl group, or a sulfo group.

8. The anthraquinone derivative according to claim 6 or 7, wherein
in Formula (2-1), R¹ and R³ each represents a hydroxyl group and R² represents an amino group.

9. An anthraquinone derivative according to claim 8 expressed by the following Formula (2-3) 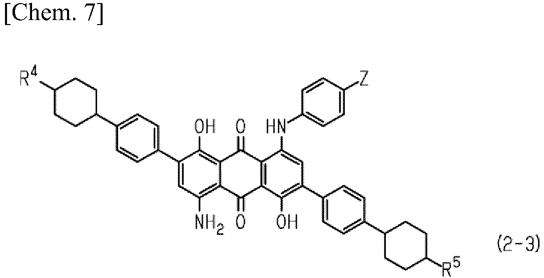 where
R⁴ and R⁵ independently represents a hydrogen atom, an alkyl group having 1 to 10 carbon atoms, or a cyclic hydrocarbon group; and
Z represents the electron-withdrawing group.

10. An anthraquinone derivative expressed by the following Formula (3-1): 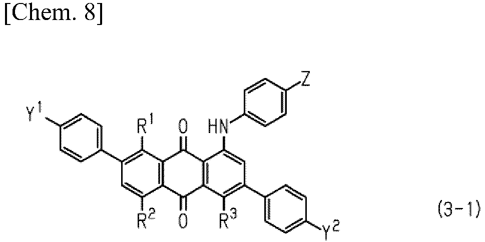 where
R¹, R² and R³ each independently represent an amino group or a hydroxyl group, at least one of R¹, R² and R³ representing a hydroxyl group;
at least one of Y¹ and Y² represents a cyclohexyl group, and the cyclohexyl group may have a substituent, and if only one of Y¹ and Y² represents a cyclohexyl group, the other of Y¹ and Y² represents a hydrogen atom, an alkyl group having 1 to 10 carbon atoms, an alkoxy group having 1 to 10 carbon atoms, an aryl group, a cyano group, a nitro group, a halogen atom, an amino group, or an alkylamino group, and the aryl group and the cyclohexyl group may have a substituent; and
Z represents a hydrogen atom, an alkyl group having 1 to 10 carbon atoms, an alkoxy group having 1 to 10 carbon atoms, a cyano group, a nitro group, a halogen atom, an amino group, or an alkylamino group.

11. An anthraquinone derivative expressed by the following Formula (3-2): 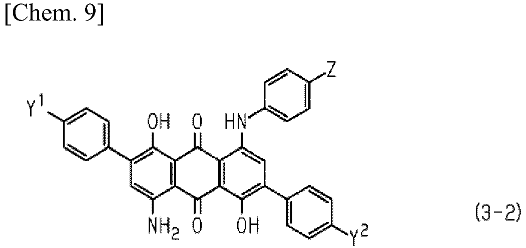 where
at least one of Y¹ and Y² represents a cyclohexyl group, and the cyclohexyl group may have a substituent, and if only one of Y¹ and Y² represents a cyclohexyl group, the other of Y¹ and Y² represents a hydrogen atom, an alkyl group having 1 to 10 carbon atoms, an alkoxy group having 1 to 10 carbon atoms, an aryl group, a cyano group, a nitro group, a halogen atom, an amino group, or an alkylamino group, and the aryl group may have a substituent; and
Z represents a hydrogen atom, an alkyl group having 1 to 10 carbon atoms, an alkoxy group having 1 to 10 carbon atoms, a cyano group, a nitro group, a halogen atom, an amino group, or an alkylamino group.

12. An anthraquinone derivative according to claim 11 expressed by the following Formula (3-3): 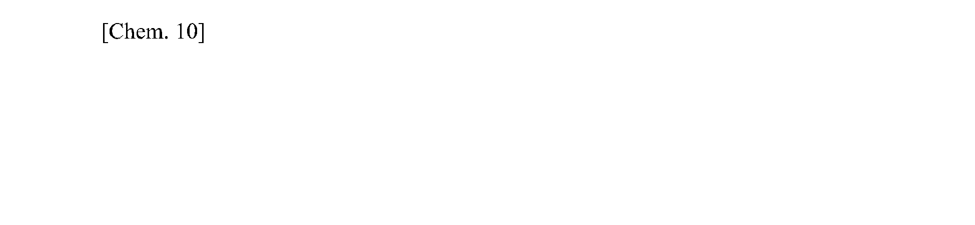 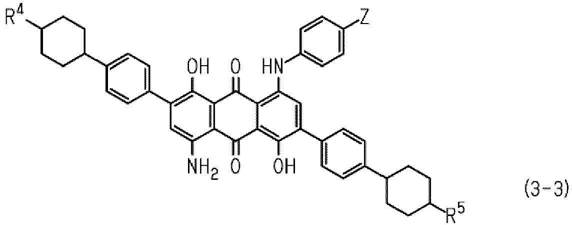 where
R4 and R⁵ each independently represent a hydrogen atom, an alkyl group having 1 to 10 carbon atoms, or a cyclic hydrocarbon group; and
Z represents a hydrogen atom, an alkyl group having 1 to 10 carbon atoms, an alkoxy group having 1 to 10 carbon atoms, a cyano group, a nitro group, a halogen atom, an amino group, or an alkylamino group.

13. An anthraquinone derivative expressed by the following Formula (8-1): 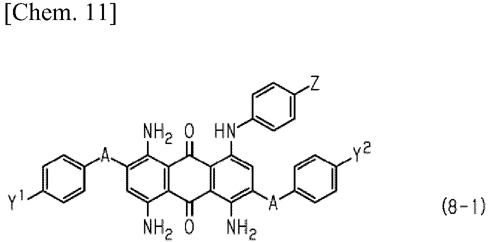 where
A represents a direct bond, an oxygen atom, or -NH-; and
Y¹, Y² and Z each independently represent a hydrogen atom, an alkyl group having 1 to 10 carbon atoms, an alkoxy group having 1 to 10 carbon atoms, a cyano group, a nitro group, a halogen atom, a halogenated alkyl group having 1 to 10 carbon atoms, an amino group, an alkylamino group, an aryl group, a piperidyl group, or a cyclohexyl group, and the aryl group and the cyclohexyl group may have a substituent.

14. An anthraquinone derivative expressed by the following Formula (8-2): 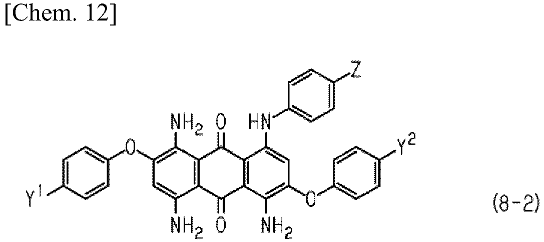 where
Y1^{,} Y² and Z each independently represent a hydrogen atom, an alkyl group having 1 to 10 carbon atoms, an alkoxy group having 1 to 10 carbon atoms, a cyano group, a nitro group, a halogen atom, a halogenated alkyl group having 1 to 10 carbon atoms, an amino group, an alkylamino group, an aryl group, a piperidyl group, or a cyclohexyl group, and the aryl group and the cyclohexyl group may have a substituent.

15. An anthraquinone derivative expressed by the following Formula (8-3): 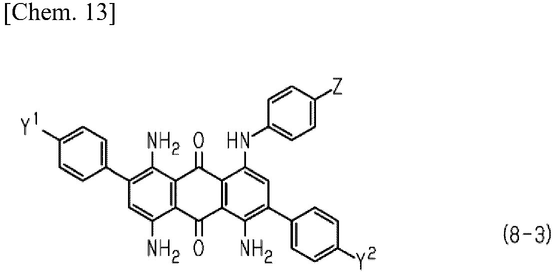 where
Y1^{,} Y² and Z each independently represent a hydrogen atom, an alkyl group having 1 to 10 carbon atoms, an alkoxy group having 1 to 10 carbon atoms, a cyano group, a nitro group, a halogen atom, a halogenated alkyl group having 1 to 10 carbon atoms, an amino group, an alkylamino group, an aryl group, a piperidyl group, or a cyclohexyl group, and the aryl group and the cyclohexyl group may have a substituent.
